# EUROPÄISCHE PATENTANMELDUNG

(11) **EP 0 600 832 A1**
(43) Veröffentlichungstag der Anmeldung: **08.06.1994**
(21) Anmeldenummer: 93810802.4
(22) Anmeldetag: 18.11.1993
(51) Int. Cl.: C07C 237/30, A61K 31/165, C07C 229/62, C07D 241/42, C07C 229/64, C07D 307/88, C07C 243/38, C07D 209/48, C07C 237/40, C07C 255/58, C07D 213/74, A61K 31/24, A61K 31/195, A61K 31/40, A61K 31/44

(54) **Diaminobenzoe- und Diaminophthalsäurederivate und ihre Verfahren als Proteinkinase-Inhibitoren**

(30) Priorität: 27.11.1992 CH 3651/92
(71) Anmelder: CIBA-GEIGY AG, CH-4002 Basel (CH)
(72) Erfinder: Kump, Wilhelm, Dr., CH-4105 Biel-Benken (CH); Trinks, Uwe, Dr., CH-4142 Münchenstein (CH); Traxler, Peter, Dr., CH-4142 Schönenbuch (CH)

(57) **Zusammenfassung**

Die Erfindung betrifft Verbindungen der Formel I, 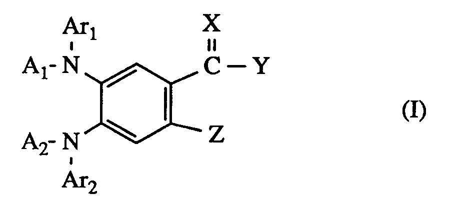
worin A₁ und A₂ Wasserstoff, unsubstituiertes oder substituiertes Niederalkyl, Niederalkenyl oder Niederalkinyl, Heterocyclylniederalkyl, Acyl, Niederalkylsulfonyl oder Arylsulfonyl bedeuten, oder zusammen unsubstituiertes oder substituiertes Niederalkylen bedeuten; Ar₁ und Ar₂ Aryl, Heteroaryl oder unsubstituiertes oder substituiertes Cycloalkyl bedeuten, X für O oder S, Y für Amino, substituiertes Amino, substituiertes Niederalkoxy oder unsubstituiertes oder substituiertes Niederalkylthio; oder, falls X für O und Z für substituiertes Methyl stehen, für Hydroxy steht; und Z für substituiertes Methyl, Carboxy oder verestertes oder amidiertes Carboxy oder Thiocarboxy steht; oder Y und Z zusammen 1-Oxamethylen bedeuten; Salze davon, Tautomere davon, Verfahren zur Herstellung, pharmazeutische Präparate, und/oder diese Verbindungen zur therapeutischen Behandlung des menschlichen oder tierischen Körpers oder zur Herstellung pharmazeutischer Präparate. Die Verbindungen hemmen Proteinkinasen.

## Beschreibung

Die Erfindung bezieht sich auf Verbindungen der Formel I, 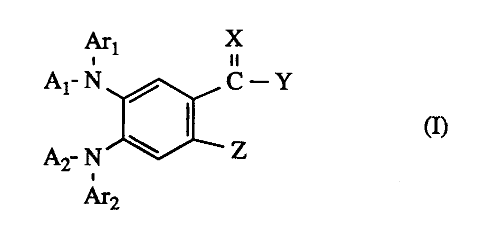
worin A₁ und A₂ unabhängig voneinander Wasserstoff, unsubstituiertes oder substituiertes Niederalkyl, unsubstituiertes oder substituiertes Niederalkenyl, unsubstituiertes oder substituiertes Niederalkinyl, Heterocyclylniederalkyl, Acyl, Niederalkylsulfonyl oder Arylsulfonyl bedeuten, oder worin A₁ und A₂ zusammen unsubstituiertes oder substituiertes Niederalkylen bedeuten; Ar₁ und Ar₂ unabhängig voneinander Aryl, Heteroaryl oder unsubstituiertes oder substituiertes Cycloalkyl bedeuten, X für O oder S, Y für Amino, substituiertes Amino, substituiertes Niederalkoxy oder unsubstituiertes oder substituiertes Niederalkylthio; oder ausserdem, falls X für O und Z für substituiertes Methyl stehen, für Hydroxy steht; und Z für substituiertes Methyl, Carboxy, verestertes Carboxy oder einen analogen Rest, in dem das Oxo des Carbonylrestes durch Thio ersetzt ist, oder amidiertes Carboxy oder oder einen analogen Rest, in dem das Oxo des Carbonylrestes durch Thio ersetzt ist, steht; oder Y und Z zusammen 1-Oxamethylen bedeuten, das über den Sauerstoff an der Stelle von Y und das Methylenkohlenstoffatom anstelle von Z gebunden ist; und Salze davon, sofern salzbildende Gruppen vorliegen, und/oder Tautomere davon, sofern tautomerisierbare Reste vorliegen, Verfahren zur Herstellung dieser Verbindungen, pharmazeutische Präparate umfassend diese Verbindungen, diese Stoffe zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Sofern vor- und nachstehend von Verfahren zur Behandlung des menschlichen oder tierischen Körpers die Rede ist, umfasst dieser Begriff auch Methoden zur Behandlung von Warmblütern und Verfahren zur Hemmung von Proteinkinasen in Warmblütern.

Das Präfix "Nieder" bezeichnet einen Rest bis und mit 7, insbesondere bis und mit 4, und vor allen Dingen mit 1 bis 3 Kohlenstoffatomen, sofern nichts anderes angegeben ist.

Niederalkyl ist vorzugsweise n-Propyl, Isopropyl, n-Butyl, Isobutyl, sek-Butyl, tert-Butyl, n-Pentyl, Neopentyl, n-Hexyl oder n-Heptyl, vorzugsweise Methyl, Ethyl oder n-Propyl.

Niederalkenyl hat 2 bis 7, vorzugsweise 3 bis 7, insbesondere 3 oder 4 Kohlenstoffatome, und ist z. B. Allyl oder Crotyl.

Niederalkinyl hat 2 bis 7, vorzugsweise 3 bis 7, insbesondere 3 oder 4 Kohlenstoffatome, und ist z. B. Propin-1- oder Propin-2-yl oder 2-Butin-1-yl.

Substituiertes Niederalkyl ist vorzugsweise wie oben definiertes Niederalkyl, welches durch bis zu 4, vorzugsweise bis zu 2 Reste ausgewählt aus Amino, wie in Aminomethyl, -ethyl, -propyl oder -butyl, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, wie Aminomethyl, Aminoethyl, Aminopropyl oder Aminobutyl, Cycloalkylamino, Phenylniederalkylamino oder Phenylamino, Acylamino, z.B. Niederalkanoylamino, Phenylniederalkanoylamino oder Phenylcarbonylamino ( ≙ Benzoylamino), Hydroxy, wie in Hydroxymethyl, Hydroxyethyl oder Hydroxypropyl, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkoxy, Acyloxy, insbesondere Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylthio, Acylthio, insbesondere Niederalkanoylthio, Carboxy, wie in Carboxymethyl, -ethyl oder -propyl, verestertes Carboxy, z.B. Niederalkoxycarbonyl, wie Methoxycarbonyl, Ethoxycarbonyl oder tert-Butoxycarbonyl, vor allem in Methoxy- oder Ethoxycarbonyl-methyl, -ethyl oder -proypl, oder Phenylniederalkoxycarbonyl, wie Benzyloxycarbonyl, Cyano, Carbamoyl, wie in Carbamoylmethyl, Carbamoylethyl oder Carbamoylpropyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl; Ureido, an einem oder beiden Stickstoffatomen durch Niederalkyl, Aryl oder Arylniederalkyl substituiertes Ureido, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, vor allem in 3-Methyl- oder 3-Ethyl-ureido-methyl, -ethyl oder -propyl, Thioureido, an einem oder beiden Stickstoffatomen durch Niederalkyl, Aryl oder Arylniederalkyl substituiertes Thioureido, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z.B. 3-Niederalkylthioureido, wie 3-Methyl- oder 3-Ethylthioureido, vor allem in 3-Methyl- oder 3-Ethyl-thioureido-methyl, -ethyl oder -propyl, Hydrazino, an einem oder beiden Stickstoffatomen durch Niederalkyl, Aryl oder Arylniederalkyl substituiertes Hydrazino, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, Amidino, wie in Amidinomethyl, -ethyl oder -propyl, an einem oder beiden Stickstoffatomen durch Niederalkyl, Aryl oder Arylniederalkyl substituiertes Amidino, insbesondere N¹- oder N²-Mono-, N¹,N²-Di- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, Guanidino, wie in Guanidinomethyl, -ethyl oder -propyl, an einem, zwei oder allen drei Stickstoffatomen durch Niederalkyl, Aryl oder Arylniederalkyl substituiertes Guanidino, insbesondere 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3-Pentaniederalkyl- oder -Phenylniederalkylguanidino, insbesondere 3,3-Diniederalkylguanidino, wie 3,3-Dimethylguanidino oder 3,3-Diethylguanidino, Oxo, das nicht am Kohlenstoff gebunden ist, welcher an den A₁ oder A₂ tragenden Stickstoff gebunden ist, wie in 2-Oxopropyl oder 3-Oxo-n-butyl, Thioxo, Imino, Niederalkylimino, Acylimino, insbesondere Niederalkanoylimino, wie Acetylimino, Hydroxyimino (HO-N=), wie in Hydroxyiminomethyl (HO-N=CH-), Hydroxyimino-ethyl oder -propyl, Niederalkoxyimino, wie Methoxyimino, Hydrazono, wie in Hydrazonomethyl, -ethyl oder -propyl, N- Mono- oder N,N-Diniederalkylhydrazono, N-Acylhydrazono, insbesondere N-Niederalkanoylhydrazono, wie Acetylhydrazono oder Niederalkoxycarbonylhydrazono, wie tert-But-oxycarbonylhydrazono, und Niederalkylthioimino, wie Methyl- oder Ethylthioimino, vor allem in Methylthioimino- oder Ethylthioimino-methyl, -ethyl oder -propyl, substituiert ist. Vorzugsweise ist Niederalkyl linear und endständig substituiert mit einem der genannten Substituenten.

Arylniederalkyl enthält vorzugsweise Aryl, wie unten definiert, und ist insbesondere Phenylniederalkyl, wie Benzyl.

Substituiertes Niederalkenyl ist vorzugsweise wie oben definiertes Niederalkenyl, insbesondere mit 3 bis 7, vor allem 3 oder 4 Kohlenstoffatomen, welches durch bis zu 4 Reste, vorzugsweise einen Rest ausgewählt aus den bei der Definition von substituiertem Niederalkyl genannten Substituenten substituiert ist. Bei bestimmten Substituenten sind durch Wechselwirkung mit der Doppelbindung Tautomere möglich. So können Hydroxy, Mercapto oder N-gebundene Substituenten, die noch einen freien Wasserstoff am bindenden Stickstoff haben, bei Bindung an ein Kohlenstoffatom einer Dreifachbindung tautomerisieren zu Oxo- bzw. Thioxo- bzw. Iminoverbindungen; mit einer Doppelbindung über Stickstoff gebundene Substituenten wie Hydroxyimino oder Hydrazono können bei Vorliegen in Konjugation zu einer Doppelbindung im Niederalkenylrest ebenfalls tautomerisieren; derartige Verbindungen können auch in Tautomeriegleichgewichten vorliegen. Bevorzugt als substituiertes Niederalkenyl sind solche Reste, bei denen keine Tautomerie auftritt, d. h. worin z.B. Hydroxy, Mercapto oder N-gebundene Substituenten, die noch einen freien Wasserstoff am bindenden Stickstoff haben, nicht an ein Kohlenstoffatom im Niederalkenylrest gebunden sind, von dem eine Doppelbindung ausgeht, und/oder wo mit einer Doppelbindung über Stickstoff gebundene Substituenten wie Hydroxyimino oder Hydrazono nicht in Konjugation mit einer Doppelbindung im Niederalkenylrest stehen. Bevorzugt gegenüber substituiertem Niederalkenyl ist jedoch unsubstituiertes Niederalkenyl A₁ und/oder A₂.

Substituiertes Niederalkinyl ist vorzugsweise wie oben definiertes Niederalkinyl, insbesondere mit 3 bis 7, vor allem 3 oder 4 Kohlenstoffatomen, welches durch bis zu 4 Reste, vorzugsweise einen Rest ausgewählt aus den bei der Definition von substituiertem Niederalkyl genannten Substituenten substituiert ist. Analog der Definition von Tautomeren und Tautomeriegleichgewichten für substituiertes Niederalkenyl können auch für substituiertes Niederalkinyl entsprechenden Tautomere und Tautomeriegleichgewichte vorliegen. Bevorzugt sind auch hier die entsprechenden Reste, bei denen keine Tautomerie auftritt - diese sind analog definiert wie bei substituiertem Niederalkenyl. Bevorzugt gegenüber substituiertem Niederalkinyl ist jedoch unsubstituiertes Niederalkinyl A₁ und/oder A₂.

Heterocyclylniederalkyl ist einer der oben genannten Niederalkylreste, vorzugsweise Methyl, Ethyl oder n-Propyl, welcher, vorzugsweise am endständigen Kohlenstoffatom, substituiert ist durch Heterocyclyl, welches insbesondere einen gesättigten, teilweise gesättigten oder ungesättigten einfachen Ring bedeutet, der über ein Ringstickstoffatom gebunden ist und 3 bis 7, in erster Linie 5 - 7 Ringatome enthält, wobei ausser dem bindenden Stickstoffatom noch bis zu zwei weitere Heteroatome ausgewählt aus Stickstoff, Schwefel und/oder Sauerstoff vorliegen können; als einfacher Ring vorliegt oder bis zu zweifach, vorzugsweise einfach benzanneliert, cyclopenta-, cyclohexa- oder cyclohepta- anneliert sein kann; und unsubstituiert oder insbesondere durch Niederalkyl, Niederalkanoyl, Hydroxy, Niederalkoxy, Halogen, Cyano und/oder Trifluormethyl substituiert sein kann, z. B. Pyrrolyl, 2,5-Dihydropyrrolyl, Pyrrolinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, oder durch Niederalkanoyl, z. B. in 4-Niederalkanoyl-piperazin-1-yl, wie 4-Acetyl-piperazin-1-yl.

Acyl bedeutet vorzugsweise Niederalkanoyl, Halogenniederalkanoyl, wie Trifluor- oder Trichloracetyl, Arylniederalkanoyl, z.B. Phenylniederalkanoyl, wie Phenylacetyl, Arylcarbonyl, wie Phenylcarbonyl, wobei Aryl in den beiden zuletzt genannten Fällen wie oben definiert ist mit der Ausnahme, dass in den zuletzt genannten Arylresten vorkommende Acylsubstituenten vorzugsweise aus Niederalkanoyl, Phenylniederalkanoyl und Phenylcarbonyl ausgewählt sind, Niederalkoxycarbonyl, wie tert-Butoxycarbonyl, oder Phenylniederalkoxycarbonyl, wie Benzyloxycarbonyl. Acyl ist in erster Linie Niederalkanoyl.

Niederalkanoyl ist vorzugsweise Formyl, Acetyl, Propionyl, n-Butyryl, Pivaloyl oder Valeroyl, insbesondere Formyl, Acetyl oder Propionyl.

Halogen steht insbesondere für Fluor, Chlor, Brom und/oder Iod, vor allem Fluor oder Iod.

Niederalkylsulfonyl (= Niederalkyl-SO₂-) ist vorzugsweise Methan- oder Ethansulfonyl.

Arylsulfonyl (= Aryl-SO₂-) ist vorzugsweise Benzol- oder Toluol-, wie 4-Toluolsulfonyl.

Niederalkylen, welches aus A₁ und A₂ zusammen gebildet wird, ist unverzweigt, hat insbesondere 1 bis 4, bevorzugt 2 bis 3, Kohlenstoffatome und ist unsubstituiert oder substituiert durch einen oder mehrere, vorzugsweise bis zu 3, insbesondere einen Substituenten ausgewählt aus Niederalkyl, wie Methyl oder Ethyl, Amino, Aminoniederalkyl, wie Aminomethyl, -ethyl oder -propyl, Mono- oder Diniederalkylamino oder Mono- oder Diniederalkylaminoniederalkyl, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Cycloalkylamino, Cycloalkylaminoniederalkyl, Phenylniederalkylamino, Phenylniederalkylaminoniederalkyl, Phenylamino, Phenylaminoniederalkyl, Acylamino, z.B. Niederalkanoylamino, Phenylniederalkanoylamino oder Phenylcarbonylamino ( ≙ Benzoylamino), Acylaminoniederalkyl, z.B. Niederalkanoylaminoniederalkyl, Phenylniederalkanoylaminoniederalkyl oder Phenylcarbonylaminoniederalkyl ( ≙ Benzoylaminoniederalkyl), Hydroxy, Hydroxyniederalkyl, z. B. Hydroxymethyl, Hydroxyethyl oder Hydroxypropyl, Niederalkoxy oder Niederalkoxyniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, z. B. 2-Methoxy- oder 2-Ethoxyethoxyniederalkyl, Phenylniederalkoxy, wie Benzyloxy, Phenylniederalkoxyniederalkyl, wie 2-Benzyloxy-ethyl, Acyloxy, insbesondere Niederalkanoyloxy, Acyloxyniederalkyl, insbesondere Niederalkanoyloxyniederalkyl, wie 2-Acetoxy-ethyl, Mercapto, Mercapto- niederalkyl, z. B. Mercaptomethyl oder Mercaptoethyl, Niederalkylthio oder Niederalkylthioniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkyl- sulfonyl, Carboxy, Niederalkoxycarbonyl, Carbamoyl, N-Niederalkylcarbamoyl, N,N-Di-niederalkylcarbamoyl und/oder Cyano substituiert oder vorzugsweise unsubstituiert ist, z.B. 2-Methylthio- oder 2-Ethylthio-ethylthioniederalkyl, Phenylniederalkylthio, wie Benzylthio, Phenylniederalkylthioniederalkyl, wie 2-Benzylthio-ethyl, Acylthio, insbesondere Niederalkanoylthio, Acylthioniederalkyl, insbesondere Niederalkanoylthioniederalkyl, wie 2-Acetylthio-ethyl, Carboxy, Carboxyniederalkyl, wie Carboxymethyl, verestertes Carboxy, z.B. Niederalkoxycarbonyl, wie Methoxycarbonyl, Ethoxycarbonyl oder tert-Butoxycarbonyl, verestertes Carboxyniederalkyl, z.B. Niederalkoxycarbonylniederalkyl, wie Methoxycarbonylmethyl, Ethoxycarbonylmethyl oder tert-Butoxycarbonylmethyl, oder Phenylniederalkoxycarbonylniederalkyl, wie Benzyloxycarbonylmethyl, Cyano, Cyanoniederalkyl, Carbamoyl, Carbamoylniederalkyl, wie Carbamoylmethyl, -ethyl oder -propyl, N-Niederalkylcarbamoyl, wie N-Methyl- oder N-Ethylcarbamoyl-methyl, -ethyl oder -propyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylnieder-alkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, 1- oder 3-Mono-, 1,3-Di- oder 3,3-Di- oder 1,3,3-Triniederalkylureidoniederalkyl, 1- oder 3-Phenylureidoniederalkyl, 1- oder 3-Mono-, 1,3- oder 3,3-Di- oder 1,3,3-Triphenylniederalkylureidoniederalkyl, z. B. 3-Niederalkylureidoniederalkyl, wie 3-Methyl- oder 3-Ethylureidoniederalkyl, vor allem 3-Methyl- oder 3-Ethylureidomethyl, -ethyl oder -propyl, Thioureido, Thioureidoniederalkyl, an einem oder beiden Stickstoffatomen durch Niederalkyl, Aryl oder Arylniederalkyl substituiertes Thioureido oder Thioureidoniederalkyl, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z. B. 3-Niederalkylthioureido, wie 3-Methyl- oder 3-Ethylthioureido, 1- oder 3-Mono-, 1,3-Di- oder 3,3-Di- oder 1,3,3-Triniederalkylthioureidoniederalkyl, 1- oder 3-Phenylthioureidoniederalkyl, 1- oder 3-Mono-, 1,3- oder 3,3-Di- oder 1,3,3-Triphenylniederalkylthioureidoniederalkyl, z. B. 3-Niederalkylthioureidoniederalkyl, wie 3-Methyl- oder 3-Ethylthioureidoniederalkyl, vor allem 3-Methyl- oder 3-Ethylthioureidomethyl, -ethyl oder -propyl, Hydrazino, Hydrazinoniederalkyl, wie Hydrazinomethyl, -ethyl oder -propyl, an einem oder beiden Stickstoffatomen durch Niederalkyl, Aryl oder Arylniederalkyl substituiertes Hydrazino oder Hydrazinoniederalkyl, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, 1- oder 2-Mono-, 1,2- oder 2,2-Di- oder 1,2,2-Triniederalkylhydrazinoniederalkyl, 1- oder 2-Phenylhydrazinoniederalkyl, 1- oder 2-Mono-, 1,2- oder 2,2-Di- oder 1,2,2-Triphenylniederalkylhydrazinoniederalkyl, z. B. 2,2-Diniederalkylhydrazinoniederalkyl, wie 2,2-Dimethyl- oder 2,2-Diethylhydrazinoniederalkyl, Amidino, Amidinoniederalkyl, wie Amidinomethyl, -ethyl oder -propyl, an einem oder beiden Stickstoffatomen durch Niederalkyl, Aryl oder Arylniederalkyl substituiertes Amidino oder Amidinoniederalkyl, insbesondere N¹- oder N²-Mono-, N¹,N²- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, N¹- oder N²-Mono-, N¹,N²- oder N¹,N¹-Di- oder N¹,N¹,N²-Triniederalkylamidinoniederalkyl, N¹- oder N²-Phenylamidinoniederalkyl, N¹- oder N²-Mono-, N¹,N²-, N¹,N¹-Di- oder N¹,N¹,N²-Triphenylniederalkylamidinoniederalkyl, z. B. N¹,N¹-Diniederalkylamidinoniederalkyl, wie N¹,N¹-Dimethyl- oder N¹,N¹-Diethylamidinoniederalkyl, Guanidino, Guanidinoniederalkyl, wie Guanidinomethyl, -ethyl oder -propyl, an einem, zwei oder allen drei Stickstoffatomen durch Niederalkyl, Aryl oder Arylniederalkyl substituiertes Guanidino oder Guanidinoniederalkyl, insbesondere 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3 3-Tetra- oder 1,1,2,3,3-Pentaniederalkyl- oder -phenylniederalkylguanidino, insbesondere 3,3-Diniederalkylguanidino, wie 3,3-Dimethylguanidino oder 3,3-Diethylguanidino, 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3-Pentaniederalkyl- oder -Phenylniederalkylguanidinoniederalkyl, insbesondere 3,3-Diniederalkylguanidinoniederalkyl, wie 3,3-Dimethylguanidinoniederalkyl oder 3,3-Diethylguanidinoniederalkyl, Oxo, Oxoniederalkyl, insbesondere Niederalkanoyl, wie Formyl, Acetyl oder Propionyl, Thioxo, Thioxoniederalkyl, Imino, Iminoniederalkyl, Niederalkylimino, Niederalkyliminoniederalkyl, Acylimino, insbesondere Niederalkanoylimino, wie Acetylimino, Acyliminoniederalkyl, insbesondere Niederalkanoyliminoniederalkyl, wie Acetyliminoniederalkyl, Hydroxyimino, Hydroxyiminoniederalkyl, wie Hydroxyiminomethyl, Hydroxyiminoethyl oder Hydroxyiminopropyl, Niederalkoxyimino oder Niederalkoxyiminoniederalkyl, wie Methoxyimino oder Methoxyiminoniederalkyl, Hydrazono, Hydrazononiederalkyl, wie Hydrazonomethyl, Hydrazonoethyl oder Hydrazonopropyl, N-Mono- oder N,N-Diniederalkylhydrazono, N-Mono- oder N,N-Diniederalkylhydrazononiederalkyl, N-Acylhydrazono, insbesondere N-Niederalkanoylhydrazono, wie Acetylhydrazono, oder Niederalkoxycarbonylhydrazono, wie tert-Butoxycarbonylhydrazono, N-Acylhydrazononiederalkyl, insbesondere N-Niederalkanoylhydrazononiederalkyl, wie Acetylhydrazononiederalkyl, oder Niederalkoxycarbonylhydrazononiederalkyl, wie tert-Butoxycarbonylhydrazononiederalkyl, Niederalkylthioimino, wie Methyl- oder Ethylthioimino, und Niederalkylthioiminoniederalkyl, wie Methyl- oder Ethylthioiminoniederalkyl, vor allem Methyl- oder Ethylthioiminomethyl, -ethyl oder -propyl.

Soweit vor- und nachstehend von Thioxo die Rede ist, ist dieser Substituent nicht an endständiges Methyl gebunden, da Thioaldehyde in der Regel instabil sind.

Aryl ist vorzugsweise Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch einen oder mehrere, vorzugsweise bis zu 5, insbesondere einen oder zwei, vor allem einen insbesondere in p-Stellung vorliegenden oder im Falle von Halogen, vor allem Fluor, bis zu 5, Substituenten aus der Gruppe bestehend aus Hydrocarbyl, z.B. Niederalkyl, Niederalkenyl, Niederalkinyl, Niederalkylen (angeknüpft an zwei benachbarten C-Atomen), Cycloalkyl, Phenylniederalkyl oder Phenyl; substituiertes Hydrocarbyl, z.B. Niederalkyl, das z.B. durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; verethertes Hydroxy, z.B. Niederalkoxy, Halogenniederalkoxy, Phenylniederalkoxy, Phenyloxy, Niederalkenyloxy, Halogenniederalkenyloxy oder Niederalkinyloxy; Niederalkylendioxy (angeknüpft an zwei benachbarten C-Atomen); verestertes Hydroxy, z.B. Niederalkanoyloxy, Phenylniederalkanoyloxy oder Phenylcarbonyloxy ( ≙ Benzoyloxy); Mercapto; verethertes Mercapto, das gegebenenfalls oxidiert ist, z.B. Niederalkylthio, Phenylniederalkylthio, Phenylthio, Niederalkylsulfinyl [-S(=O)-Niederalkyl], Phenylniederalkylsulfinyl, Phenylsulfinyl, Niederalkylsulfonyl [-S(O₂)-Niederalkyl], Phenylniederalkylsulfonyl oder Benzylsulfonyl; Halogen, Nitro, Amino; Monohydrocarbylamino, z.B. Niederalkylamino, Cycloalkylamino, Phenylniederalkylamino oder Phenylamino; Dihydrocarbylamino, z.B. Diniederalkylamino, N-Niederalkyl-N-phenylamino, N-Niederalkyl-N-phenylniederalkylamino, Niederalkylenamino oder durch -O-, -S- oder -NR'' unterbrochenes Niederalkylenamino (worin R'' für Wasserstoff, Niederalkyl oder Acyl, z.B. Niederalkanoyl, steht); Acylamino, z.B. Niederalkanoylamino, Phenylniederalkanoylamino oder Phenylcarbonylamino ( ≙ Benzoylamino); Acyl, z.B. Niederalkanoyl, Phenylniederalkanoyl oder Phenylcarbonyl (= Benzoyl); Carboxy; verestertes Carboxy, z.B. Niederalkoxycarbonyl; amidiertes Carboxy, z.B. Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy oder Phenylniederalkoxy, wie Benzyloxy, ausgewählte Reste am Phosphor substituiertes Phosphoryloxy oder durch Phenylen-1,2-dioxy am Phosphor substituiertes Phosphoryloxy (d. h. einen Rest der Formel 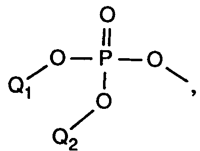
worin Q₁ und Q₂ unabhängig voneinander Wasserstoff, Niederalkyl und Phenylniederalkyl bedeuten, oder worin Q₁ und Q₂ zusammen ortho-Phenylen bedeuten), Sulfo (SO₃H); verestertes Sulfo, z.B. Niederalkoxysulfonyl; und amidiertes Sulfo, z.B. Sulfamoyl (-SO₂NH₂), N-Niederalkylsulfamoyl, N,N-Diniederalkylsulfamoyl oder N-Phenylsulfamoyl, substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; in erster Linie ist Aryl unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl oder Pentafluorphenyl.

Niederalkylen, angeknüpft an zwei benachbarten C-Atomen eines Benzolrings, ist vorzugsweise C₃-C₄-Alkylen, z.B. 1,3-Propylen oder 1,4-Butylen.

Niederalkylendioxy, angeknüpft an zwei benachbarten C-Atomen, bedeutet vorzugsweise C₁-C₂-Alkylendioxy, z.B. Methylen- oder 1,2-Ethylendioxy.

Niederalkylenamino ist vorzugsweise C₄-C₇- und insbesondere C₄-C₅-Alkylenamino, z.B. Piperidino. Durch -O-, -S- oder -NR'- unterbrochenes Niederalkylenamino steht vorzugsweise für solches C₄-C₇- und insbesondere C₄-C₅-Alkylenamino, bei dem ein Ringkohlenstoffatom durch die entsprechende Heterogruppe ersetzt ist, und ist insbesondere Morpholino, Thiomorpholino, Piperazino oder 4-Niederalkyl- oder 4-Niederalkanoylpiperazino.

Heteroaryl steht für einen ungesättigten heterocyclischen Rest und ist vorzugsweise über ein Ringkohlenstoffatom verknüpft. Es handelt sich insbesondere um einen 5- oder 6-gliedrigen Ring mit bis zu drei Heteroatomen ausgewählt aus N, O und S, vor allem N, z.B. Imidazolyl, Triazolyl, Pyridyl, Pyrimidinyl oder Triazinyl, und vor allen Dingen um Pyridyl. Diese Reste können unsubstituiert oder z.B. durch Niederalkyl, Hydroxy, Niederalkoxy, Halogen, Cyano und/oder Trifluormethyl substituiert sein.

Pyridyl ist z.B. 2-, 3- oder 4-Pyridyl.

Imidazolyl ist z.B. 2- oder 4(5)-Imidazolyl.

Triazolyl ist z.B. 1,2,4-Triazol-3- oder -4-yl oder 1,2,3-Triazol-4-yl.

Pyrimidinyl ist z.B. 2-, 4- oder 5-Pyrimidinyl.

Triazinyl ist z.B. 1,3,5-Triazin-2-yl.

Heteroaryl ist insbesondere 2-, 3- oder 4-Pyridyl, 2-, 4- oder 5-Pyrimidinyl oder 1,3,5-Triazin-2-yl.

Cycloalkyl ist vorzugsweise C₃-C₈- und insbesondere C₅-C₇-Cycloalkyl, was bedeuten soll, dass es 3 bis 8 bzw. 5 bis 7 Ringkohlenstoffe enthält, und ist z. B. Cyclopentyl, Cyclohexyl oder Cycloheptyl. Die genannten Cycloalkylreste können auch substituiert sein, z.B. durch Niederalkyl, Halogen, wie Fluor, Chlor oder Brom, oder Hydroxy.

Bei der Definition der Gruppe -(C=X)- als -(C=O)- und -(C=S)- ist -(C=O)- bevorzugt.

Substituiertes Amino ist vorzugsweise Mono- oder Diniederalkylamino, worin Niederalkyl unsubstituiert oder ferner durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, wie Methylamino, Cycloalkylamino, z.B. C₅-C₇-Cycloalkylamino, Phenylniederalkylamino, wie Benzylamino, Phenylamino, Acylamino, insbesondere Niederalkanoylamino, Halogenniederalkanoylamino, wie Trifluor- oder Trichloracetylamino, Arylniederalkanoylamino, z.B. Phenylniederalkanoylamino, wie Phenylacetylamino, oder Arylcarbonylamino (= Aroylamino), worin Aryl vorzugsweise unsubstituiertes oder wie oben definiert substituiertes Phenyl bedeutet, wie Phenylcarbonylamino ( ≙ Benzoylamino), Hydrazino, an einem oder beiden Stickstoffatomen durch Niederalkyl, welches unsubstituiert oder ferner durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, Aryl oder Arylniederalkyl substituiertes Hydrazino, worin Aryl insbesondere unsubstituiertes oder wie oben substituiertes Phenyl bedeutet, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, Hydroxyamino, Niederalkoxyamino, worin der Niederalkylrest unsubstituiert oder ferner durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, Niederalkylenamino, vorzugsweise C₄-C₇- und insbesondere C₄-C₅-Alkylenamino, wie Piperidino, oder durch -O-, -S- oder -NR'- unterbrochenes Niederalkylenamino (wie oben definiert), vorzugsweise C₄-C₇- und insbesondere C₄-C₅-Alkylenamino, bei dem ein Ringkohlenstoffatom durch die entsprechende Heterogruppe ersetzt ist, wie Morpholino, Thiomorpholino, Piperazino oder 4-Niederalkyl- oder 4-Niederalkanoylpiperazino, in erster Linie Niederalkylamino, wie Methylamino, Phenylamino oder Hydrazino.

Substituiertes Niederalkoxy ist vorzugsweise Niederalkoxy, worin der Niederalkylrest, insbesondere ein- oder zweifach, durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert ist, und ist vor allem Hydroxyniederalkoxy, wie 2-Hydroxyethoxy.

Unsubstituiertes oder substituiertes Niederalkylthio ist vorzugsweise Niederalkylthio, welches unsubstituiert oder im Niederalkylthiorest, insbesondere ein- oder zweifach, durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert ist, insbesondere Hydroxyniederalkylthio, wie 2-Hydroxyethylthio.

Substituiertes Methyl ist insbesondere durch einen Rest ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Niederalkanoylamino, Hydroxy, Niederalkoxy, worin der Niederalkylrest ein- oder zweifach durch Hydroxy, Niederalkoxy, Phenylniederalkoxy, Niederalkanoyloxy, Amino, Niederalkylamino und/oder Diniederalkylamino substituiert oder vorzugsweise unsubstituiert ist, Niederalkanoyloxy, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Cyano, Ureido, 1- oder 3-Mononiederalkylureido, Hydrazino, an einem oder beiden Stickstoffatomen durch Niederalkyl substituiertes Hydrazino, Guanidino, an einem, zwei oder allen drei Stickstoffatomen durch Niederalkyl substituiertes Guanidino, Oxo, Imino, Niederalkylimino, Acylimino, insbesondere Niederalkanoylimino, wie Acetylimino, Hydroxyimino, Niederalkoxyimino, Hydrazono, N-Mono- oder N,N-Diniederalkylhydrazono, N-Acylhydrazono, insbesondere N-Niederalkanoylhydrazono, wie Acetylhydrazono, oder Niederalkoxycarbonylhydrazono, wie tert-Butoxycarbonylhydrazono, und Niederalkylthioimino substituiertes Methyl, in erster Linie Hydroxymethyl, Niederalkanoyloxymethyl, wie Acetoxymethyl, Formyl oder ferner Hydroxyiminomethyl.

Verestertes Carboxy oder ein analoger Rest, in dem das Oxo des Carbonylrestes durch Thio ersetzt ist, bei der Definition von Z ist vorzugsweise Niederalkoxycarbonyl oder Niederalkylthio-carbonyl (Niederalkyl-S-(C=O)-), worin der Niederalkylrest unsubstituiert oder, insbesondere ein- oder zweifach, durch Hydroxy (besonders bevorzugt), Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert ist; Aryloxycarbonyl, Arylthio-carbonyl (Aryl-S-(C=O)-), Arylniederalkoxycarbonyl oder Arylniederalkylthio-carbonyl (Arylniederalkyl-S-(C=O)-), worin Aryl wie oben definiert ist, vor allem Phenyl- oder Phenylniederalkoxycarbonyl, worin der Phenylrest wie oben unter Aryl definiert substituiert ist, wie Phenyloxycarbonyl oder Benzyloxycarbonyl, oder (ausser im Falle von unsubstituiertem oder wie oben substituiertem Niederalkylthio-carbonyl, von Arylthio-carbonyl oder von Arylniederalkylthio-carbonyl) ein entsprechendes Thiocarbonylanaloges, und ist vor allem Niederalkoxycarbonyl, wie Methoxycarbonyl, oder Hydroxyniederalkoxycarbonyl, wie 2-Hydroxyethoxycarbonyl.

Amidiertes Carboxy oder ein analoger Rest, in dem das Oxo des Carbonylrestes durch Thio ersetzt ist, ist vorzugsweise Carbamoyl, N-Mono- oder N,N-Diniederalkylcarbamoyl, worin Niederalkyl unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfmyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, wie N-Methylaminocarbonyl, N-Cycloalkylcarbamoyl, z.B. C₅-C₇-Cycloalkylaminocarbonyl, N-Phenylniederalkylcarbamoyl, wie N-Benzylcarbamoyl, Phenylcarbamoyl, Hydrazinocarbonyl, an einem oder beiden Stickstoffatomen durch Niederalkyl, welches unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, Aryl oder Arylniederalkyl substituiertes Hydrazinocarbonyl, worin Aryl insbesondere unsubstituiertes oder wie oben substituiertes Phenyl bedeutet, insbesondere 1- oder 2-Mono-, 1,2- oder 2,2-Di- oder 1,2,2-Triniederalkyhydrazinocarbonyl, 1- oder 2-Phenylhydrazinocarbonyl, 1- oder 2-Mono-, 1,2- oder 2,2-Di- oder 1,2,2-Triphenylniederalkylhydrazinocarbonyl, z. B. 2,2-Diniederalkylhydrazinocarbonyl, wie 2,2-Dimethyl- oder 2,2-Diethylhydrazinocarbonyl, Hydroxyaminocarbonyl, Niederalkoxyaminocarbonyl, worin der Niederalkylrest unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, Niederalkylenaminocarbonyl, vorzugsweise C₄-C₇- und insbesondere C₄-C₅-Alkylenaminocarbonyl, wie Piperidinocarbonyl, oder durch -O-, -S- oder -NR'- (wie oben definiert) unterbrochenes Niederalkylenaminocarbonyl, vorzugsweise C₄-C₇- und insbesondere C₄-C₅-Alkylenaminocarbonyl, bei dem ein Ringkohlenstoffatom durch die entsprechende Heterogruppe ersetzt ist, wie Morpholino-, Thiomorpholino-, Piperazino-, 4-Niederalkylpiperazino- oder 4-Niederalkanoylpiperazinocarbonyl; oder ein entsprechendes Thioanaloges der genannten Reste; in erster Linie Carbamoyl, Niederalkylaminocarbonyl, wie Methylaminocarbonyl, Phenylaminocarbonyl oder Hydrazinocarbonyl.

Salze von Verbindungen der Formel I mit salzbildenden Gruppen sind in erster Linie pharmazeutisch verwendbare, nicht-toxische Salze. Beispielsweise können Verbindungen der Formel I mit basischen Gruppen, z.B. primären, sekundären oder tertiären Aminogruppen (auch in Hydrazino oder Hydrazono) Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. Carboxy, Sulfo oder Phospho bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin ₒder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Unter die Salze von Verbindungen der Formel I fallen auch Komplexe von Verbindungen der Formel I mit Übergangsmetallionen, z.B. Kupfer, Kobalt, Platin oder Mangan.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die Verbindungen der Formel I können als einzelne Isomere oder Isomerengemische vorliegen, sofern mehrere Isomeren möglich sind. Bei Vorliegen unsymmetrisch substituierter Doppelbindungen oder Ringe, z. B. bei substituiertem Niederalkenyl A₁ oder A₂, können die cis- und/oder die trans-Form vorliegen, bei doppelt gebundenem Stickstoff an Ringsystemen, wie in Hydroxyimino, beispielsweise die syn- oder die anti-Form. Asymmetrisch substituierte Kohlenstoffatome können in der (S)-, (R)- oder (R,S)-Form vorliegen. Es können mithin bei Vorliegen entsprechender struktureller Voraussetzungen Isomerengemische (wie Racemate oder Diastereomerengemische), reine Diastereomeren oder reine Enantiomeren vorliegen.

Soweit von den Verbindungen der Formel I Tautomere vorliegen können, sei es in reiner Form oder im Gleichgewicht mit anderen Tautomeren, sind diese bei den vor- und nachstehenden Definitionen ebenfalls umfasst. Tautomerisierbare Gruppen liegen z.B. vor, wenn an O, S oder N, welche an ein Kohlenstoffatom gebunden sind, von welchem eine Doppelbindung ausgeht, Wasserstoff gebunden ist, was (Thio-)Keto/Enol- bzw. Imin/Enamin-Tautomerie ermöglicht. Dies ist beispielsweise der Fall bei solchen Verbindungen der Formel I, die Thioureido- oder Guanidinoreste oder deren wie oben definierte substituierte Derivate mit mindestens einem Wasserstoffatom an einem Stickstoffatom enthalten. Möglich sind insbesondere auch Ring-Ketten-Tautomere solcher Verbindungen der Formel I, worin Y Amino oder substituiertes Amino mit einem freien Wasserstoffatom am Stickstoff und Z Formyl bedeuten. Diese Verbindungen liegen vorzugsweise in der Ringform folgender Formel IA vor, 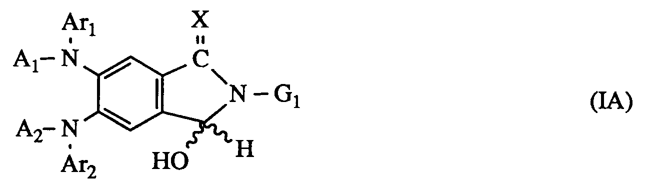
worin G₁ Wasserstoff oder einen der Substituenten von substituiertem Amino, wie oben definiert (ausser über Carbonyl gebundene Substituenten, wie Acyl), insbesondere Wasserstoff oder unsubstituiertes oder ferner wie oben bei der Definition von substituiertem Amino definiertes substituiertes Niederalkyl; Cycloalkyl oder Phenylniederalkyl bedeutet und die übrigen Reste die für Verbindungen der Formel I genannten Bedeutungen haben (wobei X vorzugsweise Sauerstoff bedeutet).

Es ist auch möglich, dass die tautomeren Formen, beispielsweise in Lösungen, im Gleichgewicht vorliegen. Alle derartigen Tautomeren, deren Auftreten dem Fachmann geläufig ist, sind ebenfalls umfasst. Bevorzugt unter den tautomerisierbaren Verbindungen der Formel I sind solche, die in nur einer tautomeren Form vorliegen oder bei denen eine tautomere Form stark überwiegt.

Die Verbindungen der Formel I weisen wertvolle, insbesondere pharmakologisch verwendbare, Eigenschaften auf. Insbesondere zeigen sie spezifische Hemmwirkungen, die von pharmakologischem Interesse sind. In erster Linie wirken sie als Protein-Tyrosinkinase-Hemmer und zeigen z.B. eine potente Hemmung der Tyrosinkinaseaktivität des Rezeptors für den epidermalen Wachstumsfaktor (EGF) und der c-erbB2-Kinase. Diese rezeptorspezifischen Enzymaktivitäten spielen eine Schlüsselrolle in der Signalübertragung in einer Vielzahl von Säugetierzellen einschliesslich Humanzellen, insbesondere von Epithelialzellen, Zellen des Immunsystems und Zellen des zentralen und peripheren Nervensystems. Die EGF-induzierte Aktivierung der rezeptorassoziierten Protein-Tyrosinkinase (EGF-R-PTK) ist bei verschiedenen Zelltypen eine Voraussetzung für die Zellteilung und damit für die Proliferation einer Zellpopulation. Durch Zugabe von EGF-Rezeptor-spezifischen Tyrosinkinasehemmern wird somit die Vermehrung dieser Zellen gehemmt.

Die Hemmung der EGF-Rezeptor-spezifischen Protein-Tyrosinkinase (EGF-R-PTK) kann z.B. mit der Methode von E. McGlynn et al. (see Europ. J. Biochem. 297, 265-275 (1992)) nachgewiesen werden. Die Verbindungen der Formel I hemmen die Enzymaktivität zu 50 % (IC₅₀) vorzugsweise in Konzentrationen ab etwa 0,001 µM oder höher, insbesondere von 10⁻⁹ bis 10⁻³ M, z.B. von etwa 10⁻⁷ M bis etwa 10⁻⁴ M. Sie zeigen ferner ebenfalls im mikromolaren Bereich, vorzugsweise im Bereich von 10⁻⁷ bis 10⁻³M, insbesondere zwischen 7 x 10⁻⁶ und 10⁻⁴M, z.B. auch eine Hemmung des Zellwachstums einer EGF-abhängigen Zellinie, etwa der epidermoiden Maus-Keratinocyten-Zellinie. Um diese Hemmung des Zellwachstums zu messen, wird die EGF-stimulierte Zellproliferation von epidermalen BALB/MK-Keratinocyten verwendet (Beschreibung des Verfahrens in Meyer, T., et al., Int. J. Cancer 43, 851 (1989)). Diese Zellen sind zur Proliferation in hohem Masse auf die Gegenwart von EGF angewiesen (Weissmann, B. E., Aaronson, S. A, Cell 32, 599 (1983). Zur Durchführung des Tests werden BALB/MK-Zellen (10 000/Vertiefung) auf 96-Loch-Mikrotiterplatten übertragen und über Nacht inkubiert. Die Testsubstanzen (gelöst in DMSO) werden in in verschiedenen Konzentrationen (in Verdünnungsreihen) zugegeben, so dass die Endkonzentration an DMSO nicht grösser als 1 % ist. Nach der Zugabe werden die Platten für drei Tage inkubiert, während denen die Kontrollkulturen ohne Testsubstanz wenigstens drei Zellteilungscyclen durchlaufen können. Das Wachstum der MK-Zellen wird gemessen mittels Methylenblau-Färbung. IC₅₀-Werte werden definiert als die Konzentration der jeweiligen Testsubstanz, die zu einer 50 %-igen Abnahme im Vergleich zu den Kontrollkulturen ohne Inhibitor führt.

Die Verbindungen der Formel I hemmen neben oder anstelle der EGF-R-PTK auch andere Tyrosinkinasen, die in die durch trophische Faktoren vermittelte Signalübertragung involviert sind, z.B. die abl-Kinase (vorzugsweise im Bereich oberhalb von 10⁻⁶M), Kinasen aus der Familie der src-Kinasen (vorzugsweise im Bereich oberhalb von 10⁻⁶M) und die c-erbB2-Kinase (HER-2), sowie Serin/Threonin-Kinasen, z.B. die Proteinkinase C (vorzugsweise im Bereich oberhalb von 10⁻⁵M), die alle in der Wachstumsregulation und Transformation bei Säugetierzellen einschliesslich Humanzellen eine Rolle spielen.

Die Hemmung der c-erbB2-Tyrosinkinase (HER-2) kann z.B. analog der für EGF-R-PTK verwendeten Methode von see Europ. J. Biochem. 297,265-275 (1992)) nachgewiesen werden. Die c-erbB2-Kinase kann nach bekannten Protokollen isoliert und ihre Aktivität bestimmt werden, z.B. nach T. Akiyama et al., Science 232, 1644 (1986), oder insbesondere nach B.M. Guy et al. (J. Biol. CHem. 267, 13851-13856 (1992)).

Somit sind die Verbindungen der Formel I auch zur Hemmung der durch diese und verwandte Tyrosinkinasen vermittelten Prozesse geeignet.

Die Antitumoraktivität in vivo wird beispielsweise getestet unter Verwendung des menschlichen Epithelzellkarzinoms A431 (ATCC No. CRL 1555), welches in weibliche BALB/c-Nacktmäuse (Bomholtgard, Dänemark) transplantiert wird. Für die Experimente werden in vivo auftretende Tumoren von etwa 1 cm³ Durchmesser unter sterilen Bedingungen aus den Tieren durch Excision gewonnen. Diese Tumoren werden homogenisiert, in 10 Volumina (w/v) Phosphat-gepufferter Saline suspendiert und s.c. (0,2 ml/Maus, z.B. 10⁶ Zellen/Maus) in die linke Flanke der Tiere injiziert. Die Behandlung mit einer Testsubstanz wird 5 bis 8 Tage nach der Transplantation eingeleitet, wenn der Durchmesser der Tumoren 4 bis 5 mm beträgt. Die jeweilige Testsubstanz (beispielsweise gelöst in ®Lauroglycol (1,2-Propylenglykol-monolaurat, Gemisch der beiden Konstitutionsisomeren, Gattefossé S.A., Saint Priest, Frankreich), ®Gelucire (Glyceride und Teilpolyglyceride von Fettsäuren, Gattefossé S.A., Saint Priest, Frankreich) oder Sesamöl) wird p.o. täglich während 15 aufeinanderfolgenden Tagen verabreicht. Das Tumorwachstum wird durch Überwachung des senkrechten Tumordurchmessers bestimmt und das Tumorvolumen nach der Formel π x L x D²/6 berechnet (L = Länge, D = Durchmesser des Tumors senkrecht zur Tumorachse). Die Ergebnisse werden ausgedrückt als Behandlung/Kontrolle (T/C) in Prozent.

Die Verbindungen der Formel I sind daher z.B. bei der Behandlung benigner oder maligner Tumoren nützlich. Sie sind in der Lage, Tumorregression zu bewirken und Tumormetastasierung und das Wachstum von Mikrometastasen zu verhindern. Insbesondere sind sie bei epidermaler Hyperproliferation (Psoriasis), bei der Behandlung von Neoplasien epithelialen Charakters, z.B. Mammakarzinomen, und bei Leukämien anwendbar. Ferner sind die Verbindungen bei der Behandlung von Erkrankungen des Immunsystems und von Entzündungen einzusetzen, soweit in diese Erkrankungen Proteinkinasen involviert sind. Auch bei der Behandlung von Erkrankungen des zentralen oder peripheren Nervensystems sind die Verbindungen einzusetzen, soweit Signalübertragung durch Proteinkinasen involviert ist. Schliesslich haben die Verbindungen der Formel I auch antimikrobielle Eigenschaften, die sie z. B. zur Behandlung von Erkrankungen geeignet machen, die durch Bakterien, wie Salmonella typhimurium, Viren, wie Vacciniavirus, und weitere Mikroben, die mit auf Wachstumsfaktoren reagierenden Proteinkinasen interagieren, verursacht werden.

Die Verbindungen der Formel I können sowohl alleine als auch in Kombination mit anderen pharmakologisch wirksamen Substanzen angewandt werden, z.B. zusammen mit (a) Inhibitoren der Enzyme der Polyaminsynthese, (b) Inhibitoren der Proteinkinase C, (c) Inhibitoren anderer Tyrosinkinasen, (d) Cytokinen, (e) negativen Wachstumsregulatoren, z.B. TGF-β oder IFN-β, (f) Aromatasehemmern, (g) Antiöstrogenen oder (h) Cytostatika.

Bei den nachfolgend genannten Gruppen von Verbindungen der Formel I kann man allgemeine Definitionen, beispielsweise von Substituenten, unabhängig voneinander durch oben bei den allgemeinen Definitionen genannte spezifischere Definitionen ersetzen.

Bevorzugt sind die Verbindungen der Formel I, worin A₁ und A₂ unabhängig voneinander Wasserstoff, Niederalkyl, substituiertes Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, C₃-C₈-Cycloalkylamino, Phenylniederalkylamino, Phenylamino, Niederalkanoylamino, Phenylniederalkanoylamino, Phenylcarbonylamino, Hydroxy, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert oder vorzugsweise unsubstituiert ist, Phenylniederalkoxy, Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylthio, Niederalkanoylthio, Carboxy, Niederalkoxycarbonyl, Phenylniederalkoxycarbonyl, Cyano, Carbamoyl,N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, Thioureido, an einem oder beiden Stickstoffatomen durch Niederalkyl, Aryl oder Arylniederalkyl substituiertes Thioureido, Hydrazino, an einem oder beiden Stickstoffatomen durch Niederalkyl, Aryl oder Arylniederalkyl substituiertes Hydrazino, Amidino, an einem oder beiden Stickstoffatomen durch Niederalkyl, Aryl oder Arylniederalkyl substituiertes Amidino, Guanidino, an einem, zwei oder allen drei Stickstoffatomen durch Niederalkyl, Aryl oder Arylniederalkyl substituiertes Guanidino, Oxo, welches nicht am Kohlenstoff gebunden ist, welcher an den A₁ oder A₂ tragenden Stickstoff gebunden ist, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono, Niederalkoxycarbonylhydrazono, Niederalkylthioimino, durch einen der für substituiertes Niederalkyl genannten Reste substituiertes oder vorzugsweise unsubstituiertes Niederalkenyl oder Niederalkinyl, Heterocyclylniederalkyl, worin Heterocyclyl ein unsubstituierter oder insbesondere durch Niederalkyl, Niederalkanoyl, Hydroxy, Niederalkoxy, Halogen, Cyano und/oder Trifluormethyl substituierter, über ein Ringstickstoffatom gebundener Rest ausgewählt aus Pyrrolyl, 2,5-Dihydropyrrolyl, Pyrrolinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl und 1,2-Dihydro- oder 1,2,3,4-Tetrahydroisochinolyl ist, der endständig an Niederalkyl gebunden ist, Niederalkylsulfonyl, Benzolsulfonyl oder Toluolsulfonyl bedeuten; oder worin A₁ und A₂ zusammen Niederalkylen bilden, welches unsubstituiert oder substituiert ist durch bis zu 3 Substituenten ausgewählt aus Niederalkyl, Amino, Aminoniederalkyl, Mono- oder Diniederalkylamino, Mono- oder Diniederalkylaminoniederalkyl, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Cycloalkylamino, Cycloalkylaminoniederalkyl, Phenylniederalkylamino, Phenylniederalkylaminoniederalkyl, Phenylamino, Phenylaminoniederalkyl, Niederalkanoylamino, Phenylniederalkanoylamino, Phenylcarbonylamino, Niederalkanoylaminoniederalkyl, Phenylniederalkanoylaminoniederalkyl, Phenylcarbonylaminoniederalkyl, Hydroxy, Hydroxyniederalkyl, Niederalkoxy oder Niederalkoxyniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkoxy, Phenylniederalkoxyniederalkyl, Niederalkanoyloxy, Niederalkanoyloxyniederalkyl, Mercapto, Mercaptoniederalkyl, Niederalkylthio oder Niederalkylthioniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylthio, Phenylniederalkylthioniederalkyl, Niederalkanoylthio, Niederalkanoylthioniederalkyl, Carboxy, Carboxyniederalkyl, Niederalkoxycarbonyl, Niederalkoxycarbonylniederalkyl, Phenylniederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylnieder-alkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, Thioureido, Thioureidoniederalkyl, an einem oder beiden Stickstoffatomen durch Niederalkyl, Aryl oder Arylniederalkyl substituiertes Thioureido oder Thioureidoniederalkyl, Hydrazino, Hydrazinoniederalkyl, an einem oder beiden Stickstoffatomen durch Niederalkyl, Aryl oder Arylniederalkyl substituiertes Hydrazino oder Hydrazinoniederalkyl, Amidino, Amidinoniederalkyl, an einem oder beiden Stickstoffatomen durch Niederalkyl, Aryl oder Arylniederalkyl substituiertes Amidino oder Amidinoniederalkyl, Guanidino, Guanidinoniederalkyl, an einem, zwei oder allen drei Stickstoffatomen durch Niederalkyl, Aryl oder Arylniederalkyl substituiertes Guanidino oder Guanidinoniederalkyl, Oxo, Oxoniederalkyl, Thioxo, Thioxoniederalkyl, Imino, Iminoniederalkyl, Niederalkylimino, Niederalkyliminoniederalkyl, Niederalkanoylimino, Niederalkanoyliminoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl; Ar₁ und Ar₂ unabhängig voneinander Aryl, Heteroaryl ausgewählt aus Imidazolyl, Triazolyl, Pyridyl, Pyrimidinyl und Triazinyl, welche unsubstituiert oder durch Niederalkyl, Hydroxy, Niederalkoxy, Halogen, Cyano und/oder Trifluormethyl substituiert sind, oder C₃-C₈-Cycloalkyl, welches unsubstituiert oder durch Niederalkoxy oder Hydroxy substituiert ist, bedeuten; X für O oder S steht; Y für Amino, Mono- oder Diniederalkylamino, worin Niederalkyl unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, C₅-C₇-Cycloalkylamino, Phenylniederalkylamino, Phenylamino, Niederalkanoylamino, Halogenniederalkanoylamino, Phenylniederalkanoylamino, Phenylcarbonylamino, Hydrazino, an einem oder beiden Stickstoffatomen durch Niederalkyl, welches durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Aryl oder Arylniederalkyl substituiertes Hydrazino, Hydroxyamino, Niederalkoxyamino, worin der Niederalkylrest durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Niederalkylenamino oder durch -O-, -S- oder -NR'- unterbrochenes Niederalkylenamino, worin R' Niederalkyl oder Niederalkanoyl bedeutet, Niederalkoxy, worin der Niederalkylrest, insbesondere ein- oder zweifach, durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, oder Niederalkylthio, welches unsubstituiert oder im Niederalkylthiorest, insbesondere ein- oder zweifach, durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, oder ausserdem, falls X Sauerstoff und Z substituiertes Methyl bedeuten, für Hydroxy steht; Z für substituiertes Methyl, worin der Substituent aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Niederalkanoylamino, Hydroxy, Niederalkoxy, worin der Niederalkylrest ein- oder zweifach durch Hydroxy, Niederalkoxy, Phenylniederalkoxy, Niederalkanoyloxy, Amino, Niederalkylamino und/oder Diniederalkylamino substituiert oder vorzugsweise unsubstituiert ist, Niederalkanoyloxy, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Cyano, Ureido, 1- oder 3-Mononiederalkylureido, Hydrazino, an einem oder beiden Stickstoffatomen durch Niederalkyl substituiertes Hydrazino, Guanidino, an einem, zwei oder allen drei Stickstoffatomen durch Niederalkyl substituiertes Guanidino, Oxo, Imino, Niederalkylimino, Hydroxyimino, Hydrazono, N-Mono- oder N,N-Diniederalkylhydrazono und Niederalkylthioimino ausgewählt ist, in erster Linie Hydroxymethyl, Niederalkanoyloxymethyl, wie Acetoxymethyl, Formyl oder ferner Hydroxyiminomethyl; Carboxy, Niederalkoxycarbonyl oder Niederalkylthiocarbonyl (Niederalkyl-S-(C=O)-), worin der Niederalkylrest, insbesondere ein- oder zweifach, durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist; Aryloxycarbonyl oder Arylniederalkoxycarbonyl, Carbamoyl, N-Mono- oder N,N-Diniederalkylcarbamoyl, worin Niederalkyl durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkyl-carbamoyl, N,N-Diniederalkylcarbamoyl und/oder Cyano substituiert oder vorzugsweise unsubstituiert ist, C₅-C₇-Cycloalkylaminocarbonyl, N-Phenylniederalkylcarbamoyl, Phenylcarbamoyl, Hydrazinocarbonyl, an einem oder beiden Stickstoffatomen durch Niederalkyl, welches durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Aryl oder Arylniederalkyl substituiertes Hydrazinocarbonyl, Hydroxyaminocarbonyl, Niederalkoxyaminocarbonyl, worin der Niederalkylrest durch Hydroxy, Niederalkoxy, Phenylniederalkoxy, Niederalkanoyloxy, Halogen, Amino, Niederalkylamino, Diniederalkylarnino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Niederalkylenaminocarbonyl oder durch -O-, -S- oder -NR'-, worin R' Niederalkyl oder Niederalkanoyl bedeutet, unterbrochenes Niederalkylenaminocarbonyl, oder ein entsprechendes Thioanaloges der genannten Reste, worin die bindende Carbonylgruppe durch Thiocarbonyl ersetzt ist; oder worin Y und Z zusammen ein bivalentes Radikal der Formel -O-CH₂- bilden, wobei der Sauerstoff anstelle von Y und der Methylenkohlenstoff anstelle von Z gebunden ist;
wobei in den genannten Resten Aryl insbesondere Phenyl bedeutet, das unsubstituiert oder durch einen oder mehrere, vorzugsweise bis zu 5, insbesondere einen oder zwei, vor allem einen, in erster Linie in p-Stellung vorliegenden, oder im Falle von Halogen, vor allem Fluor, bis zu 5, Substituenten aus der Gruppe bestehend aus Niederalkyl, Niederalkenyl, Niederalkinyl, Niederalkylen (angeknüpft an zwei benachbarten C-Atomen), C₅-C₇-Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy oder Niederalkinyloxy; Niederalkylendioxy (angeknüpft an zwei benachbarten C-Atomen); Niederalkanoyloxy, Phenylniederalkanoyloxy oder Phenylcarbonyloxy ( ≙ Benzoyloxy); Mercapto; Niederalkylthio, Phenylniederalkylthio, Phenylthio, Niederalkylsulfinyl [-S(=O)-Niederalkyl], Phenylniederalkylsulfinyl, Benzolsulfinyl, Niederalkylsulfonyl [-S(O₂)-Niederalkyl], Phenylniederalkylsulfonyl oder Benzolsulfonyl; Halogen, Nitro, Amino; Niederalkylamino, C₅-C₇-Cycloalkylamino, Phenylniederalkylamino oder Phenylamino; Diniederalkylamino, N-Niederalkyl-N-phenylamino, N-Niederalkyl-N-phenylniederalkylamino, Niederalkylenamino; Niederalkanoylamino, Phenylniederalkanoylamino oder Phenylcarbonylamino ( ≙ Benzoylamino); Niederalkanoyl, Phenylniederalkanoyl oder Phenylcarbonyl (= Benzoyl); Carboxy; Niederalkoxycarbonyl; Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy und Phenylniederalkoxy, wie Benzyloxy, ausgewählte Reste am Phosphor substituiertes Phosphoryloxy oder durch Phenylen-1,2-dioxy am Phosphor substituiertes Phosphoryloxy, Sulfo (SO₃H); Niederalkoxysulfonyl; Sulfamoyl (SO₂NH₂), N-Niederalkylsulfamoyl, N,N-Diniederalkylsulfamoyl und N-Phenylsulfamoyl, substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; pharmazeutisch annehmbare Salze davon, sofern salzbildende Gruppen vorliegen, und Tautomere davon, sofern tautomerisierbare Gruppen vorliegen, insbesondere pharmazeutische Präparate mit diesen Verbindungen als Wirkstoff, diese Verbindungen zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate zur Behandlung der genannten Erkrankungen.

Stark bevorzugt sind die Verbindungen der Formel I, worin A₁ und A₂ unabhängig voneinander Wasserstoff, Aminoniederalkyl, wie 2-Aminoethyl oder 3-Aminopropyl, Carbamoylniederalkyl, wie 3-Carbamoylpropyl, (3-Niederalkylureido)-niederalkyl, z.B. (3-Methylureido)-niederalkyl, wie 3-(3-Methylureido)-propyl, oder ferner Niederalkenyl, wie Allyl, bedeuten oder worin A₁ und A₂ zusammen 1,2-Ethylen bilden, welches unsubstituiert oder durch einen Substituenten ausgewählt aus Aminoniederalkyl, wie Aminomethyl, Hydroxyniederalkyl, wie Hydroxymethyl, und Hydroxyimino-niederalkyl, wie Hydroxyimino-methyl, substituiert ist, insbesondere 1,2-Ethylen, 1-Hydroxymethyl-1,2-Ethylen, 1-Aminomethyl-1,2-Ethylen oder 1-Hydroxyiminomethyl-1,2-Ethylen; Ar₁ und Ar₂ unabhängig voneinander unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl oder Pentafluorphenyl, 2-, 3- oder 4-Pyridyl, 2-, 4- oder 5-Pyrimidinyl oder 1,3,5-Triazin-2-yl bedeuten, vorzugsweise beide den gleichen Rest, insbesondere Phenyl oder 4-Fluorphenyl; X Sauerstoff bedeutet; Y Amino, Niederalkylamino, wie Methylamino, Phenylamino, Hydroxyniederalkoxy, wie 2-Hydroxyethoxy, oder Hydrazino bedeutet, oder ausserdem, falls Z Hydroxymethyl, Niederalkanoyloxymethyl, Formyl oder Hydroxyiminomethyl bedeutet, Hydroxy bedeutet; Z Hydroxymethyl, Niederalkanoyloxymethyl, wie Acetoxymethyl, Carboxy, Niederalkoxycarbonyl, wie Methoxycarbonyl, Hydroxyniederalkoxycarbonyl, wie 2-Hydroxyethoxycarbonyl, Carbamoyl, Formyl oder ferner Hydroxyiminomethyl, bedeutet; oder worin Y und Z zusammen ein bivalentes Radikal der Formel -O-CH₂- bilden, wobei der Sauerstoff anstelle von Y und der Methylenkohlenstoff anstelle von Z gebunden ist, Salze davon, sofern salzbildende Gruppen vorliegen, oder Tautomere davon, insbesondere Ring-Ketten-Tautomere solcher Verbindungen der Formel I, worin Y Amino oder Niederalkylamino und Z Formyl bedeuten; insbesondere pharmazeutische Präparate mit diesen Verbindungen als Wirkstoff, diese Verbindungen zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate zur Behandlung der genannten Erkrankungen.

Stärker bevorzugt sind die Verbindungen der Formel I, worin A₁ und A₂ je ein Wasserstoffatom bedeuten; Ar₁ und Ar₂ unabhängig voneinander unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl oder Pentafluorphenyl bedeuten, vorzugsweise beide den gleichen Rest, insbesondere Phenyl oder 4-Fluorphenyl; X Sauerstoff bedeutet; Y Amino bedeutet; und Z Carbamoyl bedeutet; Salze davon, sofern salzbildende Gruppen vorliegen; oder pharmazeutische Präparate mit diesen Verbindungen als Wirkstoff, diese Verbindungen zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate zur Behandlung der genannten Erkrankungen.

Stärker bevorzugt sind auch die Verbindungen der Formel I, worin A₁ und A₂ zusammen unsubstituiertes oder durch einen Rest ausgewählt aus Hydroxyniederalkyl, wie Hydroxymethyl, Aminoniederalkyl, wie Aminomethyl, und aus Hydroxyiminoniederalkyl, wie Hydroxyiminomethyl, substituiertes 1,2-Ethylen bedeuten, Ar₁ und Ar₂ unabhängig voneinander unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl oder Pentafluorphenyl bedeuten, vorzugsweise beide den gleichen Rest, insbesondere Phenyl oder 4-Fluorphenyl; X Sauerstoff bedeutet; Y Amino oder Hydroxyniederalkoxy, wie 2-Hydroxyethoxy, bedeutet; und Z Carboxy, Niederalkoxycarbonyl, wie Methoxycarbonyl, Hydroxyniederalkoxycarbonyl, wie 2-Hydroxyethoxycarbonyl, oder Carbamoyl bedeutet, oder Salze davon, sofern salzbildende Gruppen vorliegen; oder pharmazeutische Präparate mit diesen Verbindungen als Wirkstoff, diese Verbindungen zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate zur Behandlung der genannten Erkrankungen.

Besonders bevorzugt sind die Verbindungen der Formel I, worin A₁ und A₂ unabhängig voneinander Wasserstoff oder substituiertes Niederalkyl bedeuten, vorzugsweise Wasserstoff oder Niederalkyl, welches durch bis zu 2 Reste ausgewählt aus Amino, wie in Aminomethyl, -ethyl, -propyl oder -butyl, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkansulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder insbesondere unsubstituiert ist, wie Aminomethyl, Aminoethyl, Aminopropyl oder Aminobutyl, C₅-C₇-Cycloalkylamino, Phenylniederalkylamino oder Phenylamino; Niederalkanoylamino, Phenylniederalkanoylamino oder Phenylcarbonylamino ( ≙ Benzoylamino); Hydroxy, wie in Hydroxymethyl, Hydroxyethyl oder Hydroxypropyl, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert oder vorzugsweise unsubstituiert ist, Phenylniederalkoxy, Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylthio, Niederalkanoylthio, Carboxy, wie in Carboxymethyl, ethyl oder -propyl, Niederalkoxycarbonyl, wie Methoxycarbonyl, Ethoxycarbonyl oder tert-Butoxycarbonyl, vor allem in Methoxy- oder Ethoxycarbonyl-methyl, -ethyl oder -proypl, oder Phenylniederalkoxycarbonyl, wie Benzyloxycarbonyl, Cyano, Carbamoyl, wie in Carbamoylmethyl, Carbamoylethyl oder Carbamoylpropyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, vor allem in 3-Methyl- oder 3-Ethyl-ureidomethyl, -ethyl oder -propyl, Thioureido, an einem oder beiden Stickstoffatomen durch Niederalkyl, Aryl oder Arylniederalkyl substituiertes Thioureido, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z.B. 3-Niederalkylthioureido, wie 3-Methyl- oder 3-Ethylthioureido, vor allem in 3-Methyl- oder 3-Ethyl-thioureido-methyl, -ethyl oder -propyl, Hydrazino, an einem oder beiden Stickstoffatomen durch Niederalkyl, Aryl oder Arylniederalkyl substituiertes Hydrazino, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, Amidino, wie in Amidinomethyl, ethyl oder -propyl, an einem oder beiden Stickstoffatomen durch Niederalkyl, Aryl oder Arylniederalkyl substituiertes Amidino, insbesondere N¹- oder N²-Mono-, N¹,N²- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, Guanidino, wie in Guanidinomethyl, -ethyl oder -propyl, an einem, zwei oder allen drei Stickstoffatomen durch Niederalkyl, Aryl oder Arylniederalkyl substituiertes Guanidino, insbesondere 1-, 2- oder 3-Mono-, 1,1-, 3,3-, 1,2-, 1,3- oder 2,3-Di-, 1,1,2-, 1,1,3-,1,2,3-, 1,3,3- oder 2,3,3-Tri-, 1,1,2,3-, 1,2,3,3-oder 1,1,3,3-Tetra- oder 1,1,2,3,3,-Pentaniederalkyl oder -Phenylniederalkylguanidino, insbesondere 3,3-Diniederalkylguanidino, wie 3,3-Dimethylguanidino oder 3,3-Diethylguanidino, Oxo, welches nicht an dem Kohlenstoff gebunden ist, welcher am A₁ oder A₂ tragenden Stickstoff vorliegt, wie in 2-Oxopropyl oder 3-Oxo-n-butyl, Thioxo, Imino, Niederalkylimino, Niederalkanoylimino, wie Acetylimino, Hydroxyimino (HO-N=), wie in Hydroxyiminomethyl (HO-N=CH-), Hydroxyimino-ethyl oder -propyl, Niederalkoxyimino, wie Methoxyimino, Hydrazono, wie in Hydrazonomethyl, -ethyl oder -propyl, N-Mono- oder N,N-Diniederalkylhydrazono, N-Niederalkanoylhydrazono, wie Acetylhydrazono oder Niederalkoxycarbonylhydrazono, wie tert-Butoxycarbonylhydrazono, und Niederalkylthioimino, wie Methyl- oder Ethylthioimino, vor allem in Methylthioimino- oder Ethylthioimino-methyl, -ethyl oder -propyl, substituiert ist, insbesondere Wasserstoff oder durch einen der genannten Reste endständig substituiertes lineares Niederalkyl, in erster Linie Wasserstoff oder Aminoniederalkyl, wie 2-Aminoethyl oder 3-Aminopropyl, Carbamoylniederalkyl, wie 3-Carbamoylpropyl, oder (3-Niederalkylureido)-niederalkyl, z.B. (3-Methylureido)-niederalkyl, wie 3-(3-Methylureido)-propyl; Ar₁ und Ar₂ unabhängig voneinander für Aryl oder für Heteroaryl stehen; X für O steht; Y für Amino, für Niederalkylamino, wie Methylamino, für Phenylamino oder Hydrazino, oder für substituiertes Niederalkoxy, insbesondere Hydroxyniederalkoxy, wie 2-Hydroxyethoxy, steht, oder ausserdem, falls Z substituiertes Methyl bedeutet, für Hydroxy steht; und Z für substituiertes Methyl, insbesondere Hydroxymethyl, Niederalkanoyloxymethyl, wie Acetoxymethyl, oder Formyl, für Carboxy, für verestertes Carboxy, insbesondere Niederalkoxycarbonyl, wie Methoxycarbonyl, oder Hydroxyniederalkoxycarbonyl, wie 2-Hydroxyethoxycarbonyl, oder (ferner) für Hydroxyiminomethyl, steht; oder worin Y und Z zusammen ein bivalentes Radikal der Formel -O-CH₂- bilden, wobei der Sauerstoff anstelle von Y und der Methylenkohlenstoff anstelle von Z gebunden ist, wobei in den oben genannten Definitionen

Aryl insbesondere Phenyl, das unsubstituiert oder durch einen oder mehrere, vorzugsweise bis zu 5, insbesondere einen oder zwei, vor allem einen, in erster Linie in p-Stellung vorliegenden oder im Falle von Halogen, vor allem Fluor, bis zu 5, Substituenten aus der Gruppe bestehend aus Niederalkyl, Niederalkenyl, Niederalkinyl, Niederalkylen (angeknüpft an zwei benachbarten C-Atomen), C₅-C₇-Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy oder Niederalkinyloxy; oder Niederalkylendioxy (angeknüpft an zwei benachbarten C-Atomen); Niederalkanoyloxy, Phenylniederalkanoyloxy oder Phenylcarbonyloxy ( ≙ Benzoyloxy); Mercapto; Niederalkylthio, Phenylniederalkylthio, Phenylthio, Niederalkylsulfinyl [-S(=O)-Niederalkyl], Phenylniederalkylsulfinyl, Benzolsulfinyl, Niederalkylsulfonyl [-S(O₂)-Niederalkyl], Phenylniederalkylsulfonyl oder Benzolsulfonyl; Halogen, Nitro, Amino; Niederalkylamino, C₅-C₇-Cycloalkylamino, Phenylniederalkylamino oder Phenylamino; Diniederalkylamino, N-Niederalkyl-N-phenylamino, N-Niederalkyl-N-phenylniederalkylamino, Niederalkylenamino, Niederalkanoylamino, Phenylniederalkanoylamino oder Phenylcarbonylamino ( ≙ Benzoylamino); Niederalkanoyl, Phenylniederalkanoyl oder Phenylcarbonyl (= Benzoyl); Carboxy; Niederalkoxycarbonyl; Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy und Phenylniederalkoxy, wie Benzyloxy, ausgewählte Reste am Phosphor substituiertes Phosphoryloxy oder durch Phenylen-1,2-dioxy am Phosphor substituiertes Phosphoryloxy, Sulfo (SO₃H); Niederalkoxysulfonyl; Sulfamoyl (SO₂NH₂), N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; bedeutet, vorzugsweise unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl oder Pentafluorphenyl,
und Heteroaryl vorzugsweise einen über ein Ringkohlenstoffatom verknüpften 5- oder 6-gliedrigen Ring mit bis zu 3 Ringstickstoffatomen, z.B. Imidazolyl, Triazolyl, Pyridyl, Pyrimidinyl oder Triazinyl, wobei der Ring unsubstituiert oder z.B. durch Niederalkyl, Hydroxy, Niederalkoxy, Halogen, Cyano und/oder Trifluormethyl substituiert sein kann, bedeutet; in erster Linie 2-, 3- oder 4-Pyridyl, 2-, 4- oder 5-Pyrimidinyl oder 1,3,5-Triazin-2-yl; und Salze davon, sofern salzbildende Gruppen vorliegen, und/oder Tautomere davon, sofern tautomerisierbare Reste vorliegen.

Ganz besonders bevorzugt sind die Verbindungen der Formel I, worin A₁ und A₂ unabhängig voneinander Wasserstoff oder Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkansulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylamino, Phenylamino, Niederalkanoylamino, Phenylniederalkanoylamino, Phenylcarbonylamino, Hydroxy, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkoxy, Niederalkanoyloxy, Carboxy, Niederalkoxycarbonyl, Phenylniederalkoxycarbonyl, Carbamoyl, N-Niederalkylcarbamoyl, N,N-Diniederalkylcarbamoyl, Ureido, 1- oder 3-Mono- oder 1,3- oder 3,3-Di-niederalkylureido, Oxo, welches nicht am Kohlenstoff gebunden ist, welcher an den A₁ oder A₂ tragenden Stickstoff gebunden ist, Imino und Hydroxyimino (HO-N=) substituiert ist, insbesondere Wasserstoff oder durch einen der genannten Reste endständig substituiertes lineares Niederalkyl, in erster Linie Wasserstoff oder Aminoniederalkyl, wie 2-Aminoethyl oder 3-Aminopropyl, Carbamoylniederalkyl, wie 3-Carbamoylpropyl, oder (3-Niederalkylureido)-niederalkyl, z.B. (3-Methylureido)-niederalkyl, wie 3-(3-Methylureido)-propyl, bedeuten; Ar₁ und Ar₂ unabhängig voneinander für Phenyl, das unsubstituiert oder durch einen oder zwei, vor allem einen, in erster Linie in p-Stellung vorliegenden oder im Falle von Halogen, vor allem Fluor, bis zu 5, Substituenten aus der Gruppe bestehend aus Niederalkyl, Trifluormethyl, Hydroxy, Niederalkoxy, Halogen, Carboxy, Niederalkoxycarbonyl, Carbamoyl, N-Niederalkylcarbamoyl, N,N-Diniederalkylcarbamoyl und Cyano substituiert ist, oder für Heteroaryl ausgewählt aus 2-, 3- oder 4-Pyridyl, 2-, 4- oder 5-Pyrimidinyl und 1,3,5-Triazin-2-yl, welche unsubstituiert oder durch Niederalkyl, Hydroxy, Niederalkoxy, Halogen, Cyano und/oder Trifluormethyl substituiert sind, stehen; X für O steht; Y für Amino, Niederalkylamino, Phenylamino, Hydrazino oder Hydroxyniederalkoxy steht, oder ausserdem, falls Z Hydroxymethyl, Niederalkanoyloxymethyl, Formyl oder Hydroxyiminomethyl bedeutet, für Hydroxy steht; und Z für Hydroxymethyl, Niederalkanoyloxymethyl, Formyl, Carboxy, Niederalkoxycarbonyl oder Hydroxyniederalkoxycarbonyl steht, oder (ferner) für Hydroxyiminomethyl; oder worin Y und Z zusammen ein bivalentes Radikal der Formel -O-CH₂- bilden, wobei der Sauerstoff anstelle von Y und der Methylenkohlenstoff anstelle von Z gebunden ist; und Salze davon, sofern salzbildende Gruppen vorliegen, und/oder Tautomere davon, sofern tautomerisierbare Reste vorliegen.

Von allen bisher genannten Verbindungen der Formel I sind jeweils solche in allererster Linie bevorzugt, worin A₁ und A₂ Wasserstoff bedeuten; oder worin einer der beiden Reste A₁ und A₂ Wasserstoff bedeutet, der andere unsubstituiertes oder durch einen der jeweils genannten Substituenten substituiertes Niederalkyl bedeutet; oder worin A₁ und A₂ zusammen unsubstituiertes oder durch einen der jeweils genannten Substituenten substituiertes Niederalkylen bedeuten; während die übrigen Reste die genannten Bedeutungen haben, pharmazeutisch verwendbare Salze davon, sofern salzbildende Gruppen vorliegen, und Tautomere davon, sofern tautomerisierbare Gruppen vorliegen; oder insbesondere pharmazeutische Präparate mit diesen Verbindungen als Wirkstoff, diese Verbindungen zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate zur Behandlung der genannten Erkrankungen.

Sehr wichtig sind auch die Verbindungen der Formel I mit den Bezeichnungen 4,5-Bis(4-fluoranilino)phthalsäurediamid, Bis(2-hydroxyethyl)-5,8-Diphenyl-5,8-diaza-5,6,7,8-tetrahydro-naphthalin-2,3-dicarboxylat, 4,5-Dianilino-phthalsäurediamid, 4,5-Bis(2-iodanilino)phthalsäurediamid, 4,5-Bis(3-iodanilino)phthalsäurediamid, 4,5-Bis(4-iodanilino)phthalsäurediamid, 4,5-Bis(2-methoxyanilino)phthalsäurediamid, 4,5-Bis(3-methoxyanilino)phthalsäurediamid, 4,5-Bis(4-methoxyanilino)phthalsäurediamid, 4,5-Bis(2-cyananilino)phthalsäurediamid, 4,5-Bis(3-cyananilino)phthalsäurediamid, 4,5-Bis(4-cyananilino)phthalsäurediamid, 4,5-Bis(2-fluoranilino)phthalsäurediamid, 4,5-Bis(3-fluoranilino)phthalsäurediamid, 4,5-Bis(pentafluoranilino)phthalsäurediamid, 4,5-Bis(4-hydroxyanilino)phthalsäurediamid, 4,5-Bis(3-hydroxyanilino)phthalsäurediamid, 4,5-Bis(2-hydroxyaniino)phthalsäurediamid, 4,5-Bis(4-ethylanilino)phthalsäurediamid, 4,5-Bis(3-ethylanilino)phthalsäurediamid, 4,5-Bis(2-ethylanilino)phthalsäurediamid, 4,5-Bis(4-methylanilino)phthalsäurediamid, 4,5-Bis(3-methylanilino)phthalsäurediamid, 4,5-Bis(2-methylanilino)phthalsäurediamid, 5,8-Diphenyl-5,8-diaza-6-hydroxymethyl-5,6,7,8-tetrahydro-naphthalin-2,3-dicarboxamid, 6-Aminomethyl-5,8-diphenyl-5,8-diaza-5,6,7,8-tetrahydro-naphthalin-2,3-dicarboxamid, 5,8-Diphenyl-5,8-diaza-6-hydroxymethyl-5,6,7,8-tetrahydro-naphthalin-2,3-dicarbonsäuremonoamid-mono-methylester, 6-Aminomethyl-5,8-diphenyl-5,8-diaza-5,6,7,8-tetrahydro-naphthalin-2,3-dicarbonsäuremonoamid-monomethylester, 5,8-Bis(4-fluorphenyl)-5,8-diaza-6-hydroxymethyl-5,6,7,8-tetrahydro-naphthalin-2,3-dicarboxamid, 6-Aminomethyl-5,8-bis(4-fluorphenyl)-5,8-diaza-5,6,7,8-tetrahydro-naphthalin-2,3-dicarboxamid, 5,8-Bis(4-fluorphenyl)-5,8-diaza-6-hydroxymethyl-5,6,7,8-tetrahydro-naphthalin-2,3-dicarbonsäuremonoamid-monomethylester, 6-Aminomethyl-5,8-bis(4-fluorphenyl)-5,8-diaza-5,6,7,8-tetrahydro-naphthalin-2,3-dicarbonsäuremonoamid-monomethylester, 5,8-Bis(4-fluorphenyl)-5,8-diaza-6-hydroximinomethyl-5,6,7,8-tetrahydro-naphthalin-2,3-dicarbonsäuremonoamid-monomethylester, 5,8-Bis(4-fluorphenyl)-5,8-diaza-6-hydroximinomethyl-5,6,7,8-tetrahydro-naphthalin-2,3-dicarbonsäuremonoamid-monomethylester, 5,8-Bis(4-fluorphenyl)-5,8-diaza-6-hydroximinomethyl-5,6,7,8-tetrahydro-naphthalin-2,3-dicarboxamid, 5,8-Diphenyl-5,8-diaza-6-hydroximinomethyl-5,6,7,8-tetrahydro-naphthalin-2,3-dicarbonsäuremonoamid-monomethylester oder 5,8-Diphenyl-5,8-diaza-6-hydroximinomethyl-5,6,7,8-tetrahydro-naphthalin-2,3-dicarboxamid, oder Salze davon, sofern salzbildende Gruppen vorliegen.

Am stärksten bevorzugt sind die einzelnen in den Beispielen genannten Verbindungen der Formel I, deren Salze, sofern salzbildende Gruppen vorliegen, und deren Tautomeren, sofern tautomerisierbare Gruppen vorliegen, und die dort genannten Verfahren zu deren Herstellung; oder pharmazeutische Präparate mit diesen Verbindungen als Wirkstoff, diese Verbindungen zur Anwendung in einem therapeutischen Verfahren zur Behandlung des menschlichen oder tierischen Körpers und/oder die Verwendung dieser Verbindungen zur therapeutischen Behandlung des menschlichen oder tierischen Körpers oder zur Herstellung pharmazeutischer Präparate zur Behandlung der genannten Erkrankungen.

Die Verbindungen der Formel I können nach an sich bekannten Verfahren erhalten werden, indem man z.B.
a) zur Herstellung der Verbindungen der Formel I, worin X für O steht, Y für Amino, substituiertes Amino, substituiertes Niederalkoxy oder unsubstituiertes oder substituiertes Niederalkylthio steht und Z für Carboxy, verestertes Carboxy oder amidiertes Carboxy steht, und worin die übrigen Reste die für Verbindungen der Formel I genannten Bedeutungen haben, oder eines Salzes davon, ein reaktionsfähiges Säurederivat einer Dicarbonsäure der Formel II, 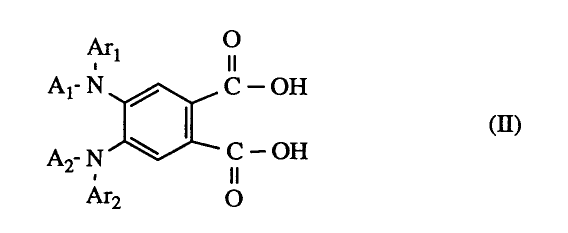 worin A₁, A₂, Ar₁ und Ar₂ die genannten Bedeutungen haben, zur Einführung des Restes Y mit einer Verbindung der Formel III,

   W₁-H (III)

   worin W₁ unsubstituiertes oder substituiertes Amino, substituiertes Niederalkoxy oder unsubstituiertes oder substituiertes Niederalkylthio bedeutet, und/oder zur Herstellung des Restes Z mit Wasser oder einer Verbindung der Formel IV

   W₂-H (IV),

   worin W₂ einen zur Herstellung von verestertem Carboxy oder amidiertem Carboxy Z geeigneten komplementären Rest bedeutet, umsetzt, wobei in den Ausgangsmaterialien der Formeln II, III und IV vorhandene funktionelle Gruppen, die nicht an der Reaktion teilnehmen sollen, erforderlichenfalls in geschützter Form vorliegen, und vor und/oder nach einer der unten genannten zusätzlichen Verfahrensmassnahmen vorhandene Schutzgruppen abspaltet, oder
b) zur Herstellung von Verbindungen der Formel I, worin Z für Hydroxymethyl oder Formyl steht und worin die übrigen Reste die für Verbindungen der Formel I genannten Bedeutungen haben, oder eines Salzes und/oder Tautomeren davon, eine Carbonsäure der Formel V, 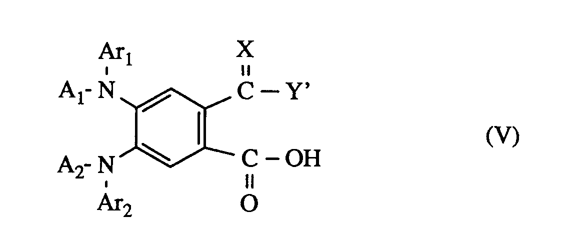 worin Y' Hydroxy oder einen der Reste Y bedeutet, wie für Verbindungen der Formel I definiert, mit der Massgabe, dass X Sauerstoff bedeutet, falls Y' Hydroxy bedeutet, und worin die übrigen Reste die für Verbindungen der Formel I genannten Bedeutungen haben, oder ein reaktionsfähiges Derivat davon reduziert, wobei im Ausgangsmaterial der Formel V vorhandene funktionelle Gruppen, die nicht an der Reaktion teilnehmen sollen, erforderlichenfalls in geschützter Form vorliegen, und vor und/oder nach einer der unten genannten zusätzlichen Verfahrensmassnahmen vorhandene Schutzgruppen abspaltet,

und gewünschtenfalls als zusätzliche Verfahrensmassnahme eine erhältliche Verbindung der Formel I in eine andere Verbindung der Formel I umwandelt, und/oder ein erhältliches Salz in die freie Verbindung oder in ein anderes Salz umwandelt, und/oder eine erhältliche freie Verbindung der Formel I in ein Salz umwandelt und/oder ein erhältliches Gemisch von isomeren Verbindungen der Formel I in die einzelnen Isomeren auftrennt.

In der folgenden näheren Beschreibung der Verfahren haben die Symbole A₁, A₂, Ar₁, Ar₂, X, Y und Z jeweils die für Verbindungen der Formel I genannten Bedeutungen, sofern nichts anderes angegeben ist.

### Zu a) Herstellung von Dicarbonsäurederivaten

Die Dicarbonsäuren der Formel II liegen als reaktionsfähige Derivate vor, wobei eine oder beide Carboxygruppen derivatisiert sein können, vorzugsweise beide, beispielsweise als aktiviertes Säurederivat, als von der freien Carboxyverbindung abgeleiteter Ester, z.B. als Niederalkylester, als reaktionsfähiges Anhydrid oder als reaktionsfähiges cyclisches Imid. Die reaktionsfähigen Derivate können ferner auch in situ gebildet werden. Es können beide Carboxygruppen gleichzeitig (Verbindung der Formel III ist gleich der Verbindung der Formel IV; ergibt Verbindungen der Formel I, worin -(C=X)-Y gleich ist wie Z) oder der Reihe nach (die Verbindung der Formel III und die der Formel IV sind gleich oder verschieden; ergibt auch Verbindungen, worin -(C=X)-Y eine andere Bedeutung hat als Z) umgesetzt werden.

Als aktiviertes Säurederivat kommt beispielsweise eine Verbindung der Formel IIa 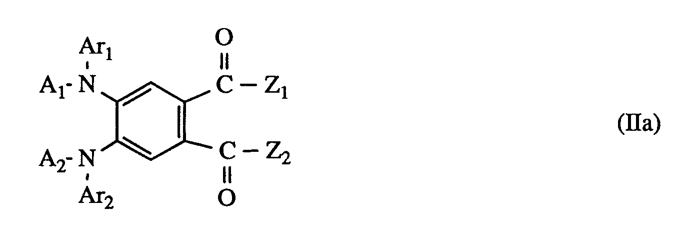
in Betracht, worin Z₁ und/oder Z₂ Hydroxy oder insbesondere reaktionsfähig aktiviertes Hydroxy bedeutet, wobei höchstens einer der beiden Reste Z₁ und Z₂ für Hydroxy steht. Die freie Carbonsäure der Formel II kann beispielsweise durch starke Säuren, wie Halogenwasserstoff-, Schwefel-, Sulfon-, oder Carbonsäure oder saure Ionenaustauscher, z.B. durch Chlor-, Bromwasserstoff- oder Iodwasserstoffsäure, Schwefelsäure, eine gegebenenfalls, z.B. durch Halogen, substituierte Alkancarbonsäure oder durch eine Säure der Formel II, vorzugsweise mit einem Überschuss der Säure der Formel II, erforderlichenfalls unter Bindung von entstehendem Reaktionswasser durch wasserbindende Mittel, unter azeotroper Abdestillation des Reaktionswassers oder unter extraktiver Veresterung, durch Säureanhydride, insbesondere anorganische Säureanhydride, beispielsweise Carbonsäureanhydride, z.B. Niederalkancarbonsäureanhydride (ausser Ameisensäureanhydrid), wie. Acetanhydrid, oder durch geeignete Aktivierungs- oder Kupplungsreagentien der nachstehend aufgeführten Art, insbesondere auch in situ, aktiviert werden. Z₁ und/oder Z₂ kann auch für eine Azidogruppe (erhältlich beispielsweise durch Umsetzung eines entsprechenden Säureesters über das entsprechende Hydrazid und dessen Behandlung mit salpetriger Säure); Halogen, insbesondere Chlor oder Brom (erhältlich beispielsweise durch Umsetzung mit organischen Säurehalogeniden, insbesondere mit Oxalyldihalogeniden, wie Oxalyldichlorid, oder vor allem mit anorganischen Säurehalogeniden, z.B. mit Säurehalogeniden des Phosphors oder Schwefels, wie Phosphortrichlorid, Phosphortribromid, Phosphorpentachlorid, Phosphorpentabromid, Phosphoroxidchlorid, Phosphoroxidbromid, Thionylchlorid oder Thionylbromid); für Cyanmethoxy, Nitrophenoxy, wie 4-Nitrophenoxy oder 2,4-Dinitrophenyoxy, oder Polyhalogenphenoxy, wie Pentachlorphenoxy (herstellbar z.B. durch Behandeln der entsprechenden Säure mit Chloracetonitril in Gegenwart einer Base, bzw. durch Umsetzung der Säure mit dem entsprechenden Nitrophenol oder Polyhalogenphenol in Gegenwart eines Kondensationsmittels, wie N,N'-Dicyclohexylcarbodiimid oder insbesondere 2-Ethoxy-1-ethoxycarbonyl-1,2-dihydrochinolin); oder für ein asymmetrisches Säureanhydrid stehen, welches beispielsweise durch Einwirkung eines Salzes, z.B. eines Alkalimetallsalzes, wie des Natrium- oder Kaliumsalzes, einer Säure der Formel II oder ihres Reaktionspartners, vorzugsweise einer Niederalkancarbonsäure, wie Essigsäure, auf ein jeweils komplementäres Säurehalogenid, insbesondere im Falle der Reaktion mit einem Salz einer Carbonsäure der Formel II ein Carbonsäurehalogenid, z.B. -Chlorid, oder im Falle der Reaktion eines Carbonsäurehalogenides der Formel IIa, worin Z₁ und Z₂ Halogen, z.B. Chlor oder Brom, bedeuten, mit einem Salz einer Niederalkancarbonsäure, insbesondere Natrium- oder Kaliumacetat, erhalten werden kann. Z₁ und Z₂ bedeuten bevorzugt Halogen, wie Chlor oder Brom, sowie Acyloxy, z.B. Niederalkanoyloxy, wie Acetyloxy.

Verbindungen der Formel II können aus den unten genannten Verbindungen der Formel IIb hergestellt werden durch Hydrolyse unter Abspaltung von R₁ und R₂, beispielsweise durch Hydroxybasen, z.B. Alkali- oder Erdalkalimetallhydroxide, wie Natrium-, Kalium- oder Bariumhydroxid, in wasserfreien oder wässrigen Lösungsmitteln oder Lösungsmittelgemischen, wie wässrigen alkoholischen Lösungen, z.B. Methanol/Wasser, bei erhöhten Temperaturen, z.B. von 40 °C bis zur Rückflusstemperatur, insbesondere von 10 °C unter der Rückflusstemperatur bis zur Rückflusstemperatur. Sind die Niederalkylreste R₁ und/oder R₂ am bindenden Kohlenstoffatom verzweigt, so ist auch saure Hydrolyse möglich, beispielsweise unter Verwendung von Schwefelsäure oder Halogenwasserstoffsäuren, wie Chlorwasserstoffsäure. Auch die Herstellung aus Analogen der Verbindungen der Formel IIb, worin anstelle von R₁ und/oder R₂ Carboxyschutzgruppen, beispielsweise die unten genannten, vorliegen, durch Schutzgruppenabspaltung ist möglich. Die Analogen der Verbindungen der Formel IIb, worin anstelle von R₁ und/oder R₂ Carboxyschutzgruppen vorliegen, werden beispielsweise erhalten, indem man unten anstelle der Verbindungen der Formel V analoge Verbindungen verwendet, worin anstelle von R₁ und/oder R₂ eine Carboxyschutzgruppe vorliegt (herstellbar analog wie die Verbindungen der Formel V) und hieraus die den Verbindungen der Formel IIb analogen Verbindungen mit Carboxyschutzgruppen anstelle von R₁ und/oder R₂ herstellt durch analoge Reaktionen, wie unten für Verbindungen der Formel IIb selbst beschrieben. Durch Schutzgruppenabspaltung unter geeigneten, vorzugsweise den unten genannten Bedingungen zur Schutzgruppenabspaltung, können aus erhaltenen Analogen der Verbindungen der Formel IIb die Verbindungen der Formel II gewonnen werden. Aus diesen können, wie oben beschrieben, die freien Verbindungen der Formel IIa hergestellt werden.

Ester von Verbindungen der Formel II sind vorzugsweise solche der Formel IIb, 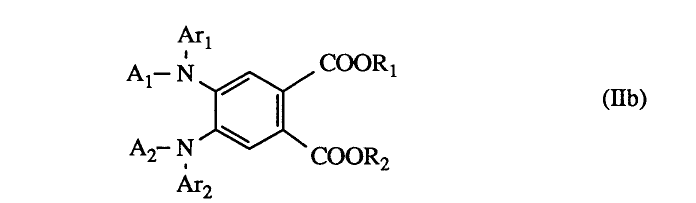
worin Ar₁, Ar₂, A₁ und A₂ die unter Formel I angegebene Bedeutung haben, R₁ Niederalkyl, wie Methyl, und R₂ Wasserstoff oder Niederalkyl, wie Methyl, bedeutet. Die Umsetzung von Verbindungen der Formel IIb, insbesondere den entsprechenden Mono- oder Diniederalkylestern, mit einer Verbindung der Formel III und/oder IV zu den genannten Verbindungen der Formel I erfolgt in geeigneten Lösungsmitteln, insbesondere polaren Lösungsmitteln, beispielsweise Alkoholen, wie Methanol oder Ethanol oder in Ethern, wie Tetrahydrofuran, Nitrilen, wie Acetonitril oder Gemischen davon, (bevorzugt, wenn Amidierung der Ester mit Aminoverbindungen der Formel III und/oder IV durchgeführt wird), oder (bevorzugt bei Umesterung mit Alkoholen der Formel III und/oder Alkoholen der Formel IV, wobei auch Ammoniak zugegen sein kann) mit oder vorzugsweise ohne inerte Lösungsmittel in einer bei der Reaktionstemperatur flüssigen Lösung oder Schmelze mit einer entsprechenden Verbindung der Formel III und/oder IV, erforderlichenfalls unter Schutzgas oder in einem Autoklaven, bei Temperaturen zwischen 30 °C und 240 °C, beispielsweise zwischen 50 °C und der Schmelztemperatur des entsprechenden Reaktionsgemisches, oder zwischen 50 °C und der Siedetemperatur des entsprechenden Reaktionsgemisches, z.B. bei etwa 50 bis 130 °C, vorzugsweise bei rund 120 °C in einem Autoklaven, bei Amidierung mit Aminoverbindungen der Formel III udn/oder IV in Ab- oder vorzugsweise Anwesenheit eines Kondensationsmittels, wie oben bei der Umsetzung einer Verbindung der Formel IIa genannt, insbesondere von 2-Ethoxy-1-ethoxycarbonyl-1,2-dihydrochinolin, in An- oder Abwesenheit von Basen, wie Hydrogencarbonat oder Carbonat (kann als Carbonat oder Hydrogencarbonat einer Aminoverbindung der Formel III und/oder IV vorliegen) bei Temperaturen zwischen 0 und 50 °C, insbesondere bei Raumtemperatur, sie erfolgt, sofern die Verbindungen der Formeln III und IV nicht identisch sind, beispielsweise in zwei aufeinanderfolgenden Reaktionsschritten erst mit einer, dann mit der anderen der Verbindungen der Formeln III und IV (wobei die gewünschte Verbindung der Formel I von eventuellen Nebenprodukten abgetrennt werden kann); oder insbesondere durch gleichzeitigen Ersatz von R₁ und R₂, sofern diese jeweils Niederalkyl bedeuten und die Verbindungen der Formeln III und IV identisch sind.

Die Ausgangsmaterialien der Formel IIb werden z.B. dadurch hergestellt, dass man ein Cyclohexadien der Formel V 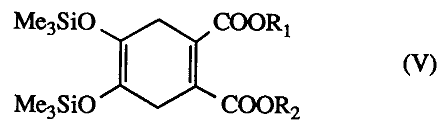
worin Me für Methyl steht (alternativ könnten auch andere Niederalkylreste vorliegen) und worin R₁ und R₂ die für Verbindungen der Formel IIb genannten Bedeutungen haben, mit einem Anilin der Formel VI

AHN-Ar (VI)

worin A insbesondere Wasserstoff, unsubstituiertes oder substituiertes Niederalkyl, unsubstituiertes oder substituiertes Niederalkenyl, unsubstituiertes oder substituiertes Niederalkinyl, Heterocyclylniederalkyl, Aryl, Acyl, Niederalkylsulfonyl oder Arylsulfonyl bedeutet, wie oben für A₁ oder A₂ definiert, und worin Ar Aryl, Heteroaryl oder unsubstituiertes oder substituiertes Cycloalkyl bedeutet, wie oben für Ar₁ oder Ar₂ definiert, oder mit einer Dianilinoniederalkylenverbindung der Formel VI',

Ar₁-NH-K-NH-Ar₂ (VI')

worin Ar₁ und Ar₂ die für Verbindungen der Formel I genannten Bedeutungen haben, z.B. Phenyl bedeuten und K substituiertes oder vorzugsweise unsubstituiertes Niederalkylen bedeutet, wie oben für aus A₁ und A₂ zusammen gebildetem unsubstituiertem oder substituiertem Niederalkylen definiert, unter Säurekatalyse, beispielsweise in einer Niederalkancarbonsäure, wie Essigsäure, bei Temperaturen zwischen 80 °C und der Rückflusstemperatur, z.B. zwischen 100 und etwa 140 °C, umsetzt [s. Matlin, Stephen A. and Barron, Kenneth, J. Chem. Res. Synop. 8, 246-247 (1990)]. Hierbei sind in den Ausgangsmaterialien funktionelle Gruppen, die nicht an der Reaktion teilnehmen sollen, erforderlichenfalls geschützt. Vorhandene Schutzgruppen können auf einer geeigneten Reaktionsstufe entfernt werden. Schutzgruppen, ihre Einführung und ihre Abspaltung sind unten beschrieben. Substituenten an den Resten Ar können auch nach der Kondensation durch Methoden der klassischen Aromaten- bzw. Heterocyclenchemie oder durch enzymatische Methoden (z.B. 4-Hydroxylierungen) eingeführt werden.

Die Herstellung der Verbindungen der Formel V erfolgt z.B. via einer Diels-Alder-Reaktion aus einem 2,3-Bis(triniederalkylsilyloxy)butadien und einem Acetylendicarbonsäure-diniederalkylester, ist ebenfalls in der angegebenen Literatur (Matlin et al.) beschrieben und analog wie dort durchführbar.

Zur Herstellung von unsymmetrischen Verbindungen der Formel IIb, worin A₁ und A₂ und/oder Ar₁ und Ar₂ verschieden sind, können beispielsweise Verbindungen der Formel V mit zwei unterschiedlichen Verbindungen der Formel VI - z.B. stufenweise - umgesetzt und die gewünschten Verbindungen der Formel IIb durch eine chromatographische Trennung, z.B. an Kieselgel, isoliert werden.

Weiterhin können zur Herstellung von Verbindungen der Formel IIb, worin die Reste die genannten Bedeutungen ausser aus A₁ und A₂ zusammen gebildetem unsubstituiertem oder substituiertem Niederalkylen haben, beispielsweise Verbindungen der Formel IIb, worin anstelle von A₁ und A₂ Wasserstoff steht,
1) mit einem Reagens der Formel VII umgesetzt werden,

   W₃-L (VII)

   worin W₃ unsubstituiertes oder substituiertes Niederalkyl, unsubstituiertes oder substituiertes Niederalkenyl, unsubstituiertes oder substituiertes Niederalkinyl oder Heterocyclylniederalkyl bedeutet, wie oben bei der Definition von A₁ und/oder A₂ genannt, umgesetzt werden, wobei L im Falle von substituiertem Niederalkenyl oder substituiertem Niederalkinyl vorzugsweise an ein Kohlenstoffatom gebunden ist, von dem keine Mehrfachbindung ausgeht, und so gewählt ist, dass die Umsetzung durch Substitution schneller erfolgt als die Addition an die Mehrfachbindung; und L eine nukleofuge Gruppe, vorzugsweise Toluolsulfonyloxy oder Halogen, wie Chlor, Brom oder Jod, oder falls der Rest W₃ 2 oder mehr Kohlenstoffatome enthält, Oxa (-O-) oder Thia (-S-), welches an zwei vizinale Kohlenstoffatome gebunden ist (wobei ein Oxiran oder Thiiran gebildet wird, welches bei Alkylierung, die insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, oder einem Harnstoffderivat, wie 1,3-Dimethyl-3,4,5,6-tetrahydro-2(1H)-pyrimidinon (DMPU), erfolgt, und bei anschliessender Hydrolyse unter Entstehung einer 2-Hydroxy- oder 2-Mercaptoniederalkylgruppe reagiert), oder Aza (-NH-), welches an zwei vizinale Kohlenstoffatome gebunden ist (wobei ein Aziran gebildet wird, welches bei Umsetzung, die insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, oder einem Harnstoffderivat, wie 1,3-Dimethyl-3,4,5,6-tetrahydro-2(1H)-pyrimidinon (DMPU), erfolgt, und bei anschliessender Hydrolyse unter Bildung einer 2-Aminoniederalkylgruppe reagiert), wobei funktionelle Gruppen in Ausgangsmaterialien, die nicht an der Reaktion teilnehmen sollen, frei oder in geschützter Form vorliegen, und vorhandene Schutzgruppen auf geeigneten Reaktionsstufen erforderlichenfalls abgespalten werden.
   Die Umsetzung findet vorzugsweise in Gegenwart von einer starken Base statt, wie einem Alkalimetallhydrid, z. B. Natriumhydrid, einem Alkalimetallamid, wie Natriumamid, oder einem Alkalimetall-diniederalkylamid, wie Lithiumdiispropylamid, insbesondere von Natriumhydrid oder Natriumamid, welches z.B. als Dispersion in Öl zugesetzt werden kann, unter Verwendung eines gegenüber der molaren Menge der Verbindung der Formel IIb, worin anstelle von A₁ und A₂ je ein Wasserstoffatom steht, äquimolaren Menge oder eines Überschusses der Base, beispielsweise der 1- bis 5-fachen molaren Menge, vor allem der 1- bis 2-fachen molaren Menge, bei Temperaturen zwischen Raumtemperatur und der Rückflusstemperatur des Reaktionsgemisches, insbesondere zwischen etwa 80 und etwa 100 °C, in aprotischen, insbesondere polaren Lösungsmitteln, wie Säureamiden, z. B. Dimethylformamid, 1,3-Dimethyl-3,4,5,6-tetrahydro-2(1H)-pyrimidinon (DMPU) oder Hexamethylphosphorsäuretriamid, oder Gemischen von solchen Lösungsmitteln, in An- oder Abwesenheit von Schutzgas, wie Argon oder Stickstoff, wobei im Falle der Verwendung von Alkalimetallamiden als Basen entstehender Ammoniak durch Anlegen eines Vakuums, z. B. von 0,1 bis 100, insbesondere von 0,5 bis 10 Torr, entfernt wird; und/oder
2) mit einer Säure der Formel VII',

   W₃'-L' (VII')

   worin W₃' Acyl, Niederalkylsulfonyl oder Arylsulfonyl bedeutet, wie oben bei der Definition von A₁ und/oder A₂ genannt, oder einem reaktionsfähigen Derivat hiervon umgesetzt werden, wobei L' Wasserstoff oder einen Rest wie oben für Z₁ in Verbindungen der Formel IIa definiert bedeutet, insbesondere Halogen, unter Bedingungen analog den bei der Umsetzung von Verbindungen der Formel II mit Verbindungen der Formel III und/oder IV beschriebenen.

Sollen Verbindungen der Formel I hergestellt werden, worin nur einer der Reste A₁ und A₂ die oben genannten Bedeutungen ausser Wasserstoff hat, während der andere Wasserstoff bedeutet, wird die Reaktion mit einer molaren Menge der Verbindung der Formel VII oder VII' durchgeführt, welche vorzugsweise der 0,2- bis 2-fachen molaren Menge, z. B. der 1- bis 1,6-fachen molaren Menge, gegenüber der molaren Menge der Verbindung der Formel IIb, worin anstelle von A₁ und A₂ Wasserstoff steht, entspricht. Sollen beide Wasserstoffatome in der Verbindung der Formel IIb, worin für A₁ und A₂ Wasserstoff steht, durch die bei der Definition von A₁ und A₂ genannten Reste ausser Wasserstoff ersetzt werden, so wird vorzugsweise ein Überschuss, z. B. ein 2- bis 10-, insbesondere ein etwa zwei- bis dreifacher Überschuss der Verbindung der Formel VII oder VII' eingesetzt.

Die Verbindungen der Formeln V, VI, VI', VII und VII' sind bekannt, kommerziell erhältlich oder nach an sich bekannten Verfahren herstellbar.

In den Verbindungen der Formel VII ist Niederalkyl, Niederalkenyl oder Niederalkinyl W₃ vorzugsweise unsubstituiert oder substituiert durch geschütztes Amino, insbesondere in Aminoniederalkyl, z.B. Phthalimidoniederalkyl, wie Phthalimidopropyl, beispielsweise durch Niederalkoxycarbonyl, wie tert-Butyloxycarbonyl, geschütztes und im Mononiederalkylrest unsubstituiertes unsubstituiertes oder wie oben substituiertes (und dann erforderlichenfalls an den Substituenten geschütztes) Mononiederalkylamino, insbesondere in entsprechend geschütztem und substituiertem Mononiederalkylaminoniederalkyl, in den beiden N-Niederalkylresten unsubstituiertes oder wie oben definiert substituiertes (und dann erforderlichenfalls an Substituenten geschütztes) Diniederalkylamino, insbesondere in entsprechendem unsubstituiertem oder subtituiertem Diniederalkylaminoniederalkyl, N-geschütztes Cycloalkylamino, insbesondere in N-geschütztem Cycloalkylaminoniederalkyl, N-geschütztes Phenylniederalkylamino, insbesondere in N-geschütztem Phenylniederalkylaminoniederalkyl, N-geschütztes Phenylamino, insbesondere in N-geschütztem Phenylaminoniederalkyl, Acylamino, wie in Acylaminoniederalkyl, geschütztes Hydroxy, insbesondere in Hydroxyniederalkyl, worin die Hydroxygruppe geschützt vorliegt, Niederalkoxy, worin der endständige Niederalkylrest unsubstituiert oder wie oben substituiert (und dann erforderlichenfalls mit geschützten Substituenten) vorliegt, wie in entsprechendem substituiertem geschütztem oder unsubstituiertem Niederalkoxyniederalkyl, Phenylniederalkoxy, insbesondere in Phenylniederalkoxyniederalkyl, Acyloxy, insbesondere in Acylniederalkoxyniederalkyl, geschütztes Mercapto, insbesondere in Mercaptoniederalkyl, worin die Mercaptogruppe in geschützter Form vorliegt, Niederalkylthio, worin der endständige Niederalkylrest unsubstituiert oder wie oben substituiert (und dann erforderlichenfalls mit geschützten Substituenten) vorliegt, insbesondere in entsprechend substituiertem geschütztem oder unsubstituiertem Niederalkylthioniederalkyl, Phenylniederalkylthio, wie in Phenylniederalkylthioniederalkyl, Acylthio, wie in Acylthioniederalkyl, geschütztes Carboxy, wie in geschütztem Carboxyniederalkyl, verestertes Carboxy, wie in verestertem Carboxyniederalkyl, Cyano, wie in Cyanoniederalkyl, Oxo, wie in Oxoniederalkyl (erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann), und/oder Thioxo, insbesondere in Thioxoniederalkyl (erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann), oder Heterocyclyl, wie in erforderlichenfalls geschütztem Heterocyclylniederalkyl.

Benutzt man anstelle einer Verbindung der Formel VII eine entsprechende Verbindung, in der am Rest W₃, welcher hier wie oben für A₁ und A₂ definiertes unsubstituiertes oder substituiertes Niederalkyl bedeutet, zusätzlich zu einem der genannten nukleofugen Reste L ein Halogenatom vorliegt, so kann durch Eliminierung von Halogenwasserstoff in Gegenwart der zu Reaktion verwendeten Base, z. B. von Natriumamid, welche beispielsweise im Überschuss eingesetzt wird, unter den oben genannten Reaktionsbedingungen die entsprechende unsubstituierte oder substituierte Niederalkenylverbindung erhalten werden. Analog können unsubstituierte oder substituierte Niederalkinylreste aus entsprechenden Analogen der Verbindung der Formel VII erhalten werden, in denen zusätzlich zu einem der genannten nukleofugen Reste L zwei weitere Halogenatome vorliegen.

In erhältlichen Verbindungen der Formel IIb können Carboxygruppen, die in A₁ und/oder A₂ als Substituent vorliegen, in Carbamoyl-, N-Mono- oder N,N-Diniederalkylcarbamoyl-, N-Hydroxycarbamoyl- oder N-Phenylcarbamoylgruppen (auch in N-Aryl- und N-Arylniederalkylcarbamoylgruppen) umgewandelt werden, beispielsweise durch Umsetzung mit Ammoniak, einem Niederalkylamin oder einem Diniederalkylamin, Hydroxylamin oder Phenylamin, in Gegenwart eines Kondensationsmittels, z.B. einem Carbodiimid, wie Dicyclohexylcarbodiimid oder einem polaren Derivat davon, in polaren organischen Lösungsmitteln, wie Ethanol, oder in Gegenwart von einem N.N'-Carbonyldiazolid, wie N.N'-Dicarbonylimidazol (vgl. H. A. Staab, Angew. Chem. 74, 407-423 (1962)) in inerten organischen Lösungsmitteln, z.B. chlorierten Kohlenwasserstoffen, wie Methylenchlorid oder Chloroform, oder in Ethern, wie Diethylether, Tetrahydrofuran oder Dioxan, über das entsprechende Carbonsäureazolid. Man erhält so die entsprechenden Verbindungen der Formel IIb, worin A₁ und/oder A₂ Carbamoyl, N-Mono- oder N,N-Diniederalkylcarbamoyl, N-Hydroxycarbamoyl oder N-Phenylcarbamoyl enthält. Entsprechende Thiocarbamoylsubstituenten in A₁ und A₂ können aus Carboxy durch Umwandlung in ein Carbonylhalogenid, z. B. mit einem anorganischen Säurehalogenid, wie Phosphortrichlorid, Phosphorpentachlorid oder Thionylchlorid, oder einem organischen Säurehalogenid, wie Oxalyldichlorid, und anschliessende Umsetzung z.B. mit Phosphorpentachlorid, Schwefelwasserstoff und Ammoniak oder den zuletzt genannten Aminen erhalten werden.

In Verbindungen der Formel IIb, worin A₁ und/oder A₂ z.B. ein Hydroxyniederalkyl bedeuten, kann durch nukleophile Substitution die Hydroxygruppe in eine Hydrazino-, durch Niederalkyl, Aryl und/oder Arylniederalkyl N-substituierte Hydrazinogruppe, eine Guanidinogruppe oder eine durch Niederalkyl, Aryl und/oder Arylniederalkyl N-substituierte Guanidinogruppe umgewandelt werden. Beispielsweise kann eine Hydroxyverbindung durch Umsetzung mit aromatischen Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, in Ab- oder vorzugsweise Anwesenheit von geeigneten Basen, z.B. tertiären Stickstoffbasen, wie Triethylamin oder N-Methylmorpholin, in den entsprechenden Ester der aromatischen Sulfonsäure überführt werden und dann dieser Ester mit Hydrazin, Guanidin, den entsprechend substituierten Derivaten, oder Salzen davon, wobei auch Schutzgruppen vorliegen können, unter den Bedingungen der nukleophilen Substitution umgesetzt werden, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel, gegebenenfalls (z.B. zur Umsetzung von an Aryl gebundenem Stickstoff) in inerten organischen Lösungsmitteln, wie Dimethylformamid oder 1,3-Dimethyl-3,4,5,6-tetrahydro-2(1H)-pyrimidinon, unter Zugabe starker Basen, wie Natriumamid oder Natriumhydrid. Erforderlichenfalls werden vorhandene Schutzgruppen abgespalten. Man erhält Verbindungen der Formel IIb, worin A₁ und/oder A₂ Substituenten ausgewählt aus Hydrazino, an einem oder beiden Stickstoffatomen durch Niederalkyl, Aryl oder Arylniederalkyl substituiertem Hydrazino, Guanidino und an einem, zwei oder allen drei Stickstoffatomen durch Niederalkyl, Aryl oder Arylniederalkyl substituiertem Guanidino enthält. Diejenigen Verbindungen der Formel IIb, worin Substituenten ausgewählt aus Hydrazino und am endständigen N-Atom durch Niederalkyl, Aryl und/oder Arylniederalkyl substituiertem Hydrazino vorliegen, kann man auch erhalten ausgehend von einer entsprechenden Oxoverbindung der Formel IIb durch deren Umsetzung mit Stickstoffbasen ausgewählt aus Hydrazin und durch Niederalkyl, Aryl und/oder Arylniederalkyl an einem der beiden N-Atome bis zu zweifach substituiertem Hydrazin, wie weiter unten für die Umsetzung einer Oxoverbindung mit Stickstoffbasen beschrieben, und anschliessende Reduktion einer erhaltenen entsprechenden Iminoverbindung, vorzugsweise durch katalytische Hydrierung mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, wie Niederalkanolen, z.B. Methanol oder Ethanol, oder Gemischen davon, z.B. Methanol/Tetrahydrofuran-Gemischen, bei Temperaturen zwischen -20 und 60 °C, z.B. zwischen 0 und 40 °C, wie bei Raumtemperatur.

In Verbindungen der Formel IIb, worin A₁ und/oder A₂ z.B. ein Cyanoniederalkyl bedeuten, können Cyanogruppen z.B. durch partielle Hydrolyse, im Sinne einer Graf-Ritter-Reaktion oder über Imino-niederalkylester-Salze in Carbamoyl- oder N-Niederalkylcarbamoylgruppen in Verbindungen der Formel IIb überführt werden. Die Bedingungen bei der Hydrolyse des Cyano-Zwischenproduktes können so gewählt werden, dass die Reaktion auf der Stufe des Amids abgebrochen wird. Zu diesem Zweck ist insbesondere die Hydrolyse mit Säuren geeignet, wobei z.B. 80%-ige Schwefelsäure (unter Erwärmen), Polyphosphorsäure (bei 110-150 °C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen. Auch mit Hilfe der Graf-Ritter-Reaktion gelingt die Herstellung N-monoalkylierter Amide der Formel IIb aus den entsprechenden Nitrilen. Hierzu setzt man die Nitrile in Gegenwart einer starken Säure, vornehmlich 85-90%-iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen oder Alkoholen. Die Imino-niederalkylester der Formel IIb erhält man z.B. durch säurekatalysierte Anlagerung von Alkoholen an die Nitrilvorläufer. Diese Anlagerung kann alternativ durch Basen, z.B. Alkoholate wie Natriummethoxid, katalysiert werden. Setzt man anstelle von Alkoholen entsprechende Mercaptane ein, beispielsweise in Gegenwart von Stickstoffbasen, wie Triethylamin oder N-Methylmorpholin, so erhält man die entsprechenden Imino-niederalkylthioester. Aus den Iminoniederalkylestern erhält man die Carbamoylderivate, aus den Imino-niederalkylthioestern die entsprechenden Thiocarbamoylderivate im Sinne einer Pinner-Spaltung durch thermischen Zerfall der Iminoester-Salze bei Temperaturen oberhalb von etwa 80 °C. Die Thiocarbamoylverbindungen können auch direkt erhalten werden durch Umsetzung von Cyanogruppen mit Schwefelwasserstoff analog der partiellen Hydrolyse, beispielsweise in Gegenwart von tertiären Aminen, wie Triethylamin.

Verbindungen der Formel IIb, worin A₁ und/oder A₂ z.B. ein Amidinoniederalkyl oder ein an einem Stickstoffatom durch bis zu zwei Reste ausgewählt aus Niederalkyl, Aryl und Arylniederalkyl substituiertes Amidinoniederalkyl bedeuten, lassen sich durch Umsetzung einer entsprechenden Imino-niederalkylester- oder Imino-niederalkylthiolestervorstufe (als Säureadditionssalz, z.B. -(C=NH)-OC₂H₅·HCl bzw. -C(=NH)-SC₂H₅·HI, wie oben beschrieben hergestellt aus einer entsprechenden Cyanoverbindung) mit Ammoniak oder dem entsprechenden Niederalkyl-, Aryl- oder Arylniederalkylamin herstellen. Die Cyanovorstufen können beispielsweise auch durch Umsetzung mit einem Alkalimetallamid, oder durch Reaktion mit einem entsprechenden Ammoniumsalz, z.B. einem entsprechenden Ammoniumhalogenid, in die freien, mono- oder disubstituierten Amidine überführt werden. Verbindungen der Formel IIb, worin A₁ und/oder A₂ ein an allen beiden Stickstoffatomen durch Aryl, Arylniederalkyl oder Niederalkyl substituiertes Amidino enthalten, lassen sich aus den (analog wie oben für Niederalkylcarbamoyl beschrieben herstellbaren) Verbindungen herstellen, worin in Formel IIb anstelle von Carbamoyl in A₁ und/oder A₂ durch Niederalkyl, Aryl oder Arylniederalkyl N-substituiertes Carbamoyl als Substituent enthalten ist, z. B. durch Umsetzung mit POCl₃ oder PCl₅ in die entsprechenden Imidsäurechloride (z.B. -(C=NH-niederalkyl)-Cl), welche nach Reaktion mit Ammoniak bzw. einem primären oder sekundären Amin substituierte Amidine der Formel IIb ergeben (vgl. Chem. Abstr. 81, 91186a (1974)).

In erhältlichen Verbindungen der Formel IIb können Aminogruppen, die in A₁ und/oder A₂ als Substituent vorliegen, in Ureido- oder an einem oder beiden Stickstoffatomen durch je bis zu einen Rest ausgewählt aus Niederalkyl, Aryl und Arylniederalkyl substituiertes Ureido umgewandelt werden, indem man Verbindungen der Formel IIb, worin z.B. A₁ und/oder A₂ Aminoniederalkyl oder N-Mononiederalkylaminoniederalkyl bedeuten, oder in denen anstelle von einem oder beiden der Reste A₁ und A₂ ein Arylaminoniederalkyl und/oder ein Arylniederalkylaminoniederalkyl stehen (herstellbar z. B. durch Umsetzung von Verbindungen der Formel IIb, worin anstelle von A₁ oder A₂ Wasserstoff steht, mit Analogen der Verbindungen der Formel VII, worin ausser der nukleofügen Gruppe L noch eine weitere nukleofuge Gruppe, z.B. Halogen, enthalten ist, unter Bedingungen analog der Umsetzung von Verbindungen der Formel IIb, worin anstelle von A₁ und/oder A₂ Wasserstoff steht, unter erneuter Anwendung analoger Reaktionsbedingungen und Substitution des zweiten nukleofugen Restes entweder mit einem Arylamin oder einem Arylniederalkylamin, oder aus Verbindungen der Formel IIb, worin A₁ und/oder A₂ ein Hydroxyniederalkyl bedeuten, indem man die Hydroxygruppe in einen nukleofugen Rest, z.B. durch Behandlung mit einem aromatischen Sulfonsäurehalogenid, wie Toluolsulfonsäurechlorid, überführt und diesen dann mit einem Arylamin oder einem Arylniederalkylamin unter Bedingungen analog der Umsetzung von Verbindungen der Formel IIb, worin anstelle von A₁ und/oder A₂ Wasserstoff steht, mit Verbindungen der Formel VII umsetzt), mit einem Niederalkyl-, Aryl- oder Arylniederalkylisocyanat oder N-geschütztem Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60 °C, insbesondere etwa bei Raumtemperatur, wobei funktionelle Gruppen, die nicht an der Reaktion teilnehmen sollen, erforderlichenfalls geschützt sind, und vorhandene Schutzgruppen auf einer geeigneten Reaktionsstufe abspaltet.

Analog kann man in Verbindungen der Formel IIb Aminogruppen, die in A₁ und/oder A₂ als Substituent vorliegen, in Thioureido- oder an einem oder beiden Stickstoffatomen durch je bis zu einen Rest ausgewählt aus Niederalkyl, Aryl oder Arylniederalkyl substituiertes Thioureido umwandeln, indem man anstelle der Isocyanate entsprechende Thioisocyanate verwendet.

Verbindungen der Formel IIb, worin A₁ und/oder A₂ z.B. ein am endständigen Stickstoff durch 2 Reste ausgewählt aus Niederalkyl, Aryl und Arylniederalkyl substituiertes Ureidoniederalkyl bedeuten, können beispielsweise hergestellt werden, indem man entsprechende Aminoniederalkylverbindungen der Formel IIb mit Phosgen oder Analogen davon, z. B. N.N'-Carbonyldiazoliden, wie N.N'-Carbonyldiimidazol (vgl. H. A. Staab, Angew. Chem. 74, 407-423 (1962)), umsetzt und die entstehenden Chlorocarbonyl- oder Azolidocarbonyl-aminoverbindungen dann mit durch 2 Reste ausgewählt aus Niederalkyl, Aryl und Arylniederalkyl substituiertem Ammoniak umsetzt, oder umgekehrt die entsprechenden Aminoniederalkylverbindungen der Formel IIb mit dem Reaktionsprodukt von durch 2 Reste ausgewählt aus Niederalkyl, Aryl und Arylniederalkyl substituiertem Ammoniak mit Phosgen oder Analogen davon, z.B. den N.N'-Carbonyldiazoliden, wie N.N'-Carbonyldiimidazol, umsetzt unter Erhalt der entsprechend substituierten Ureidoverbindungen. Die Reaktionen werden vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und der Rückflusstemperatur, insbesondere zwischen 0 und 30 °C, durchgeführt.

Verbindungen der Formel IIb, worin A₁ und/oder A₂ z.B. ein am endständigen Stickstoff durch 2 Reste ausgewählt aus Niederalkyl, Aryl und Arylniederalkyl substituiertes Thioureidoniederalkyl enthalten, können auf analoge Weise beispielsweise hergestellt werden, indem man entsprechende Aminoniederalkylverbindungen der Formel IIb mit Thiophosgen oder Analogen davon, z. B. N.N'-Thiocarbonyldiazoliden, wie N.N'-Thiocarbonyldiimidazol (vgl. H. A. Staab, Angew. Chem. 74, 407-423 (1962)), umsetzt und die entstehenden Chlorothiocarbonyl- oder Azolidothiocarbonyl-aminoverbindungen dann mit durch 2 Reste ausgewählt aus Niederalkyl, Aryl und Arylniederalkyl substituiertem Ammoniak umsetzt, oder umgekehrt die entsprechenden Aminoniederalkylverbindungen der Formel IIb mit dem Reaktionsprodukt von durch 2 Reste ausgewählt aus Niederalkyl, Aryl und Arylniederalkyl substituiertem Ammoniak mit Thiophosgen oder Analogen davon, z.B. den N.N'-Thiocarbonyldiazoliden, wie N.N'-Thiocarbonyldiimidazol, umsetzt.

In Verbindungen der Formel IIb kann z.B. ein Hydroxyniederalkyl A₁ und/oder A₂ zur entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle sekundärer Alkohole kann ausserdem mit weniger selektiven Oxidationsmitteln, wie Chromsäure, Dichromat/Schwefelsäure, Dichromat/Eisessig, Salpetersäure, Braunstein, Selendioxid oder Dimethylsulfoxid in Gegenwart von Oxalylchlorid, in Wasser, wässrigen oder organischen Lösungsmitteln, wie halogenierten Kohlenwasserstoffen, z.B. Methylenchlorid, oder Carbonsäureamiden, wie Dimethylformamid, oxidiert werden, vorzugsweise bei Temperaturen zwischen - 50 °C und Rückflusstemperatur, insbesondere zwischen - 10 und 50 °C. Man erhält Verbindungen der Formel IIb, in denen der Rest A₁ und/oder A₂ eine Oxogruppe trägt.

Verbindungen der Formel IIb, worin A₁ und/oder A₂ Imino, Niederalkylimino, Acylimino, Hydroxyimino, Niederalkoxyimino, Hydrazono, N-Mono- oder N,N-Diniederalkylhydrazono und/oder N-Acylhydrazono enthalten, z.B. als Subsituenten in einem substituierten Niederalkyl, können aus entsprechenden Oxo-Verbindungen der Formel IIb hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in welchem die (vorzugsweise wie zuletzt beschrieben durchgeführte) Oxidation einer Hydroxy- zu einer Oxoverbindung durchgeführt wird.

So kann eine Oxoverbindung durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in ein entsprechendes Iminoderivat überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; wobei in Wasser (in An- oder Abwesenheit von Tensiden), einem wässrigen Lösungsmittelgemisch, wie einem Gemisch von Wasser mit einem oder mehreren Alkoholen, z. B. Methanol, Ethanol oder Isopropanol, Diniederalkylsulfoxiden, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen -20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur, umgesetzt wird.

Solchermassen erhältliche Oxoverbindungen der Formel IIb können in die entsprechenden Thioxoverbindungen überführt werden, beispielsweise durch Umsetzung mit Phosphorpentasulfid oder vorzugsweise Phosphorpentasulfid-Ersatzstoffen, wie Laweson's Reagenz (= 2,4-Bis-(4-methoxyphenyl)-2,4-dithioxo-1,3,2,4-dithiaphosphetan), wobei die Reaktion in inerten organischen Lösungsmitteln, beispielsweise in einem halogenierten Kohlenwasserstoff, wie Dichlormethan, bei Temperaturen von 30 °C bis zur Rückflusstemperatur, insbesondere unter Rückfluss, durchgeführt wird.

Verbindungen der Formel IIb, worin A₁ und/oder A₂ Acyliminosubstituenten enthalten, z.B. als Substituenten in einem substituierten Niederalkyl, können aus entsprechenden Iminoedukten erhalten werden durch deren Umsetzung mit einer freien Säure, die den Acylrest enthält, beispielsweise in Gegenwart von Kondensationsmitteln, wie Carbodiimiden, z.B. Dicyclohexylcarbodiimid, oder mit einem aktivierten Säurederivat davon, z.B. einem Carbonsäurehalogenid, gegebenenfalls in Gegenwart einer geeigneten Base, z.B. eines tertiären Amins, wie bereits definiert, vorzugsweise unter Feuchtigkeitsausschluss.

Verbindungen der Formel IIb, worin A₁ und/oder A₂ Niederalkylthioiminosubstituenten tragen, z.B. an substituiertem Niederalkyl, können vorzugsweise hergestellt werden, indem man entsprechende Iminoausgangsmaterialien der Formel IIb mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenyl-halogeniden, wie Methylsulfenylchlorid, umsetzt, vorzugsweise unter Verwendung der Salze der Iminoverbindungen oder in Gegenwart von Alkalihydroxiden, wie Natrium- oder Kaliumhydroxid, vorzugsweise in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

An dieser Stelle sei ausdrücklich vermerkt, dass Verbindungen der Formel IIb, worin A₁ und/oder A₂ zusätzlich zu oder anstelle von substituiertem Niederalkyl aus substituiertem Niederalkenyl und substituiertem Niederalkinyl ausgewählt sind, auch wenn diese in den Abschnitten für die Herstellung von Verbindungen der Formel IIb nicht explizit erwähnt sind, in sinnvoller und zweckmässig Weise mit analogen Herstellungsverfahren dargestellt werden können, wie für die Verbindungen der Formel IIb mit substituierten Niederalkylresten beschrieben.

Verbindungen der Formel IIb, worin A₁ und/oder A₂ z.B. Heterocyclylniederalkyl bedeuten, können aus entsprechenden Verbindungen der Formel IIb, worin wenigstens einer der Substituenten A₁ und A₂ Wasserstoff bedeutet oder aus entsprechenden Analogen, worin anstelle von A₁ und A₂ jeweils Wasserstoff steht, erhalten werden, vorzugsweise durch Umsetzung mit Verbindungen der Formel VIII,

Heterocyclylniederalkyl-L'' (VIII)

worin Heterocyclylniederalkyl wie oben definiert ist und L'' eine nukleofuge Gruppe bedeutet, wie oben für L bei Verbindungen der Formel VII definiert, unter nukleophiler Substitution der nukleofugen Gruppe L''. Die Reaktionsbedingungen entsprechen dabei vorzugsweise den bei der Alkylierungsreaktion mit Verbindungen der Formel VII genannten Bedingungen in Gegenwart einer starken Base.

Zur Herstellung von Verbindungen der Formel IIb, worin A₁ und A₂ zusammen unsubstituiertes oder substituiertes Niederalkylen bedeuten, werden beispielsweise Verbindungen der Formel IIb, worin anstelle von A₁ und A₂ Wasserstoff steht, mit einem alkylierenden Reagens der Formel IX,

L₁-B-L₂ (IX)

worin B unsubstituiertes oder substituiertes Niederalkylen als bivalentes, jeweils über eines seiner Kohlenstoffatome gebundenes Radikal (unsubstituiert oder beispielsweise substituiert durch die bei der Definition von aus A₁ und A₂ zusammen gebildetem substituiertem Niederalkylen genannten Substituenten, vorzugsweise einen dieser Substituenten) bedeutet, und worin L₁ und L₂ unabhängig voneinander eine nukleofuge Gruppe bedeuten, wie oben für L bei der Definition von Verbindungen der Formel VII beschrieben, umgesetzt, wobei erforderlichenfalls solche funktionelle Gruppen in den Ausgangsmaterialien und Endprodukten, die nicht an der Reaktion teilnehmen sollen, durch Schutzgruppen geschützt sind, die auf einer geeigneten Reaktionsstufe entfernt werden. Die bevorzugten Schutzgruppen, ihre Einführung und ihre Abspaltung wurden oben erwähnt.

L₁ ist vorzugsweise eine nukleofuge Gruppe, insbesondere aliphatisch - oder aromatisch - substituiertes Sulfonyloxy, z.B. Methansulfonyloxy oder p-Toluolsulfonyloxy (Tosyloxy), Halogen, wie Chlor, Brom oder Jod, oder Cyano, während L₂, falls ein Niederalkylenrest B zwei oder mehr Kohlenstoffatome enthält, Oxa (-O-) oder Thia (-S-), welches an zwei vizinale Kohlenstoffatome gebunden ist (wobei ein Oxiran oder Thiiran gebildet wird, welches bei Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, erfolgt, und bei anschliessender Hydrolyse unter Entstehung einer 1-Hydroxy- oder 1-Mercaptoniederalkylgengruppe reagiert), oder Aza (-NH-), welches an zwei vizinale Kohlenstoffatome gebunden ist (wobei ein Aziran gebildet wird, welches bei Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, erfolgt, und bei anschliessender Hydrolyse unter Bildung einer 1-Aminoniederalkylengruppe reagiert), bedeuten kann.

Die Umsetzung erfolgt vorzugsweise unter den Reaktionsbedingungen, die für die Umsetzung von Verbindungen der Formel IIb, worin anstelle von A₁ und A₂ jeweils ein Wasserstoffatom steht, mit Verbindungen der Formel VII als bevorzugt beschrieben sind.

Die Verbindung der Formel IX wird vorzugsweise in der äquimolaren Menge oder im Überschuss gegenüber der Verbindung der Formel IIb eingesetzt, worin anstelle von A₁ und A₂ je ein Wasserstoffatom steht, insbesondere in der 1- bis 3-fachen molaren Menge, z.B. der 1- bis 1,5-fachen molaren Menge, wie der etwa 1,2-fachen Menge. Die zur Reaktion eingesetzte starke Base wird vorzugsweise im Überschuss gegenüber der Verbindung der Formel IIb eingesetzt, worin A₁ und A₂ je ein Wasserstoffatom bedeuten, insbesondere in der 2- bis 10-fachen molaren Menge, z.B. in der 2- bis 3-fachen molaren Menge.

Die Reaktion kann so geführt werden, dass die nukleofugen Gruppen L₁ und L₂ praktisch gleichzeitig in einem Ansatz substituiert werden, oder die nukleofugen Gruppen L₁ und L₂ können der Reihe nach in verschiedenen Ansätzen substituiert werden.

In den Verbindungen der Formel IX ist B vorzugsweise unsubstituiertes oder durch einen oder mehrere, insbesondere einen der folgenden Substituenten substituiertes Niederalkylen: Niederalkyl; geschütztes Amino oder Aminoniederalkyl, z.B. Phthalimido oder Phthalimidoniederalkyl, wie Phthalimidopropyl oder, beispielsweise durch Niederalkoxycarbonyl, wie tert-Butyloxycarbonyl, geschütztes und im Mononiederalkylrest unsubstituiertes oder wie oben substituiertes (und dann erforderlichenfalls an den Substituenten geschütztes) Mononiederalkylamino oder Mononiederalkylamino-niederalkyl; in den beiden N-Niederalkylresten unsubstituiertes oder wie oben definiert substituiertes (und dann erforderlichenfalls an Substituenten geschütztes) Diniederalkylamino oder Diniederalkylaminoniederalkyl; N-geschütztes Cycloalkylamino oder Cycloalkylaminoniederalkyl; N-geschütztes Phenylniederalkylamino oder Phenylniederalkylaminoniederalkyl; N-geschütztes Phenylamino oder Phenylaminoniederalkyl; Acylamino oder Acylaminoniederalkyl; Hydroxy (zusätzlich zu der Definition von aus A₁ und A₂ gebildetem substituiertem Niederalkylen in Zwischenprodukten möglich) oder Hydroxyniederalkyl, wobei die Hydroxygruppe geschützt vorliegt; Niederalkoxy oder Niederalkoxyniederalkyl, worin der endständige Niederalkylrest unsubstituiert oder wie oben substituiert (und dann erforderlichenfalls mit geschützten Substituenten) vorliegt; Phenylniederalkoxy oder Phenylniederalkoxyniederalkyl; Acyloxy oder Acyloxyniederalkyl; Mercapto oder Mercaptoniederalkyl, worin die Mercaptogruppe in geschützter Form vorliegt; Niederalkylthio oder Niederalkylthioniederalkyl, worin der endständige Niederalkylrest unsubstituiert oder wie oben substituiert (und dann erforderlichenfalls mit geschützten Substituenten) vorliegt; Phenylniederalkylthio oder Phenylniederalkylthioniederalkyl; Acylthio oder Acylthioniederalkyl; geschütztes Carboxy oder Carboxyniederalkyl; verestertes Carboxy oder Carboxyniederalkyl; Cyano oder Cyanoniederalkyl; Oxo oder Oxoniederalkyl (erforderlichenfalls geschützt durch Acetalbildung, z. B. mit Niederalkanolen, insbesondere mit Ethan1,2-diol, wobei die Schutzgruppe auf der gewünschten Stufe durch Hydrolyse in Gegenwart einer Säure, wie Essigsäure oder Schwefelsäure, abgespalten werden kann); oder Thioxo oder Thioxoniederalkyl (erforderlichenfalls geschützt durch Thioacetalbildung, z.B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann).

In erhältlichen Verbindungen der Formel IIb kann Carboxy (in einer Carboxy- oder Carboxyniederalkylgruppe, die in einem aus A₁ und A₂ zusammen gebildetem substituiertem Niederalkylen vorliegt) in Carbamoyl, N-Mono- oder N,N-Diniederalkylcarbamoyl, N-Hydroxycarbamoyl oder N-Phenylcarbamoyl (auch in N-Aryl- und N-Aryl- niederalkylcarbamoylgruppen) umgewandelt werden, beispielsweise durch Umsetzung mit Ammoniak, einem Niederalkylamin oder einem Diniederalkylamin, Hydroxylamin oder Phenylamin, oder jeweils einem Salz davon, in Gegenwart eines Kondensationsmittels, z.B. einem Carbodiimid, wie Dicyclohexylcarbodiimid oder einem polaren Derivat davon, in polaren organischen Lösungsmitteln, wie Ethanol, oder einem N.N'-Carbonyldiazolid, wie N.N'-Dicarbonylimidazol (vgl. H. A. Staab, Angew. Chem. 74, 407-423 (1962)) in inerten organischen Lösungsmitteln, z.B. chlorierten Kohlenwasserstoffen, wie Methylenchlorid oder Chloroform, oder in Ethern, wie Diethylether, Tetrahydrofuran oder Dioxan, über das entsprechende Carbonsäureazolid. Man erhält so entsprechende Verbindungen der Formel IIb, in denen ein aus A₁ und A₂ zusammen gebildetes substituiertes Niederalkylen vorliegt, welches durch Carbamoyl, N-Mono- oder N,N-Diniederalkylcarbamoyl, N-Hydroxycarbamoyl und/oder N-Phenylcarbamoyl substituiert ist. Entsprechende Thiocarbamoylsubstituenten in aus A₁ und A₂ zusammen gebildetem substituiertem Niederalkylen können aus Carboxy durch Umwandlung in ein Carbonylhalogenid, z.B. mit anorganischen Säurehalogeniden, wie Phosphortrichlorid, Phosphorpentachlorid oder Thionylchlorid, oder organischen Säurehalogeniden, wie Oxalyldichlorid, und nachfolgende Umsetzung z.B. mit Phosphorpentachlorid, Schwefelwasserstoff und Ammoniak, einem Niederalkylamin oder einem Diniederalkylamin, Hydroxylamin oder Phenylamin erhalten werden.

In Verbindungen der Formel IIb, worin ein aus A₁ und A₂ zusammen gebildetes substituiertes Niederalkylen durch Hydroxy und/oder Hydroxyniederalkyl substituiert ist, kann durch nukleophile Substitution die Hydroxygruppe in eine Hydrazino-, durch Niederalkyl, Aryl und/oder Arylniederalkyl N-substituierte Hydrazinogruppe, eine Guanidinogruppe oder eine durch Niederalkyl, Aryl und/oder Arylniederalkyl N-substituierte Guanidinogruppe umgewandelt werden. Beispielsweise kann Hydroxy durch Umsetzung mit aromatischen Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, in Ab- oder vorzugsweise Anwesenheit von geeigneten Basen, z.B. tertiären Stickstoffbasen, wie Triethylamin oder N-Methylmorpholin, in durch die entsprechende aromatische Sulfonsäure verestertes Hydroxy überführt werden und dann dieser Ester mit Hydrazin, Guanidin, den entsprechend substituierten Derivaten, oder Salzen davon, wobei auch Schutzgruppen vorliegen können, unter den Bedingungen einer nukleophilen Substitution umgesetzt werden, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel, erforderlichenfalls (z. B. zur Umsetzung von an Aryl gebundenem Stickstoff) in inerten organischen Lösungsmitteln, wie Dimethylformamid oder 1,3-Dimethyl-3,4,5,6-tetrahydro-2(1H)-pyrimidinon, unter Zugabe starker Basen, wie Natriumamid oder Natriumhydrid. Fass sinvoll und zweckmässig, werden vorhandene Schutzgruppen abgespalten. Man erhält Verbindungen der Formel IIb, worin A₁ und A₂ zusammen ein substituiertes Niederalkylen mit Substituenten ausgewählt aus Hydrazino, an einem oder beiden Stickstoffatomen durch Niederalkyl, Aryl oder Arylniederalkyl substituiertem Hydrazino, Guanidino und an einem, zwei oder allen drei Stickstoffatomen durch Niederalkyl, Aryl oder Arylniederalkyl substituiertem Guanidino bilden.

In Verbindungen der Formel IIb, worin A₁ und A₂ zusammen ein durch Cyano und/oder Cyanoniederalkyl substituiertes Niederalkylen bilden, können Cyanogruppen z.B. durch partielle Hydrolyse, im Sinne einer Graf-Ritter-Reaktion, oder über Imino-niederalkylester-Salze in Carbamoyl- oder N-Niederalkylcarbamoylgruppen in Verbindungen der Formel IIb überführt werden. Die Bedingungen bei der Hydrolyse des Cyano-Zwischenproduktes können so gewählt werden, dass die Reaktion auf der Stufe des Amids abgebrochen wird. Zu diesem Zweck ist insbesondere die Hydrolyse mit Säuren geeignet, wobei z.B. 80%-ige Schwefelsäure (unter Erwärmen), Polyphosphorsäure (bei 110-150 °C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen. Mit Hilfe der Graf-Ritter-Reaktion gelingt die Herstellung N-monoalkylierter Amide der Formel IIb aus den entsprechenden Nitrilen. Hierzu setzt man die Nitrile in Gegenwart einer starken Säure, vornehmlich 85-90%-iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen oder Alkoholen. Die Imino-niederalkylester der Formel IIb erhält man z.B. durch säurekatalysierte Anlagerung von Alkoholen an die Cyanoverbindungen (als Salze). Diese Anlagerung kann alternativ durch Basen, z.B. Alkoholate wie Natriummethoxid, katalysiert werden. Setzt man anstelle von Alkoholen entsprechende Mercaptane ein, beispielsweise in Gegenwart von Stickstoffbasen, wie Triethylamin oder N-Methylmorpholin, so erhält man die entsprechenden Imino-niederalkylthioester. Aus den Imino-niederalkylestern erhält man die Carbamoylderivate, aus den Imino-niederalkylthioestern die entsprechenden Thiocarbamoylderivate im Sinne einer Pinner-Spaltung durch thermischen Zerfall der Iminoester-Salze bei Temperaturen oberhalb von etwa 80 °C. Die Thiocarbamoylverbindungen können auch direkt erhalten werden durch Umsetzung von Cyanogruppen mit Schwefelwasserstoff analog der partiellen Hydrolyse, beispielsweise in Gegenwart tertiärer Stickstoffbasen, wie Triethylamin.

Verbindungen der Formel IIb, worin ein aus A₁ und A₂ zusammen gebildetes substituiertes Niederalkylen durch Amidino, Amidinoniederalkyl, an einem Stickstoffatom durch bis zu zwei Reste ausgewählt aus Niederalkyl, Aryl und Arylniederalkyl substituiertes Amidino und/oder an einem Stickstoffatom durch bis zu zwei Reste ausgewählt aus Niederalkyl, Aryl und Arylniederalkyl substituiertes Amidinoniederalkyl substituiert ist, lassen sich durch Umsetzung der wie oben aus entsprechenden Cyano- oder Cyanoniederalkylausgangsmaterialien hergestellten Imino-niederalkylester oder Imino-niederalkylthiolester (als Säureadditionssalze, z.B. -(C=NH)-OC₂H₅·HCl bzw. -C(=NH)-SC₂H₅·HI) herstellen, indem mit Ammoniak oder entsprechenden primären oder sekundären Niederalkyl-, Aryl- und/oder Arylniederalkylaminen umgesetzt wird. Die entsprechenden Cyanovorstufen können beispielsweise auch durch Umsetzung mit einem Alkalimetallamid, oder durch Reaktion mit einem primären oder sekundären Ammoniumsalz, z.B. einem primären oder sekundären Ammoniumhalogenid, in die entsprechenden freien, mono- oder disubstituierten Amidine überführt werden. Verbindungen der Formel IIb, worin A₁ und A₂ zusammen ein substituiertes Niederalkylen bedeuten, welches ein an allen beiden Stickstoffatomen durch Aryl, Arylniederalkyl oder Niederalkyl substituiertes Amidino oder Amidinoniederalkyl als Substituenten trägt, lassen sich auch aus den (wie oben für Niederalkylcarbamoyl beschrieben herstellbaren) entsprechenden Verbindungen herstellen, worin in Formel IIb durch Niederalkyl, Aryl oder Arylniederalkyl N-substituiertes Carbamoyl steht, z.B. durch Umsetzung mit POCl₃ oder PCl₅ in entsprechende Imidsäurechloride (z.B. -(C=NH-niederalkyl)-Cl), welche nach Reaktion mit Ammoniak bzw. einem entsprechenden Amin substituierte Amidine der Formel II ergeben (vgl. Chem. Abstr. 81, 91186a (1974)).

In erhältlichen Verbindungen der Formel IIb können in Amino- und/oder Aminoniederalkylresten (die als Substituent in einem aus A₁ und A₂ zusammen gebildeten substituierten Niederalkylen vorliegen) vorkommende Aminoreste in Ureido, Ureidoniederalkyl oder an einem oder beiden Stickstoffatomen durch je bis zu einen Rest ausgewählt aus Niederalkyl, Aryl oder Arylniederalkyl substituiertes Ureido oder Ureidoniederalkyl umgewandelt werden, indem man entsprechende Aminoverbindungen der Formel IIb, in denen Aminoreste vorliegen, solche, in denen N-Mononiederalkylaminoreste vorliegen oder solche, in denen anstelle von Amino Arylamino oder Arylniederalkylamino steht (herstellbar z. B. durch Umsetzung von Verbindungen der Formel IIb, worin das aus A₁ und A₂ zusammen gebildete substituierte Niederalkylen Hydroxy enthält, welches durch Veresterung, z. B. durch Umsetzung mit einem aromatischen Sulfonsäurehalogenid, wie Toluolsulfonsäurechlorid, in einen nukleofugen Rest, z.B. aromatisches Sulfonyloxy, überführt wird, analog der Umsetzung von Verbindungen der Formel IIb, worin anstelle von A₁ und A₂ Wasserstoff steht, mit Verbindungen der Formel VII unter nukleophiler Substitution des nukleofugen aromatischen Sulfonyloxy entweder mit einem Arylamin oder einem Arylniederalkylamin), mit einem Niederalkyl-, Aryl- oder Arylniederalkylisocyanat oder einem N-geschützten Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60 °C, insbesondere etwa bei Raumtemperatur, wobei funktionelle Gruppen, die nicht an der Reaktion teilnehmen sollen, erforderlichenfalls geschützt sind, und vorhandene Schutzgruppen gewünschtenfalls abspaltet.

Analog lassen sich in Verbindungen der Formel IIb Aminogruppen, die als Substituent in einem aus A₁ und A₂ zusammen gebildeten substituierten Niederalkylen vorliegen, in Thioureido oder an einem oder beiden Stickstoffatomen durch je bis zu einen Rest ausgewählt aus Niederalkyl, Aryl oder Arylniederalkyl substituiertes Thioureido umwandeln, indem man anstelle der Isocyanate entsprechende Thioisocyanate verwendet.

Verbindungen der Formel IIb, worin als Substituent in einem aus A₁ und A₂ zusammen gebildeten substituierten Niederalkylen am endständigen Stickstoff durch bis zu 2 Reste ausgewählt aus Niederalkyl, Aryl und Arylniederalkyl substituiertes Ureido und/oder Ureidoniederalkyl vorliegt, können beispielsweise hergestellt werden, indem man entsprechende Aminoverbindungen der Formel IIb mit Phosgen oder Analogen davon, z. B. N.N'-Carbonyldiazoliden, wie N.N'-Carbonyldiimidazol (vgl. H. A. Staab, Angew. Chem. 74,407-423 (1962)), umsetzt und die entstehenden Chlorocarbonyl- oder Azolidocarbonyl-aminoverbindungen dann mit durch bis zu 2 Reste ausgewählt aus Niederalkyl, Aryl und Arylniederalkyl substituiertem Ammoniak umsetzt, oder umgekehrt die entsprechenden Aminoverbindungen der Formel IIb mit dem Reaktionsprodukt von durch 2 Reste ausgewählt aus Niederalkyl, Aryl und Arylniederalkyl substituiertem Ammoniak mit Phosgen oder Analogen davon, z.B. den N.N'-Carbonyldiazoliden, wie N.N'-Carbonyldiimidazol, umsetzt unter Erhalt der entsprechend substituierten Ureidoverbindungen. Die Reaktionen werden vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und Rückflusstemperatur, insbesondere zwischen 0 und 30 °C, durchgeführt. Setzt man anstelle der Aminoverbindungen der Formel IV Analoge ein, in denen Mono-Niederalkylamino, Arylamino und/oder Arylniederalkylamino vorliegt, kann man auch die entsprechenden Verbindungen der Formel II erhalten, worin bis zu drei Substituenten ausgewählt aus Niederalkyl, Aryl und/oder Arylniederalkyl an den beiden Stickstoffatomen von Ureidogruppen vorliegen.

Verbindungen der Formel IIb, worin (als Substituent in einem aus A₁ und A₂ gebildeten substituierte Niederalkylen) am endständigen Stickstoff durch 2 Reste ausgewählt aus Niederalkyl, Aryl und Arylniederalkyl substituiertes Thioureidoniederalkyl vorliegt, können auf analoge Weise beispielsweise hergestellt werden, indem man entsprechende Aminoverbindungen der Formel IIb mit Thiophosgen oder Analogen davon, z. B. N.N'-Thiocarbonyldiazoliden, wie N.N'-Thiocarbonyldiimidazol (vgl. H. A. Staab, Angew. Chem. 74, 407-423 (1962)), umsetzt und die entstehenden Chlorothiocarbonyl- oder Azolido-thiocarbonylaminoverbindungen dann mit durch 2 Reste ausgewählt aus Niederalkyl, Aryl und Arylniederalkyl substituiertem Ammoniak umsetzt, oder umgekehrt die entsprechenden Aminoverbindungen der Formel IIb mit dem Reaktionsprodukt von durch 2 Reste ausgewählt aus Niederalkyl, Aryl und Arylniederalkyl substituiertem Ammoniak mit Thiophosgen oder Analogen davon, z.B. den N.N'-Thiocarbonyldiazoliden, wie N.N'-Thiocarbonyldiimidazol, umsetzt.

Eine Verbindung der Formel IIb, worin (als Substituent in einem aus A₁ und A₂ zusammen gebildeten substituierten Niederalkylen) Hydroxy und/oder Hydroxyniederalkyl vorliegt, kann zu einer entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chrom säure, Dichromat/Schwefelsäure, Dichromat/Eisessig, Salpetersäure, Braunstein und Selendioxid oxidiert werden. Man erhält Verbindungen der Formel IIb, in denen ein aus A₁ und A₂ zusammen gebildetes substituiertes Niederalkylen durch Oxo substituiert ist.

Verbindungen der Formel IIb, worin (in einem aus A₁ und A₂ zusammen gebildeten substituierten Niederalkylen) ein oder mehrere Substituenten ausgewählt aus Imino, Niederalkylimino, Acylimino, Hydroxyimino, Niederalkoxyimino, Hydrazono, N-Mono- oder N,N-Diniederalkylhydrazono, N-Acylhydrazono und einem durch Imino, Niederalkylimino, Acylimino, Hydroxyimino, Niederalkoxyimino, Hydrazono, N-Mono- oder N,N-Diniederalkylhydrazono und/oder N-Acylhydrazono substituierten Niederalkyl vorliegen, können aus entsprechenden Oxo-Verbindungen der Formel IIb hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in dem die Oxidation einer Hydroxy- zur Oxoverbindung durchgeführt wird, welche beispielsweise wie zuletzt beschrieben erfolgt.

So können die Oxoverbindungen durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in die entsprechenden Iminoderivate überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; wobei in Wasser, einem wässrigen Lösungsmittelgemisch, wie einem Gemisch von Wasser mit einem oder mehreren Alkoholen, z. B. Methanol, Ethanol oder Isopropanol, Diniederalkylsulfoxiden, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Verbindungen der Formel IIb, worin (als Substituent in einem aus A₁ und A₂ gebildeten substituierten Niederalkylen) Acylimino und/oder Acyliminoniederalkyl vorliegt, können aus den entsprechenden Iminoverbindungen erhalten werden durch Umsetzung mit den entsprechenden freien Säuren, welche den Acylrest enthalten, beispielsweise in Gegenwart von Kondensationsmitteln, wie Carbodiimiden, z. B. Dicyclohexylcarbodiimid, oder aktivierten Säurederivaten davon, z. B. Carbonsäurehalogeniden, gegebenenfalls in Gegenwart einer geeigneten Base, z.B. eines tertiären Amins, wie bereits definiert, vorzugsweise unter Feuchtigkeitsausschluss.

Verbindungen der Formel IIb, worin aus A₁ und A₂ zusammen gebildetes Niederalkylen Niederalkylthioiminosubstituenten trägt, können vorzugsweise hergestellt werden, indem man entsprechende Iminoausgangsmaterialien der Formel IIb mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenylhalogeniden, wie Methylsulfenylchlorid, umsetzt, vorzugsweise unter Verwendung der Salze der Iminoverbindungen oder in Gegenwart von Alkalihydroxiden, wie Natrium- oder Kaliumhydroxid, vorzugsweise in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

Aus den erhältlichen Verbindungen der Formel IIb werden die Verbindungen der Formel II vorzugsweise durch Hydrolyse, z.B. im sauren oder alkalischen Medium, erhalten. Vorzugsweise erfolgt die Hydrolyse der Verbindung der Formel IIb in einer wässrigen alkoholischen Lösung einer Hydroxybase, z.B. mit einer Lösung eines Alkalimetallhydroxides, wie Natrium- oder Kaliumhydroxid, in einem Gemisch aus Wasser und Ethanol oder Methanol, bei bevorzugten Temperaturen von 0 °C bis zur Rückflusstemperatur des entsprechenden Reaktionsgemisches, insbesondere zwischen etwa 40 °C und der Rückflusstemperatur. Besonders bevorzugt ist die Reaktionsführung unter Sauerstoffausschluss, beispielsweise unter Schutzgas, wie Argon oder Stickstoff.

Die übrigen Ausgangsmaterialien sind bekannt, kommerziell erhältlich oder nach an sich bekannten Verfahren herstellbar.

Anhydride von Dicarbonsäuren der Formel II sind vorzugsweise innere Anhydride der Formel IIc, 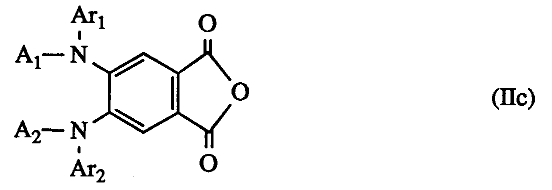
worin A₁, A₂, Ar₁und Ar₂ die unter Formel I angegebenen Bedeutungen haben. Diese Anhydride eignen sich bei Umsetzung mit Verbindungen der Formel III insbesondere zur Herstellung von Verbindungen der Formel I, worin X für O steht, Y Amino, substituiertes Amino, substituiertes Niederalkoxy oder unsubstituiertes oder substituiertes Niederalkylthio bedeutet und Z Carboxy bedeutet. Die Reaktion findet vorzugsweise in inerten Lösungsmitteln, beispielsweise Ethern, z.B. Diniederalkylethern, wie Diethylether, oder cyclischen Ethern, wie Dioxan oder Tetrahydrofuran, bei Temperaturen zwischen 0 °C und Rückflusstemperatur, vorzugsweise zwischen 20 und 60 °C, z.B. bei etwa 50 °C, statt.

Innere Anhydride der Formel IIc lassen sich vorzugsweise aus einer freien Dicarbonsäure der Formel II durch Umsetzung mit Säureanhydriden der Formel X,

R₃-(C=O)-O-(C=O)-R₃' (X)

worin R₃ und R₃' unabhängig voneinander für Wasserstoff oder Niederalkyl, nicht jedoch beide für Wasserstoff, stehen, insbesondere Acetanhydrid, herstellen.

Cyclische Imide von Dicarbonsäuren der Formel II sind vorzugsweise solche der Formel IId, 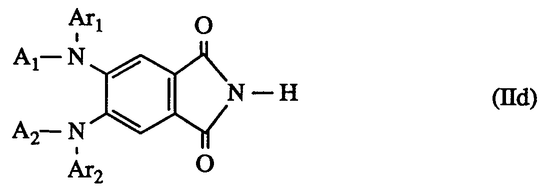
worin A₁, A₂, Ar₁ und Ar₂ die unter Formel I angegebenen Bedeutungen haben. Diese Verbindungen eignen sich vor allem zur Herstellung von Verbindungen der Formel I, worin X für O steht, Y für Amino steht und Z für freies oder verestertes Carboxy steht (Umsetzung mit Verbindungen der Formel IV, worin W₂ ein zur Herstellung von verestertem Carboxy Z geeigneter komplementärer Rest ist oder mit Wasser); oder worin X für O steht, Y für substituiertes Niederalkoxy oder substituiertes Niederalkylthio steht und Z für Carbamoyl steht (Umsetzung mit Verbindungen der Formel III, worin W₁ substituiertes Niederalkoxy oder substituiertes Niederalkylthio bedeutet). Die Reaktion erfolgt vorzugsweise in An- oder, falls die eingesetzte Verbindung der Formel III oder IV bei der Reaktionstemperatur flüssig ist, Abwesenheit aprotischer Lösungsmittel, wie Ether, z.B. Diethylether, Dioxan oder Tetrahydrofuran, bei erhöhten Temperaturen, z.B. zwischen 50 °C und Rückflusstemperatur, insbesondere zwischen 50 und 65 °C. Im Falle der Umsetzung mit Wasser (Hydrolyse) erfolgt die Reaktion vorzugsweise in wässrigen Lösungsmitteln, z.B. wässrigen Ethern, wie Wasser/Dioxan oder Wasser/Tetrahydrofuran, bei Temperaturen zwischen 0 und 50 °C, insbesondere etwa bei Raumtemperatur, in Gegenwart einer Hydroxybase, z.B. eines Alkalimetallhydroxides, wie Natrium- oder Kaliumhydroxid.

Die Verbindungen der Formel IId lassen sich nach an sich bekannten Verfahren herstellen, beispielsweise, indem man eine Verbindung der Formel IIb, worin R₁ und R₂ Niederalkyl, wie Methyl, bedeuten, mit Ammoniak umsetzt. Die Umsetzung entspricht der an sich bekannten Aminolyse von Phthalsäurediestern mit Ammoniak und verläuft meist nur bei hohen Temperaturen. Bevorzugt wird der Dimethylester verwendet. Besonders bevorzugt ist die Umsetzung in einem Lösungsmittel, insbesondere einem hochsiedenden Alkohol, z. B. einem Diol, wie Ethylenglykol, bei Temperaturen von 100 bis 150 °C, z. B. von etwa 120 °C, unter Durchleiten von Ammoniak; oder die Umsetzung der Niederalkylester bei den selben Temperaturen in Anwesenheit von Ammoniak und Lösungsmitteln, beispielsweise einem Alkohol, z.B. einem Niederalkanol, wie Methanol oder Ethanol, oder ferner in Abwesenheit eines Lösungsmittels, in einem Autoklaven bei erhöhtem Druck.

In Verbindungen der Formel III ist W₁ unsubstituiertes oder substituiertes Amino, substituiertes Niederalkoxy oder unsubstituiertes oder substituiertes Niederalkylthio, wie für die entsprechenden Reste Y definiert.

In Verbindungen der Formel IV ist ein zur Herstellung von verestertem Carboxy oder amidiertem Carboxy Z geeigneter komplementärer Rest ein Radikal, welches aus dem entsprechenden oben für Z definierten Radikal durch Entfernung der bindenden Carbonylgruppe entsteht, z.B. Niederalkoxy oder Niederalkylthio, worin der Niederalkylrest unsubstituiert oder, insbesondere ein- oder zweifach, durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert ist; oder Aryloxy, Arylthio Arylniederalkoxy oder Arylniederalkylthio, worin Aryl wie oben definiert ist; Amino; N-Mono- oder N,N-Diniederalkylamino, worin Niederalkyl unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, N-Cycloalkylamino, N-Phenylniederalkylcamino, Phenylamino, Hydrazino, an einem oder beiden Stickstoffatomen durch Niederalkyl, welches unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, Aryl oder Arylniederalkyl substituiertes Hydrazino, worin Aryl insbesondere unsubstituiertes oder wie oben substituiertes Phenyl bedeutet, Hydroxyamino, Niederalkoxyamino, worin der Niederalkylrest unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, Niederalkylenamino oder durch -O-, -S- oder -NR'- (wie oben definiert) unterbrochenes Niederalkylenamino, bei dem ein Ringkohlenstoffatom durch die entsprechende Heterogruppe ersetzt ist.

Wie erwähnt, können Derivate von Carbonsäuren der Formel II oder deren Derivaten mit mindestens einer freien Carboxygruppe, die als Acylierungsmittel verwendet werden, auch in situ gebildet werden. So kann man z. B. N,N'-disubstituierte Amidinoester in situ bilden, indem man das Gemisch des Ausgangsmaterials der Formel III oder IV und der als Acylierungsmittel verwendeten Säure der Formel II oder der Formel IIb, worin einer der Reste Z₁ oder Z₂ Hydroxy bedeutet, in Gegenwart eines geeigneten N,N'-disubstituierten Carbodiimids, z. B. N,N'-Cyclohexylcarbodiimid, zur Reaktion bringt, beispielsweise in Gegenwart einer geeigneten Base, wie Triethylamin. Weiter kann man Amino- oder Amidoester der als Acylierungsmittel verwendeten Säuren in Gegenwart des zu acylierenden Ausgangsmaterials der Formel III oder IV bilden, indem man das Gemisch der entsprechenden Säure- und Amino-Ausgangsmaterialien in Gegenwart eines N,N'-disubstituierten Carbodiimids, z. B. N,N'-Dicyclohexylcarbodiimid, und eines N-Hydroxyamins oder N-Hydroxy-amids, z. B. N-Hydroxysuccinimid, gegebenenfalls in Anwesenheit einer geeigneten Base, z. B. 4-Dimethylamino-pyridin, umsetzt. Ferner kann man durch Umsetzung mit N,N,N',N'-Tetraalkyluroniumverbindungen, wie O-Benztriazol-1yl-N,N,N',N-tetra-methyl-uronium-hexalluorphosphat, Aktivierung in situ erreichen. Schliesslich können Phosphorsäureanhydride der Carbonsäuren der Formel II in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart von N-Hydroxybenztriazol, umsetzt.

Diese und die übrigen im bei der Beschreibung der Herstellungsverfahren genannten Reaktionen können unter an sich bekannten Reaktionsbedingungen, bei üblichen Temperaturen, in An- oder Abwesenheit von inerten Lösungs- oder Verdünnungsmitteln durchgeführt werden, beispielsweise in Säureamiden, z.B. Carbonsäureamiden, wie Dimethylformamid, Dimethylacetamid oder 1,3-Dimethyl-3,4,5,6-tetrahydro-2(1H)-pyrimidinon (DMPU), oder Amiden anorganischer Säuren, wie Hexamethylphosphorsäuretriamid, Ethern, z.B. cyclischen Ethern, wie Tetrahydrofuran oder Dioxan, oder acyclischen Ethern, wie Diethylether oder Ethylenglykoldimethylether, halogenierten Kohlenwasserstoffen, wie Haloniederalkanen, z.B. Methylenchlorid oder Chloroform, Ketonen, wie Aceton, Nitrilen, wie Acetonitril, Säureanhydriden, wie Acetanhydrid, Estern, wie Essigsäureethylester, Bisalkansulfinen, wie Dimethylsulfoxid, Stickstoffheterocyclen, wie Pyridin, oder Gemischen dieser Lösungsmittel, insbesondere in wasserfreien Lösungsmitteln oder Lösungsmittelgemischen, wobei die jeweils geeigneten Lösungsmittel für die oben genannten Reaktionen ausgewählt werden können, soweit sinnvoll und zweckmässig, unter Verwendung von Salzen der eingesetzten Verbindungen, insbesondere von Metallsalzen eingesetzter Cabonsäuren, wie den Alkali- oder Erdalkalimetallsalzen, z.B. Natrium- oder Kaliumsalzen, in Ab- oder Anwesenheit von Katalysatoren, Kondensationsmitteln oder neutralisierenden Agentien, je nach Art der Reaktion und/oder der Reaktionsteilnehmer unter atmosphärischem Druck oder in einem geschlossenen Gefäss, unter Normaldruck oder ferner unter erhöhtem Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind die spezifisch genannten Reaktionsbedingungen; sehr bevorzugt sind Reaktionsbedingungen, die den in den Beispielen genannten analog sind. Auch kann das Acylierungsmittel, z.B. ein Carbonsäurehalogenid oder ein Carbonsäureanhydrid, selbst als Lösungsmittel dienen. Der Reaktionsablauf wird zweckmässig mittels üblicher analytischer Methoden, insbesondere mit Dünnschichtchromatographie, verfolgt.

Alle genannten Ausgangsmaterialien (d.h. Ausgangsstoffe oder Zwischenverbindungen) können an funktionellen Gruppen, die nicht an den beschriebenen Reaktionen teilnehmen sollen, erforderlichenfalls geschützt sein, wobei vorhandene Schutzgruppen zu einem geeigneten Zeitpunkt abgespalten werden. Erhaltene Verbindungen der Formel I mit Schutzgruppen können durch Schutzgruppenabspaltung in die freien Verbindungen der Formel I überführt werden, oder den unten genannten zusätzlichen Verfahrensmassnahmen unterworfen werden.

Zu den Schutzgruppen für funktionelle Gruppen in Ausgangsmaterialien, deren Umsetzung vermieden werden soll, insbesondere Carboxy-, Amino-, Hydroxy-, Mercapto- und Sulfogruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Charakteristisch für Schutzgruppen ist auch, dass sie in den Endstoffen nicht vorliegen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkyl-, z. B. Methylgruppen, und eine Amino- oder Carboxygruppe eines zweiten Moleküls der Formel I substituiert sein. Verbindungen mit solchen Schutzgruppen lassen sich z. B. mit entsprechenden Halogensilanen wie Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl, oder eine in Form eines Lactons, insbesondere eines γ-Lactons, geschützte Carboxygruppe.

Die Freisetzung von geschütztem Carboxy erfolgt nach üblichen, z. B. den in den oben genannten Standardwerken über Schutzgruppen genannten Verfahren.

So kann geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als γ-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe ist durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z.B. das Chlorid- oder Bromidanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl, besonders bevorzugt tert-Butoxycarbonyl oder Benzyloxycarbonyl.

Bevorzugt sind auch bivalente Aminoschutzgruppen, wie mono- oder disubstituierte Methylidengruppen, wie 1-Niederalkoxy (beispielsweise Methoxy oder Ethoxy)-niederalkyliden (beispielsweise Ethyliden oder 1-n-Butyliden), z.B. =C(CH₃)(OC₂H₅), ferner z.B. =C(CH₃)₂ oder =CH-Phenyl, und insbesondere Bisacylreste, z.B. der Phthalylrest, welcher zusammen mit dem zu schützenden Stickstoffatom ein 1H-isoindol-1,3(2H)-dion (Phthalimidogruppe) bildet.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisenoder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, bevorzugt in polaren Lösungsmitteln, wie Diniederalkylniederalkanoylamiden, z. B. Dimethylformamid, Ethern, wie cyclischen Ethern, z. B. Dioxan, oder Alkoholen, wie Methanol, Ethanol oder Propanol, wobei Methanol besonders bevorzugt ist, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig- oder Trifluoressigsäure, gespalten werden, gegebenenfalls in Gegenwart von Wasser, und eine als Silylamino geschützten Aminogruppe kann z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten. Die Abspaltung der Phthalylgruppe kann z.B. mittels Hydrazinhydrat geschehen oder mittels einer Säure, z.B. einer Mineralsäure, wie Chlorwasserstoffsäure, oder einer organischen Säure, wie Essigsäure, gegebenenfalls in Gegenwart von organischen Lösungsmitteln, z.B. Methanol oder Tetrahydrofuran.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine Hydroxygruppe kann beispielsweise durch eine Acylgruppe, z. B. unsubstituiertes oder durch Halogen, wie Chlor, substituiertes Niederalkanoyl, wie Acetyl oder 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Triphenylmethoxycarbonyl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines γ-Lactones, geschützt sein.

Bevorzugt ist eine geschützte Hydroxygruppe durch Triniederalkylsilyl oder als Lacton geschützt, insbesondere durch tert-Butyl-dimethylsilyl.

Eine Mercaptogruppe kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z. B. Isopropyliden, Cycloalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt. Veresterte Hydroxygruppen, z. B. Niederalkanoyloxy, wie Acetyloxy, können auch durch Esterasen freigesetzt werden, acyliertes Amino beispielsweise durch geeignete Peptidasen.

Eine Sulfogruppe kann beispielsweise durch Niederalkyl, z. B. Methyl oder Ethyl, durch Phenyl oder als Sulfonamid, beispielsweise als Imidazolid, geschützt sein.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, beispielsweise Natriumcarbonat, freigesetzt.

Oxo wird erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann, Thioxoniederalkyl wird erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann.

Die Temperaturen für die Freisetzung der geschützten funktionellen Gruppen liegen vorzugsweise zwischen -80 und 100 °C, besonders bevorzugt zwischen -20 und 50 °C, beispielsweise zwischen 10 und 35 °C, wie im Bereich der Raumtemperatur oder bei Rückflusstemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

### Zu b) Reduktion einer Carboxyfunktion

Die Monocarbonsäuren der Formel V, worin Y' dieselben Bedeutungen hat wie Y in Verbindungen der Formel I (ausser Hydroxy, welches durch eine Carboxyschutzgruppe ersetzt sein kann), liegen vorzugsweise in aktivierter Form vor. Bei den reaktionsfähigen Derivaten handelt es sich insbesondere um solche der Formel IIe, 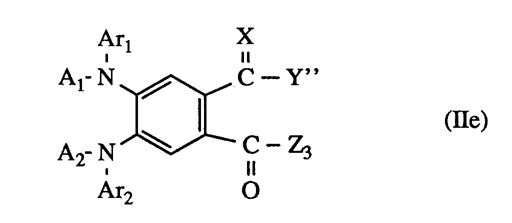
worin Y'' dieselben Bedeutungen hat wie Y in Verbindungen der Formel I oder eine Carboxyschutzgruppe bedeutet, falls X für Sauerstoff steht, Z₃ Halogen, wie Chlor oder Brom, bedeutet und die übrigen Reste die genannten Bedeutungen haben. Man erhält so Verbindungen der Formel I, worin Z Hydroxymethyl oder Formyl bedeutet,
Bevorzugte reaktionsfähige Derivate sind auch cyclische Imide der Formel IIf, 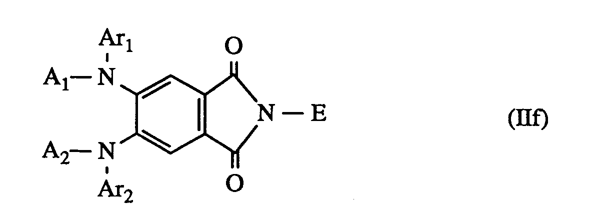
worin E Wasserstoff oder Niederalkyl bedeutet und die übrigen Reste die genannten Bedeutungen haben.

Die Reduktion erfolgt vorzugsweise mit komplexen Hydriden, insbesondere mit Lithiumaluminiumhydrid oder LiAlH[OC(CH₃)₃]₃ in Ethern, wie Diethylether oder Tetrahydrofuran, Pyridin oder N-Alkylmorpholinen, wie N-Methylmorpholin, oder mit Natriumborhydrid (erforderlichenfalls in Gegenwart von LiCl) in Wasser, Diglycol, Methanol, Ethanol oder Isopropanol oder Gemischen dieser Lösungsmittel), bei Temperaturen zwischen 10 °C und der Rückflusstemperatur.

Verbindungen der Formel IIe werden beispielsweise hergestellt, indem man eine gemäss Verfahren a) erhaltene Verbindung der Formel I, worin Z eine Carboxygruppe bedeutet und die übrigen Reste die genannten Bedeutungen haben (also eine Verbindung der Formel V) mit einem anorganischen Säurehalogenid, z.B. Phosphortrichlorid, Phosphortribromid, Phosphorpentachlorid, Phosphorpentabromid, Thionylchlorid oder Thionylbromid, oder mit Oxalyldichlorid umsetzt, erforderlichenfalls in Gegenwart von Lösungsmitteln, bei Temperaturen zwischen 10 °C und Rückflusstemperatur, z.B. zwischen Raumtemperatur und 80 °C, und unter Wasserausschluss.

Verbindungen der Formel IIf, worin E Niederalkyl, insbesondere Methyl, bedeutet, werden vorzugsweise analog den oben genannten Verbindungen der Formel IId aus Verbindungen der Formel IIb hergestellt, indem man mit Niederalkylaminen anstelle von Ammoniak umsetzt unter Reaktionsbedingungen analog den oben für die Herstellung von Verbindungen der Formel IId aus Verbindungen der Formel IIb genannten. Verbindungen der Formel IIe, worin E Wasserstoff bedeutet, sind identisch mit Verbindungen der Formel IId und werden vorzugsweise wie diese hergestellt.

Alternativ können Verbindungen der Formel IIf auch vorzugsweise aus Anhydriden der Formel IIc hergestellt werden, indem man mit Ammoniak oder Niederalkylaminen bei höheren Temperaturen oder in Gegenwart von Hexamethyldisilazan und Methanol etwa bei Raumtemperatur umsetzt, vorzugsweise in einem hochsiedenden Alkohol, wie 2-Ethoxyethanol, bei Temperaturen zwischen 50 und 130 °C, z.B. bei etwa 100 bis 120 °C.

Die Dicarbonsäuren, worin X Sauerstoff und Y' Hydroxy bedeuten, liegen vorzugsweise in Form reaktionsfähiger Derivate vor. Zu den reaktionsfähigen Derivaten zählen insbesondere reaktionsfähige Anhydride, wie die unter Verfahren a) genannten inneren Anhydride der Formel IIc, oder die dort genannten cyclischen Imide der Formel IId, worin jeweils die Reste die genannten Bedeutungen haben und die wie oben hergestellt werden.

Die inneren Anhydride der Formel IIc werden vorzugsweise mit einem komplexen Hydrid unter den Reaktionsbedingungen, wie oben bei der Reduktion von Verbindungen der Formel V beschrieben, reduziert, insbesondere mit Natriumborhydrid in Methanol oder Ethanol bei Temperaturen zwischen Raumtemperatur und der Rückflusstemperatur, z.B. etwa bei Raumtemperatur bis 40 °C. Man erhält Verbindungen der Formel I, worin X Sauerstoff bedeutet, Y Hydroxy bedeutet, Z Hydroxymethyl bedeutet und die übrigen Reste die genannten Bedeutungen haben.

Die cyclischen Imide der Formel IIf werden vorzugsweise unter denselben Bedingungen reduziert, wie zuletzt für die inneren Anhydride der Formel IIc beschrieben, insbesondere mit Natriumborhydrid in Methanol oder Ethanol bei Temperaturen zwischen Raumtemperatur und der Rückflusstemperatur, z.B. etwa bei Raumtemperatur bis etwa 40 °C. Man erhält Verbindungen der Formel I, worin X Sauerstoff bedeutet, Y Amino oder Niederalkylamino bedeutet, Z Hydroxymethyl bedeutet und die übrigen Reste die genannten Bedeutungen haben.

Alle genannten Ausgangsmaterialien können an funktionellen Gruppen, die nicht an den beschriebenen Reaktionen teilnehmen sollen, erforderlichenfalls geschützt sein, wobei vorhandene Schutzgruppen zu einem geeigneten Zeitpunkt abgespalten werden. Erhaltene Verbindungen der Formel I mit Schutzgruppen können durch Schutzgruppenabspaltung in die freien Verbindungen der Formel I überführt werden oder (erforderlichenfalls nach partieller oder vollsatändiger Schutzgruppenabspaltung) den unten genannten zusätzlichen Verfahrensmassnahmen unterworfen werden. Der Schutz der genannten Verbindungen, geeignete Schutzgruppen und ihre Abspaltung sind unter Verfahren a) beschrieben.

### Zusätzliche Verfahrensmassnahmen:

Verbindungen der Formel I können in an sich bekannter Weise in andere Verbindungen der Formel I umgewandelt werden.

Es ist möglich, einzelne der genannten Umwandlungen durchzuführen, oder aber auch geeignete Kombinationen zu wählen, also zwei oder mehr Umwandlungen mit einer Verbindung der Formel I durchzuführen. Funktionelle Gruppen in Ausgangsmaterialien der Formel I und anderen Ausgangsmaterialien, die nicht an der jeweiligen Umsetzung teilnehmen sollen, liegen erforderlichenfalls in geschützter Form vor. Die Schutzgruppen werden zu geeigneten Zeitpunkten abgespalten. Die Einführung der Schutzgruppen, die Schutzgruppen selbst und ihre Abspaltung sind wie unter Verfahren a) beschrieben.

Zum Beispiel kann eine Verbindung der Formel I, worin die Gruppe -C(=X)- für -C(=O)-steht, mit einem geeigneten Reagens umgesetzt werden, so dass eine andere Verbindung der Formel I, worin die Gruppe -C(=X)- für -C(=S)- steht, erhalten wird. Analog kann verestertes oder amidiertes Carboxy Z in einen Rest überführt werden, worin das Oxo des Carbonylrestes durch Thio ersetzt ist. Ein geeignetes Reagens für die Ueberführung von -C(=O)- in -C(=S)- sind z.B. Phosphorpentasulfid oder vorzugsweise Phosphorpentasulfid-Ersatzstoffe, z.B. das Lawesson-Reagens (= 2,4-Bis-(4-methoxyphenyl)-2,4-dithioxo-1,3,2,4-dithiaphosphetan), wobei die Reaktion beispielsweise in einem halogenierten Kohlenwasserstoff, wie Dichlormethan, bei Temperaturen von 30 °C bis zur Rückflusstemperatur, insbesondere bei Rückflusstemperatur, durchgeführt wird.

Verbindungen der Formel I, worin einer der Reste A₁ und A₂ Wasserstoff bedeutet, können durch Umsetzung mit geeigneten Reagentien in andere Verbindungen der Formel I, worin keiner der Reste A₁ und A₂ mehr Wasserstoff bedeutet, überführt werden.

Zur Einführung von A₁ oder A₂ = unsubstituiertes oder substituiertes Niederalkyl kommt z.B. die Behandlung mit der Base LDA und anschliessende Umsetzung mit einem unsubstituierten oder substituierten Diniederalkylether oder einem unsubstituierten oder substituierten Niederalkylhalogenid in Frage. Substituiertes Niederalkyl ist dabei wie für den entsprechenden Rest A₁ und/oder A₂ in Verbindungen der Formel I definiert.

Verbindungen der Formel I, worin Ar₁ und/oder Ar₂ durch Halogen, vorzugsweise Brom, substituiertes Aryl, insbesondere Phenyl oder Naphthyl, bedeutet, können in die entsprechenden Derivate umgewandelt werden, in denen eines oder alle der in Aryl Ar₁ und/oder Ar₂ vorhandenen Halogenatome durch Cyano ersetzt sind, beispielsweise durch Umsetzung mit einem Cyanidsalz eines Übergangsmetalles, insbesondere CuCN, bei Temperaturen zwischen 50 und 150 °C, vorzugsweise zwischen 60 und 140 °C, in einem inerten polaren Lösungsmittel, wie einem N,N-Diniederalkyl-niederalkancarbonsäureamid, z. B. Dimethylformamid, ohne oder mit anschliessender Zugabe eines Katalysators, z. B. eines Übergangsmetallhalogenides, wie Eisen(III)chlorid, in wässriger Lösung, (siehe auch Rosenmund et al., Ber. 52, 1749 (1916); von Braun et al., Ann. 488, 111 (1931).

In Verbindungen der Formel I können die Reste Ar₁ und/oder Ar₂, welche unsubstituiertes oder substituiertes Aryl bedeuten, vorzugsweise unsubstituiertes Phenyl oder Naphthyl, unabhängig voneinander unter Einführung einer oder mehrerer Nitrogruppen nitriert werden, beispielsweise unter üblichen Bedingungen zur Einführung einer Nitrogruppe in Aromaten, z. B. mit konzentrierter oder 100%-iger Salpetersäure bei Temperaturen zwischen 0 und 100 °C, vorzugsweise zwischen 10 und 40 °C, in einem inerten Lösungsmittel, z. B. einem organischen Säureanhydrid, wie Acetanhydrid. Entstehen dabei mehrere verschiedene Produkte mit unterschiedlicher Position und Anzahl der Nitrogruppen, so können diese nach üblichen Methoden, beispielsweise säulenchromatographisch, getrennt werden.

Nitrosubstituenten innerhalb der Reste Ar₁ und/oder Ar₂ können zu Aminogruppen reduziert werden, beispielsweise durch Hydrierung unter üblichen Bedingungen, z. B. durch Hydrierung in Gegenwart eines zur selektiven Reduktion der Nitrogruppen geeigneten Hydrierkatalysators, wie Raney-Nickel, in einem inerten Lösungsmittel, z. B. einem cyclischen oder acyclischen Ether, wie Tetrahydrofuran, unter Normaldruck oder bei bis zu 5 bar erhöhtem Druck.

Verbindungen der Formel I mit veretherten Hydroxygruppen, beispielsweise mit Niederalkoxyresten als Substituenten innerhalb von Ar₁ und/oder Ar₂ oder an A₁ und/oder A₂, können durch Etherspaltung in die entsprechenden Hydroxy-substituierten Verbindungen der Formel I überführt werden. Die Etherspaltung erfolgt unter an sich bekannten Bedingungen, beispielsweise in Gegenwart von Halogenwasserstoffsäuren, wie Bromwasserstoff oder Iodwasserstoff, in An- oder Abwesenheit von Lösungsmitteln, wie Carbonsäuren, z. B. Niederalkancarbonsäuren, wie Essigsäure, bei Temperaturen zwischen 20 °C und der Rückflusstemperatur des Reaktionsgemisches, oder vorzugsweise unter schonenden Bedingungen mit Borhalogeniden, insbesondere Bortribromid, in einem inerten Lösungsmittel, wie einem chlorierten Kohlenwasserstoff, z. B. Methylenchlorid oder Chloroform, bei Temperaturen zwischen -80 und 0 °C, insbesondere zwischen -50 und -20 °C.

Verbindungen der Formel I, in denen Hydroxy vorliegt, z.B. in A₁, A₂, aus diesen beiden Resten zusammen gebildetem substituiertem Niederalkylen und/oder Hydroxymethyl Z, können durch Oxidation zu den entsprechenden Carbonylverbindungen und direkt anschliessende oder erst nach der Isolierung der Carbonylverbindung erfolgende Umsetzung mit Hydroxylamin oder einem Salz davon oder weiteren Aminoverbindungen in die entsprechenden Iminoverbindungen, wie Hydroxyiminoverbindungen, überführt werden. Die weiteren Substituenten, Reagentien und bevorzugten Reaktionsbedingungen finden sich bei der Beschreibung der Herstellung von Verbindungen der Formel IIb, worin als Substituent in aus A₁ und A₂ zusammen gebildetem substituiertem Niederalkylen Imino, Niederalkylimino, Acylimino, Hydroxyimino, Niederalkoxyimino, Hydrazono, N-Mono- oder N,N-Diniederalkylhydrazono und/oder N-Acylhydrazono vorliegt, aus entsprechenden Oxo-Verbindungen der Formel IIb, wobei an Stelle der Oxoverbindungen der Formel IIb solche der Formel I einzusetzen sind.

Verbindungen der Formel I, in denen Hydroxyimino als Substituent in A₁ und/oder A₂ oder einem aus diesen beiden Resten zusammen gebildetem Niederalkylen vorliegt, und/oder in Hydroxyiminomethyl Z können unter Hydrierung zu den entsprechenden Aminoverbindungen umgesetzt werden. Die Hydrierung erfolgt dabei vorzugsweise katalytisch mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, z.B. Niederalkanolen, wie Methanol oder Ethanol, oder Gemischen davon, z.B. in Methanol/Tetrahydrofuran-Gemischen, bei Temperaturen zwischen -20 und 60 °C, vorzugsweise zwischen 0 und 40 °C, z.B. etwa bei Raumtemperatur.

In Verbindungen der Formel I mit primären Hydroxygruppen, beispielsweise solchen Verbindungen der Formel I, in denen substituiertes Niederalkyl, substituiertes Niederalkenyl oder substituiertes Niederalkinyl A₁ und/oder A₂ primäre Hydroxygruppen enthalten, wie in Hydroxyethyl, oder in denen ein aus A₁ und A₂ zusammen gebildetes substituiertes Niederalkylen vorliegt, welches als Substituent Hydroxyniederalkyl, wie Hydroxymethyl, enthält, können primäre Hydroxygruppen zu entsprechenden Carboxy- oder Carboxyniederalkylresten oxidiert werden, beispielsweise durch Oxidation mit Chromsäure, Dichromat/Schwefelsäure, Salpetersäure, Braunstein oder Kaliumpermanganat, vorzugsweise mit Kaliumpermanganat in neutralem oder alkalischem Medium, z.B. in wässriger alkoholischer Lösung, bei bevorzugten Temperaturen zwischen -20 und 50 °C, insbesondere zwischen 0 °C und Raumtemperatur. Man erhält entsprechende Carboxy-substituierte Verbindungen der Formel I.

In Verbindungen der Formel I, worin ein substituiertes Niederalkyl A₁ und/oder A₂ Carboxy enthält, wie in Carboxymethyl A₁ und/oder A₂, oder worin ein aus A₁ und A₂ zusammen gebildetes substituiertes Niederalkylen vorliegt, welches als Substituent Carboxy- oder Carboxyniederalkyl enthält, beispielsweise in solchen Verbindungen, die nach dem zuletzt beschriebenen Verfahren aus Verbindungen der Formel I mit primären Hydroxygruppen hergestellt sind, können Carboxygruppen durch Reaktion mit Diazomethan (ergibt Methyloxycarbonyl) oder mit Niederalkanolen, wie Methanol oder Ethanol, in die entsprechenden Niederalkoxycarbonylgruppen überführt werden. Die Reaktion mit Diazomethan erfolgt beispielsweise in wässrig-alkoholischer Lösung, z.B. in Wasser/Methanol, oder vorzugsweise in Ether, in Gegenwart einer etherischer Lösung von Diazomethan, z.B. Diazomethan in Diethylether, bei Temperaturen zwischen -20 und 30 °C, z.B. zwischen 0 °C und Raumtemperatur. Die Reaktion mit Niederalkanolen erfolgt vorzugsweise in Gegenwart von Kondensationsmitteln, wie Carbodiimiden, z.B. Dicyclohexylcarbodiimid, in dem betreffenden Niederalkanol, dem ein weiteres inertes organisches Lösungsmittel, vorzugsweise Dimethylformamid oder Dimethylacetamid, zugesetzt sein kann, bei Temperaturen zwischen 0° und der Rückflusstemperatur, vorzugsweise zwischen 10 und 40 °C. Man erhält entsprechende Niederalkoxycarbonylverbindungen der Formel I.

Verbindungen der Formel I, worin als Substituent in einem aus A₁ und A₂ zusammen gebildeten substituierten Niederalkylen Hydroxy und/oder Hydroxyniederalkyl vorliegt, und/oder worin Hydroxymethyl Z vorliegt, können zu einer entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein, Selendioxid oder Dimethylsulfoxid in Gegenwart von Oxalylchlorid, in Wasser, wässrigen oder organischen Lösungsmitteln, wie halogenierten Kohlenwasserstoffen, z.B. Methylenchlorid, oder Carbonsäureamiden, wie Dimethylformamid, oxidiert werden, vorzugsweise bei Temperaturen zwischen - 50 °C und Rückflusstemperatur, insbesondere zwischen - 10 und 50 °C. Man erhält Verbindungen der Formel I, in denen aus A₁ und A₂ zusammen gebildetes substituiertes Niederalkylen und/oder substituiertes Methyl Z durch Oxo substituiert ist.

Verbindungen der Formel I, worin Z Hydroxymethyl bedeutet, können nach Überführung der Hydroxygruppe in eine Abgangsgruppe, z.B. durch Umsetzung mit aromatischen Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, oder Halo, wie Chloro oder insbesondere Bromo (herstellbar durch Umsetzung mit einem anorganischen Säurehalogenid, wie Phosphortrichlorid oder -bromid, Phosphorpentachlorid oder -bromid oder Thionylchlorid oder -bromid), wobei die Herstellung der Abgangsgruppe erforderlichenfalls in inerten Lösungsmitteln, z.B. Ethern, wie Tetrahydrofuran oder Dioxan, oder halogenierten Kohlenwasserstoffen, wie Chloroform oder Methylenchlorid, bei Temperaturen zwischen Raumtemperatur und Rückflusstemperatur, in An- oder Abwesenheit von tertiären Stickstoffbasen, wie Pyridin, N-Methylmorpholin oder Triethylamin, erfolgt; in die entsprechenden Verbindungen überführt werden, worin Z durch Mononiederalkylamino, Diniederalkylamino oder Niederalkylthio, worin der Niederalkylrest unsubstituiert oder wie oben substituiert ist, Cyano, Hydrazino, an einem oder beiden Stickstoffatomen durch Niederalkyl substituiertes Hydrazino, Guanidino oder durch an einem, zwei oder allen drei Stickstoffatomen durch Niederalkyl substituiertes Guanidino substituiertes Methyl bedeutet. Die Umsetzung erfolgt, je nach einzuführendem Substituenten, mit einem Mono- oder Diniederalkylamin; einem Niederalkylmercaptan mit unsubstituiertem oder substituiertem Niederalkyl in Gegenwart von Basen, wie Hydroxybasen, z.B. Alkalimetallhydroxiden, wie Natrium- oder Kaliumhydroxid; mit Cyanidsalzen, wie Alkalimetallcyaniden, z.B. Natrium- oder Kaliumcyanid; mit Hydrazin, Guanidin, oder den entsprechend substituierten Derivaten; wobei auch Schutzgruppen vorliegen können, unter den Bedingungen der nukleophilen Substitution, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel. Schutzgruppen können abgespalten werden. Die Verbindungen der Formel I, worin substituiertes Methyl Z Substituenten ausgewählt aus Hydrazino und am endständigen N-Atom durch Niederalkyl substituiertes Hydrazino enthält, kann man auch ausgehend von einer entsprechenden Oxoverbindung der Formel I durch deren Umsetzung mit Stickstoffbasen ausgewählt aus Hydrazin und durch Niederalkyl bis zu zweifach substituiertem Hydrazin, wie weiter unten für die Umsetzung einer Oxoverbindung mit Stickstoffbasen beschrieben, und anschliessende Reduktion einer erhaltenen entsprechenden Iminoverbindung, vorzugsweise durch katalytische Hydrierung mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, wie Niederalkanolen, z.B. Methanol oder Ethanol, oder Gemischen davon, z.B. Methanol/Tetrahydrofuran-Gemischen, bei Temperaturen zwischen -20 und 60 °C, vorzugsweise zwischen 0 und 40 °C, z.B. etwa bei Raumtemperatur, herstellen.

Verbindungen der Formel I, worin Z Hydroxymethyl bedeutet, können nach Überführung der Hydroxygruppe in eine Abgangsgruppe, wie zuletzt beschrieben, z.B. in eine Toluolsulfonyloxygruppe, mit unsubstituierten oder im Niederalkylrest entsprechend substituierten Niederalkanolen umgesetzt werden, wobei man Verbindungen der Formel I erhält, worin Z durch unsubstituiertes oder substituiertes Niederalkoxy substituiertes Methyl bedeutet. Die Umsetzung erfolgt vorzugsweise mit einem Alkoholat des unsubstituierten oder substituierten Niederalkanoles (worin erforderlichenfalls nicht an der Reaktion zu beteiligende Schutzgruppen in geschützter Form vorliegen), welches z.B. durch Umsetzung mit einem Alkalimetall, wie Natrium, in Ethern, z.B. Diethylether, Dioxan oder Tetrahydrofuran, erhalten wird, insbesondere unter langsamer Zugabe des Alkoholates zu einer Lösung der entsprechenden Verbindung der Formel I mit in eine Abgangsgruppe überführter Hydroxygruppe in einem inerten, aprotischen Lösungsmittel, z.B. demselben, in dem das Niederalkanolat gelöst ist, bei Temperaturen zwischen Raum- und Rückflusstemperatur.

Verbindungen der Formel I, worin Z Aminomethyl oder Niederalkylaminomethyl bedeutet, können in entsprechende Verbindungen umgewandelt werden, worin Z Ureidomethyl oder 1- oder 3-Mononiederalkylureidomethyl bedeutet, indem man entsprechende Aminoverbindungen der Formel I mit einem Niederalkylisocyanat oder einem N-geschützten Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60 °C, insbesondere etwa bei Raumtemperatur, wobei funktionelle Gruppen, die nicht an der Reaktion teilnehmen sollen, erforderlichenfalls geschützt sind, und vorhandene Schutzgruppen abspaltet. Man kann auch entsprechende Aminoverbindungen der Formel I mit Phosgen oder Analogen davon, z. B. N.N'-Carbonyldiazoliden, wie N.N'-Carbonyldiimidazol (vgl. H. A. Staab, Angew. Chem. 74, 407-423 (1962)), umsetzen und die entstehenden Chlorocarbonyl- oder Azolidocarbonyl-aminoverbindungen dann mit durch Niederalkyl substituiertem Ammoniak umsetzen, oder umgekehrt die entsprechenden Aminoverbindungen der Formel I mit dem Reaktionsprodukt von durch Niederalkyl substituiertem Ammoniak mit Phosgen oder Analogen davon, z.B. den N.N'-Carbonyldiazoliden, wie N.N'-Carbonyldiimidazol, umsetzen unter Erhalt von 3-Niederalkylureidomethyl Z. Die Reaktion wird vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und Rückflusstemperatur, insbesondere zwischen 0 und 30 °C, durchgeführt.

Verbindungen der Formel I, worin in einem aus A₁ und A₂ zusammen gebildeten substituierten Niederalkylen ein oder mehrere Substituenten ausgewählt aus Imino, Niederalkylimino, Acylimino, Hydroxyimino, Niederalkoxyimino, Hydrazono, N-Mono- oder N,N-Diniederalkylhydrazono, N-Acylhydrazono und durch Imino, Niederalkylimino, Acylimino, Hydroxyimino, Niederalkoxyimino, Hydrazono, N-Mono- oder N,N-Diniederalkylhydrazono und/oder N-Acylhydrazono substituiertem Niederalkyl vorliegt, und/oder Verbindungen der Formel I, worin Z durch Imino, Niederalkylimino, Acylimino, Hydroxyimino, Niederalkoxyimino, Hydrazono, N-Mono- oder N,N-Diniederalkylhydrazono oder N-Acylhydrazono substituiertes Methyl bedeutet, können aus entsprechenden Oxo-Verbindungen der Formel I hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in dem die Oxidation einer Hydroxy- zur Oxoverbindung durchgeführt wird, welche beispielsweise wie zuletzt beschrieben erfolgt.

So können die Oxoverbindungen durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in die entsprechenden Iminoderivate überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; wobei in Wasser (in An- oder Abwesenheit von Tensiden), einem wässrigen Lösungsmittelgemisch, wie einem Gemisch von Wasser mit einem oder mehreren Alkoholen, z. B. Methanol, Ethanol oder Isopropanol, Diniederalkansulfinen, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur, umgesetzt wird.

Verbindungen der Formel I, worin Z Niederalkylthioiminomethyl bedeutet, können vorzugsweise hergestellt werden, indem man entsprechende Iminoausgangsmaterialien der Formel I mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenyl-halogeniden, wie Methylsulfenylchlorid, umsetzt, vorzugsweise unter Verwendung der Salze der Iminoverbindungen oder in Gegenwart von Alkalihydroxiden, wie Natrium- oder Kaliumhydroxid, in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

In einer Verbindung der Formel I kann Aminomethyl Z in Niederalkanoylaminomethyl Z oder Hydroxymethyl Z in Niederalkanoyloxymethyl überführt werden. Dies geschieht beispielsweise durch Umsetzung mit einer (gewünschtenfalls in situ) aktivierten Niederalkansäure (herstellbar analog der Herstellung der aktivierten Säurederivate der Formel IIa), z.B. einem Niederalkansäureanhydrid der Formel X, wie Acetanhydrid, oder einem Niederalkansäurehalogenid, wie -chlorid oder -bromid, gelöst in einem Niederalkansäureanhydrid oder einem inerten Lösungsmittel, wie einem Ether, z.B. Tetrahydrofuran oder Dioxan, oder einem N,N-Diniederalkyl-niederalkancarbonsäureamid, wie Dimethylformamid, bei Temperaturen zwischen 0 °C und Rückflusstemperatur, insbesondere etwa bei Raumtemperatur, in An- oder Abwesenheit von tertiären Stickstoffbasen, wie Pyridin, N-Methylmorpholin oder Triethylamin.

Eine Verbindung der Formel I, worin X für O steht, Y für Amino steht und Z Hydroxymethyl bedeutet, kann in eine Verbindung der Formel I umgewandelt werden, worin Y und Z zusammen 1-Oxamethylen bedeuten, das über den Sauerstoff anstelle von Y und das Methylenkohlenstoffatom anstelle von Z gebunden ist. Die Umwandlung geschieht vorzugsweise durch Destillation im Hochvakuum, beispielsweise im Kugelrohr, bei Drucken von 0,001 bis 0,5 torr, vorzugsweise bei 0,2 bis 0,05 torr, bei hohen Temperaturen, die zur Destillation ausreichen, z.B. bei 180 bis 250 °C.

Aus einer Verbindung der Formel I, worin worin Y und Z zusammen 1-Oxamethylen bedeuten, das über den Sauerstoff anstelle von Y und das Methylenkohlenstoffatom anstelle von Z gebunden ist, kann durch Umsetzung mit einer Verbindung der Formel III, wie oben definiert, worin W₁ substituiertes Amino bedeutet, insbesondere Mono- oder Diniederalkylamino, Hydrazino, Phenylamino oder Phenylniederalkylamino, eine Verbindung der Formel I erhalten werden, worin X für O steht, Y für substituiertes Amino und Z für Hydroxymethyl steht. Die Umsetzung erfolgt vorzugsweise in einem Alkohol, wie Methanol oder Ethanol, in An- oder Abwesenheit eines Antioxidans, wie Natriumascorbat, bei Temperaturen zwischen 30 und 100 °C, z.B. zwischen 50 und 80 °C, bei Temperaturen oberhalb der Rückflusstemperatur in einem geschlossenen Rohr.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Diastereomere z.B. durch Verteilung zwischen mehrphasigen Lösungsmittelgemischen, Umkristallisieren und/oder chromatographische Trennung, beispielsweise an Kieselgel, Racemate z.B. durch Bilden von Salzen mit optisch reinen salzbildenden Reagentien und Auftrennen des so erhältlichen Diastereomerengemisches, z.B. mittels fraktionierter Kristallisation, oder durch Chromatographie an optisch aktiven Säulenmaterialien.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 250°C, vorzugsweise von etwa -20°C bis etwa 150°C, z.B. bei Raumtemperatur bis zu der Rückflusstemperatur, bei Schmelzen bei bis zu 220 °C, unter atmosphärischem Druck oder in einem geschlossenen Gefäss, gegebenenfalls unter Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind die jeweils spezifisch genannten Reaktionsbedingungen.

Lösungs- und Verdünnungsmittel sind beispielsweise Wasser, Alkohole, z.B. Niederalkanole, wie Methanol, Ethanol oder Propanol, Diole, wie Ethylenglykol, Triole, wie Glycerin, oder Arylalkohole, wie Phenol, Säureamide, z.B. Carbonsäuramide, wie Dimethylformamid, Dimethylacetamid oder 1,3-Dimethyl-3,4,5,6-tetrahydro-2(1H)-pyrimidinon (DMPU), oder Amide anorganischer Säuren, wie Hexamethylphosphorsäuretriamid, Ether, z.B. cyclische Ether, wie Tetrahydrofuran oder Dioxan, oder acyclische Ether, wie Diethylether oder Ethylenglykoldimethylether, halogenierte Kohlenwasserstoffe, wie Haloniederalkane, z.B. Methylenchlorid oder Chloroform, Ketone, wie Aceton, Nitrile, wie Acetonitril, Säureanhydride, wie Acetanhydrid, Ester, wie Essigsäureethylester, Bisalkansulfine, wie Dimethylsulfoxid, Stickstoffheterocyclen, wie Pyridin, Kohlenwasserstoffe, z.B. Niederalkane, wie Heptan, oder Aromaten, wie Benzol oder Toluol, oder Gemische dieser Lösungsmittel, wobei die jeweils geeigneten Lösungsmittel für die oben genannten Reaktionen ausgewählt werden können.

Infolge der engen Beziehung zwischen den Verbindungen der Formel I und ihren Vorstufen in freier Form und in Form von Salzen und/oder Tautomeren sind vor- und nachstehend unter den freien Verbindungen und Ausgangsmaterialien sinn- und zweckmässig gegebenenfalls auch die entsprechenden Salze bzw. freien Verbindungen und/oder Tautomeren zu verstehen, sofern die Verbindungen eine oder mehrere salzbildende Gruppen, z. B. basische Gruppen, wie Amino- oder Iminogruppen, auch solche, die an nicht mehr als ein ungesättigtes Kohlenstoffatom (wie die Gruppen -NA₁Ar₁ und/oder -NA₂Ar₂ am C-Atom des zentralen Phenylringes, worin Ar₁ und A₁ und/oder Ar₂ und A₂ nicht über ein ungesättigtes Kohlenstoffatom gebunden sind) und/oder saure Gruppen (wie Carboxy oder Sulfo (SO₃H)) und/oder tautomerisierbare Gruppen enthalten. Sofern im Zusammenhang mit Ausgangsmaterialien, oder Verbindungen der Formel I vor- und nachstehend ein Substituent, eine Verbindung, ein Tautomeres, ein Salz, Substituenten, Verbindungen, Tautomere oder Salze erwähnt werden, ist dies, soweit sinnvoll und zweckmässig, unabhängig von der Verwendung des Singulars oder Plurals zu verstehen als "ein oder mehrere". Ausgangsmaterialien können auch in geschützter Form verwendet werden, soweit dies erforderlich, sinnvoll und zweckmässig ist, wobei die Schutzgruppen zu geeigneten Zeitpunkten abgespalten werden können. Schutzgruppen, ihre Einführung und ihre Abspaltung sind insbesondere wie oben unter Verfahren a) definiert.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsmaterialien eingesetzt, die zu den eingangs als bevorzugt beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsmaterial unter den Reaktionsbedingungen gebildet oder in Form eines Derivates, z.B. eines Salzes davon, verwendet wird.

Reihenfolge und Reaktionsbedingungen aller beschriebenen Umsetzungen sind vorzugsweise so zu wählen, wie es für den Fachmann als sinn- und zweckmässig anzusehen ist.

Die vorliegende Erfindung betrifft auch pharmazeutische Präparate, die als Wirkstoff Verbindungen der Formel I umfassen. Besonders bevorzugt sind Präparate zur enteralen, insbesondere oralen, sowie zur parenteralen Verabreichung. Die Präparate enthalten vorzugsweise den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Bevorzugt ist eine pharmazeutische Zusammensetzung, welche geeignet ist zur Verabreichung an einen Warmblüter, insbesondere Menschen, der an einer der genannten Erkrankungen, insbesondere einer Erkrankung, die auf eine Hemmung einer Proteinkinase anspricht, beispielsweise Psoriasis oder ein Tumor, leidet, umfassend eine zur Hemmung der Proteinkinase wirksame Menge einer Verbindung der Formel I oder eines Salzes davon, falls salzbildende Gruppen vorliegen, zusammen mit mindestens einem pharmazeutisch annehmbaren Trägermaterial.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon oder Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, z.B. fetten Oelen, ®Lauroglykol (1,2-Propylenglykol-monolaurat, Gattefossé S.A., Saint Priest, France), ®Gelucire (Gattefossé S.A., Saint Priest, France) oder Sesamöl, Paraffinöl oder flüssigen Polyethylenglykolen, wie PEG 300 (Mᵣ im wesentlichen 285-315) oder PEG 400 (Mᵣ im wesentlichen 380-420), gelöst oder suspendiert, wobei ebenfalls Stabilisatoren oder Detergentien, wie ®Tween (Polyoxyethylen-Sorbitan-monofettsäureester, Marke der Atlas Chem. Ind. Inc., USA) zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls eine Methode und/oder ein Verfahren zur Behandlung der oben genannten Krankheitszustände, insbesondere solcher, die auf eine Hemmung von Proteinkinasen ansprechen. Die Verbindungen der vorliegenden Erfindung können prophylaktisch oder therapeutisch verabreicht werden, vorzugsweise in einer gegen die genannten Krankheiten wirksamen Menge an einen Warmblüter, z. B. Menschen, der einer derartigen Behandlung bedarf, wobei man sie vorzugsweise in Form von pharmazeutischen Präparaten verwendet. Dabei wird bei einem Körpergewicht von etwa 70 kg eine tägliche Dosis von 1 mg bis 5000 mg, z.B. etwa 0,1 g bis etwa 5 g, vorzugsweise von etwa 0,5 g bis etwa 2 g, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: ber. = berechnet; Ether = Diethylether; FAB-MS = Fast Atom Bombardment Mass Spectroscopy; FD-MS = Felddesorptions-Massenspektroskopie; gef. = gefunden; gesätt. = gesättigt; mbar = Druckeinheit (1 mbar = 1 Hectopascal); ¹H-NMR = Protonen-Kernresonanz; RT = Raumtemperatur, Smp. = Schmelzpunkt; THF = Tetrahydrofuran; torr = Druckeinheit (1 torr entspricht 1 mm Quecksilbersäule); Zers. = unter Zersetzung.

Mengenverhältnisse von Lösungsmittel-/Elutionsmittelgemischen beziehen sich, wenn nicht anders angegeben, auf Volumenverhältnisse (v/v).

### Beispiel 1: 4,5-Bis(4-fluoranilino)phthalsäurediamid

In einem Autoklaven werden 290 mg (0,7 mmol) 4,5-Bis(4-fluoranilino)phthalsäuredimethylester in 2 ml Methanol gelöst und dann 20 ml Ammoniak einkondensiert. Der verschlossene Autoklav wird 18 h auf 120° erwärmt. Der Autoklav wird abgekühlt, überschüssiger Ammoniak bei RT mit Stickstoff abgeblasen und der Rückstand mit Ethylacetat ausgewaschen. Die gelbe Suspension wird filtriert und gut mit Ethylacetat nachgewaschen. Auf diese Weise wird die Titelverbindung in Form gelber Kristalle erhalten, Smp. 192-194°, FAB-MS: 383 [M⁺+H].

Die Ausgangsmaterialien werden wie folgt hergestellt:

### a) 4,5-Bis(4-fluoranilino)phthalsäuredimethylester

Eine Lösung von 2,4 g (6 mmol) 4,5-Bis(trimethylsilyloxy)cyclohexa-1,4-dien-1,2-dicarbonsäuredimethylester (Beispiel 1b) und 2,3 ml (24 mmol) 4-Fluoranilin in 60 ml Eisessig wird während 2 h unter Rückflussbedingungen gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft.

Der Eindampfrückstand wird mit Ethylacetat/Hexan 2:1 an Kieselgel chromatographiert, die Produktfraktionen eingedampft und aus Ethylacetat/Hexan umkristallisiert. Auf diese Weise wird die Titelverbindung in Form gelber Kristalle erhalten, ¹H-NMR (CDCl₃): δ = 7.40 (s, 2H), 7.10-6.80 (m, 8H), 5.70 (br s, 2H), 3.83 (s, 6H).

### b) 4,5-Bis(trimethylsilyloxy)cyclohexa-1,4-dien-1,2-dicarbonsäuredimethylester

Unter Argon wird eine Lösung von 7,1 g (50 mmol) Acetylendicarbonsäuredimethylester (Fluka, Schweiz) in 30 ml Toluol zu 12,5 g (50 mmol) 2,3-Bis(trimethylsilyloxy)-1,3-butadien (Aldrich, Bundesrepublik Deutschland) (95%) getropft und anschliessend 19 h unter Rückflussbedingungen gekocht. Das Reaktionsgemisch wird abgekühlt, das Lösungsmittel abgedampft und der Rückstand im Hochvakuum (0,1 mbar, 124-127°) destilliert. Auf diese Weise wird die Titelverbindung als gelbes, hochviskoses Oel erhalten, ¹H-NMR (CDCl₃):δ= 0.18 (s, 18H), 3.09 (s, 4H), 3.78 (s, 6H).

### Beispiel 2: 4,5-Dianilino-phthalsäuremonoamid-monomethylester

350 mg (1.06 mmol) 4,5-Dianilino-phthalimid werden in 10 ml Methanol 7 Tage unter Rückflussbedingungen gekocht. Das Reaktionsgemisch wird eingedampft und mit Ethylacetat/Hexan 2:1 an Kieselgel chromatographiert. Die Produktfraktionen werden vereinigt und aus Ethylacetat/Hexan kristallisiert. Auf diese Weise wird die Titelverbindung in Form blassgelber Kristalle erhalten, Smp. 176-178°C, FAB-MS: 362 [M⁺+H].

Die Ausgangsmaterialien werden wie folgt hergestellt:

### a) 4,5-Dianilino-phthalimid

Eine Suspension von 230 mg (0,7 mmol) 4,5-Dianilino-phthalsäuredimethylester in 23 ml Ethylenglykol wird auf 120° erwärmt; unter Rühren wird während 24 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 40:1 an Kieselgel chromatographiert. Die Produktfraktionen werden vereinigt und eingedampft. Auf diese Weise wird die Titelverbindung in Form gelber Kristalle erhalten, Smp. 215-217°, FAB-MS: 330 [M⁺+H].

### b) 4,5-Dianilino-phthalsäuredimethylester

Eine Lösung von 5,6 g (15 mmol) 4,5-Bis(trimethylsilyloxy)cyclohexa-1,4-dien-1,2-dicarbonsäuredimethylester (Beispiel 1 b) und 5,5 ml (60 mmol) Anilin in 60 ml Eisessig wird während 4 h unter Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Das Rohprodukt wird aus Ethanol umkristallisiert. Auf diese Weise wird die Titelverbindung in Form gelber Kristalle erhalten, Smp. 178°, FAB-MS: 377 [M⁺+H].

### Beispiel 3: 4,5-Dianilino-phthalsäuremonamid-Natriumsalz

998 mg (3 mmol) 4,5-Dianilino-phthalimid (Beispiel 2 a) werden in einer Mischung aus 20 ml 0.1 N Natronlauge und 30 ml THF vier Tage bei RT gerührt, Das Reaktionsgemisch wird filtriert und das Filtrat mit Ethylacetat extrahiert. Aus der Wasserphase kristallisiert die Titelverbindung beim Stehenlassen aus: weisse Kristalle, Zers.p. 170°C, FAB-MS: 348 [M⁺+H].

### Beispiel 4: 4,5-Dianilino-phthalsäure-di-2-hydroxyethylester und 4,5-Bis(anilino)phthalsäuremonomethy-mono-2-hydroxyethylester

Eine Suspension von 230 mg (0,7 mmol) 4,5-Dianilino-phthalsäuredimethylester in 23 ml Ethylenglykol wird mit Ammoniak-Gas gesättigt, auf 120° erwärmt und während 24 h gerührt. Das Reaktionsgemisch wird abgekühlt, mit gesätt. Natriumchlorid-Lösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit einem Gradienten von Hexan/Ethylacetat 2:1 bis 1:10 an Kieselgel chromatographiert, die Produktfraktionen vereinigt und eingedampft. Auf diese Weise werden die Titelverbindungen in Form blassgelber Kristalle erhalten: 4,5-Dianilino-phthalsäure-di-2-hydroxyethylester, Smp. 162-163°C, FAB-MS: 437 [M⁺+H], 4,5-Bis(anilino)phthalsäuremonomethyl-mono-2-hydroxyethylester Smp. 170-171°, FAB-MS: 407 [M⁺+H].

### Beispiel 5: Bis(2-hydroxyethyl)-5,8-diphenyl-5,8-diaza-5,6,7,8-tetrahydro-naphthalin-2,3-dicarboxylat:

Analog zu Beispiel 4 werden 40 mg (0.1 mmol) 5,8-Diphenyl-5,8-diaza-5,6,7,8-tetrahydro-naphthalin-2,3-dicarbonsäuredimethylester in 4 ml mit Ammoniak-Gas gesättigten Ethylenglykol während 24 h auf 120° erwärmt. Das Reaktionsgemisch wird abgekühlt, mit gesätt. Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 20:1 an Kieselgel chromatographiert, die Produktfraktionen vereinigt und eingedampft. Auf diese Weise wird die Titelverbindung in Form eines blassgelben Wachses erhalten: ¹H-NMR (CDCL₃): 7.45 (m, 4H), 7.3 (t, 6H), 7.1 (s, 2H), 4.3 (t, 4H), 3.8 (m, 8H); FAB-MS: 463 [M⁺+H].

Das Ausgangsmaterial wird wie folgt hergestellt:

### a) 5,8-Diphenyl-5,8-diaza-5,6,7,8-tetrahydro-naphthalin-2,3-dicarbonsäuredimethylester

Eine Lösung von 2,24 g (6 mmol) 4,5-Bis(trimethylsilyloxy)cyclohexa-1,4-dien-1,2-dicarbonsäuredimethylester (Beispiel 1 b) und 5,1 g (24 mmol) N,N'-Diphenylethylendiamin (Sigma, Schweiz) in 24 ml Eisessig wird während 2 h unter Rückflussbedingungen gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Das Rohprodukt wird mit Hexan/Ethylacetat 3:1 an Kieselgel chromatographiert, die Produktfraktionen eingedampft und der Rückstand aus Ethanol umkristallisiert. Auf diese Weise wird die Titelverbindung in Form oranger Kristalle erhalten, FAB-MS: 402 [M⁺], 403 [M⁺+H].

### Beispiel 6: 4,5-Dianilino-2-hydroxymethyl-benzoesäureamid

Man versetzt eine Lösung von 4,5-Dianilino-phthalimid (Beispiel 2 a, 0,30 g, 0,91 mMol) in Methanol (40 ml) unter Rühren solange portionenweise mit festem Natriumborhydrid (0,3 g, 0,008 Mol), bis die anfangs gelbgefärbte Reaktionslösung farblos geworden ist und sich im Dünnschichtchromatogramm (Kieselgel, Ethylacetat/Hexan 1:1) kein Ausgangsmaterial mehr nachweisen lässt. Danach gibt man Kochsalzlösung zu und extrahiert das gebildete Produkt mit Ethylacetat. Nach dem Trocknen (Na₂SO₄) und Eindampfen des Ethylacetatextrakts bleibt ein farbloser Rückstand, der aus Methanol/Ether kristallisiert. Nach zweimaliger Kristallisation erhält man 2-Hydroxymethyl-4,5-dianilinobenzoesäureamid in farblosen, derben Kristallen, die bei 200°C unter Zersetzung schmelzen. C₂₀H₁₉N₃O₂: Molekulargewicht 333 Gef. 333 (FD-MS).

### Beispiel 7: 4,5-Bis(4-fluoranilino)-2-hydroxymethyl-benzoesäureamid

Man versetzt eine Lösung von 4,5-bis(4-fluoranilino)-phthalimid (0,25 g 0,685 mMol) in Methanol (40 ml) unter Rühren solange portionenweise mit festem Natriumborhydrid (0,6 g, 0,016 Mol), bis die anfangs gelbgefärbte Reaktionslösung farblos geworden ist und sich im Dünnschichtchromatogramm (Kieselgel, Ethylacetat/ Hexan 1:1) kein Ausgangsmaterial mehr nachweisen lässt. Danach gibt man Kochsalzlösung zu und extrahiert das gebildete Produkt mit Ethylacetat. Nach dem Trocknen (Na₂SO₄) und Eindampfen des Ethylacetatextrakts bleibt ein farbloser Rückstand, der aus Ethylacetat/Ether zweimal kristallisiert wird. Man erhält so 2-Hydroxymethyl-4,5-di(4-fluoranilino)-benzoesäureamid in farblosen Kristallen, Smp. 81-82°C.

C₂₀H₁₇F₂N₃O₂: Molekulargewicht 369.37, ber. C 65,03%; H 4,64%; N 11,38%; gef. C 65,09%; H 4,67%; N 11,55 %.

Die Ausgangsmaterialien werden wie folgt hergestellt:

### a) 4,5-Bis(4-fluoranilino)-phthalimid

Analog zu Beispiel 2 a werden 290 mg (0,7 mmol) 4,5-Bis(4-fluoranilino)phthalsäuredimethylester in 22 ml Ethylenglykol auf 120° erwärmt und unter Rühren während 18 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 1:1 an Kieselgel chromatographiert, die Produktfraktionen vereinigt und eingedampft. Auf diese Weise wird die Titelverbindung in Form oranger Kristalle erhalten, Smp. > 220°C, FAB-MS: 366 [M⁺+H].

### b) 4,5-Bis(4-fluoranilino)-phthalsäuredimethylester

Eine Lösung von 2,4 g (6 mmol) 4,5-Bis(trimethylsilyloxy)cyclohexa-1,4-dien-1,2-dicarbonsäuredimethylester (Beispiel 1 b) und 2,3 ml (24 mmol) 4-Fluoranilin in 60 ml Eisessig wird während 2 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Der Eindampfrückstand wird mit Ethylacetat/Hexan 2:1 an Kieselgel chromatographiert, die Produktfraktionen eingedampft und aus Ethylacetat/Hexan umkristallisiert. Auf diese Weise wird die Titelverbindung in Form gelber Kristalle erhalten, ¹H-NMR (CDCl₃): δ=7.40 (s, 2H), 7.10-6.80 (m, 8H), 5.70 (br s, 2H), 3.83 (s, 6H).

### Beispiel 8: 4,5-Dianilino-2-hydroxymethyl-benzoesäure

In eine Lösung von 4,5-Dianilino-phthalsäureanhydrid (0,250 g, 0,75 mM) in Methanol (50 ml) wird unter Rühren solange portionenweise festes Natriumborhydrid eingetragen, bis die anfangs stark gelbgefärbte Reaktionslösung praktisch farblos geworden ist. Nun setzt man Kochsalzlösung und Wasser zu, stellt mit Zitronensäurelösung auf pH 6 und nimmt das gebildete Produkt durch mehrmalige Extraktion mit Ethylacetat auf. Nach dem Trocknen (Na₂SO₄) und Eindampfen des Ethylacetates bleibt ein farbloses Material zurück, das aus Ethylacetat/Ether kristallisiert. Man erhält die 2-Hydroxymethyl-4,5-dianilinobenzoesäure in langen, dünnen Prismen vom Smp. 158-169°C (Zersetzung).

C₂₀H₁₈N₂O₃: Molekulargewicht ber. 334, gef. 334 (FD-MS).

Das Ausgangsmaterial wird wie folgt hergestellt:

### a) 4,5-Dianilino-phthalsäureanhydrid

Eine Lösung von 4,5-Dianilino-phthalsäure in Essigsäureanhydrid wird 30 min auf 60°C erhitzt, wobei starke Gelbfärbung auftritt. Nach dem Eindampfen bleiben gelbe Kristalle von 4,5-Dianilino-phthalsäureanhydrid zurück, die aus Aceton/Ether umkristallisiert werden und dann bei 196-197°C schmelzen.

C₂₀H₁₄N₂O₃: Molekulargewicht ber. 330, gef. 330 (FD-MS).

### b) 4,5-Dianilino-phthalsäure

Man erhitzt 4,5-Dianilino-phthalsäuredimethylester (Beispiel 2b, 3,05 g) in einem Gemisch aus Methanol (500 ml) und 1M Natronlauge (100 ml) während 2 h unter Sauerstoffausschluss und Rückfluss. Danach dampft man das Methanol im Vakuum ab und säuert die alkalische Lösung des Reaktionsprodukts mit Salzsäure an. Die Titelverbindung scheidet sich rasch kristallin ab und wird nach Umkristallisieren aus Methylenchlorid in schwach gelblichen spitzen Prismen erhalten, die bei 169°C unter Zersetzung schmelzen.

C₂₀H₁₆N₂O₄: Molekulargewicht ber. 348, gef. 348 (FD-MS).

### Beispiel 9: 5,6-Dianilino-2-oxa-indan-1-on

2-Hydroxymethyl-4,5-dianilinobenzoesäureamid (Beispiel 6) liefert bei der Destillation im Hochvakuum (Kugelrohr, 0,1 torr, Badtemperatur 210°C) ein farbloses, öliges Destillat, das in Aceton/Ether auskristallisiert. Das Titelprodukt bildet schwach gelbliche, nahezu farblose Kristalle vom Smp. 189-190°C.

C₂₀H₁₆N₂O₂: Molekulargewicht ber. 316, gef. 316 (FD-MS).

### Beispiel 10: 4,5-Dianilino-2-hydroxymethyl-benzoesäuremethylamid

5,6-Dianilino-2-oxa-indan-1-on (Beispiel 9,70 mg) wird mit Methylamin in Ethanol (33 prozentig, 15 ml) im geschlossenen Rohr unter Zusatz von 5 mg Natriumascorbat (Antioxydans) während 3 h auf 60°C erhitzt. Das Reaktionsprodukt (als violette Lösung in Ethylacetat/ Aceton 1:1) wird mit Ethylacetat durch Kieselgel filtriert, wobei dunkles, violettes Material festgehalten wird. Das farblose Eluat hinterlässt nach dem Eindampfen Kristalle, die aus Aceton/Ether umkristallisiert werden. Man erhält so das 2-Hydroxymethyl-4,5-dianilino-benzoesäuremethylamid in farblosen Prismen, Smp. 167-169°C.

C₂₁H₂₁N₃O₂: Molekulargewicht ber. 347, gef. 347 (FD-MS).

### Beispiel 11: 4,5-Dianilino-2-hydroxymethyl-benzoesäurehydrazid

5,6-Dianilino-2-oxa-indan-1-on (Beispiel 9, 0,131 g, 0,041 Mol) wird mit Methanol (20 ml) und Hydrazinhydrat (0,4 ml) im geschlossenen Rohr 12 h auf 70°C erhitzt. Man dampft das Reaktionsgemisch ein und kristallisiert den Rückstand zweimal aus Ethylacetat/Ether. Das 2-Hydroxymethyl-4,5-dianilinobenzoesäurehydrazid bildet farblose Prismen (0,070 g, 49 % der Theorie) vom Smp. 166-167°C.

C₂₀H₂₀N₄O₂: Molekulargewicht ber. 348, gef. 348 (FD-MS).

### Beispiel 12: 3-Hydroxy-4,5-dianilino-2-azaindan-1-on (Tautomeres von 4,5-Dianilino-2-formyl-benzoesäureamid):

Zu einer Lösung von 4,5-Dianilinophthalimid (Beispiel 2 a, 0,493g; 1,49 mmol) in Tetrahydrofuran (150 ml) gibt man unter Rühren portionenweise festes Lithiumaluminiumhydrid (insgesamt 0,34 g; 8,9 mmol). Danach ist die anfänglich stark gelb gefärbte Lösung nur mehr schwach gelblich gefärbt. Nun setzt man Wasser und soviel Zitronensäure zu, dass ein pH-Wert von etwa 4,5 erhalten wird. Man filtriert durch Celite® (Filterhilfsmittel auf Kieselgurbasis, Fluka, Schweiz), wäscht mit Tetrahydrofuran und Ethylacetat nach und extrahiert darauf das Filtrat mit Ethylacetat. Nach dem Trocknen und Eindampfen bleibt ein amorpher Lack zurück, der beim Anreiben mit einer geringen Menge Ethylacetat kristallisiert. Man erhält so schwach gelblich gefärbte Kristalle von 4,5-Dianilino-2-formylbenzoesäureamid, die zur Reinigung aus Methanol/Ether umkristallisiert werden. Smp. ab ca. 220°C (Zers.)).
C₂₀H₁₇N₃O₂: Molekulargewicht ber. 331, gef 331 (FD-MS).

### Beispiel 13: 4,5-Dianilino-2-hydroxyiminomethyl-benzoesäureamid

Die Titelverbindung wird nach oben genannten Verfahren hergestellt.

### Beispiel 14: 2-Acetoxymethyl-4,5-dianilino-benzoesäureamid

Eine Aufschlämmung von 76 mg 4,5-Dianilino-2-hydroxymethylbenzoesäureamid (Beispiel 6) in 3 ml Essigsäureanhydrid und 3 ml Pyridin wird rasch in Lösung gebracht. Nach 3 h bei Raumtemperatur wird das Reaktionsgemisch im Vakuum unter Toluolzusatz eingedampft. Der farblose Rückstand besteht aus der Titelverbindung.

C₂₀H₂₁N₃O₃: Molekulargew. ber. 375, gef. 375 (FD-MS).

### Beispiel 15: 4,5-Dianilinophthalsäuremonoanilid

Man erhitzt ein Gemisch von 0,106 g 4,5-Dianilinophthalsäureanhydrid (Beispiel 8a), 20 ml Tetrahydrofuran und 0,2 g Anilin während 90 min auf 50°C. Dann dampft man im Vakuum zur Trockne. Den Eindampfrückstand löst man in Ether, filtriert von einer Trübung ab und setzt der filtrierten Lösung Pentan zu. Nach kurzer Zeit scheiden sich blassgelbe Kristalle von 4,5-Dianilinophthalsäuremonoanilid ab. Smp. etwa 120°C (Zers.).

C₂₆H₂₁N₃O₃: Molekulargew. ber. 423, gef. 423 (FD-MS).

### Beispiel 16: (A) 5,8-Diphenyl-5,8-diaza-6-hydroxymethyl-5,6,7,8-tetrahydro-naphthalin-2,3-dicarbonsäuredi(2-hydroxyethyl)ester und (B) 5,8-Diphenyl-5,8-diaza-5,6,7,8-tetrahydro-naphthalin-2,3-dicarbonsäuremono(2-hydroxyethyl)ester-monomethylester:

Eine Suspension von 230 mg (0,7 mmol) 5,8-Diphenyl-5,8-diaza-6-hydroxymethyl-5,6,7,8-tetrahydro-naphthalin-2,3-dicarbonsäuredimethylester in 40 ml Ethylenglykol wird auf 120° erwärmt; unter Rühren wird während 24 h ein schwacher Strom Ammoniak-Gas durchgeleitet. Das Reaktionsgemisch wird abgekühlt, mit gesättigter Kochsalzlösung versetzt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan auf Kieselgel aufgetragen und chromatographiert. Die ersten Produktfraktionen werden eingedampft und der Rückstand aus Methanol/Diethylether umkristallisiert. Auf diese Weise wird die Titelverbindung (B) in Form gelber Kristalle erhalten: Smp. 155°C, FAB-MS: 463 [M⁺+H].
Die zweiten Produktfraktionen werden vereinigt und eingedampft. Der Rückstand wird aus Ethylacetat umkristallisiert. Auf diese Weise wird die Titelverbindung (A) in Form gelber Nadeln erhalten: Smp. 170-172°C, FAB-MS: 493 [M⁺+H].

Die Ausgangsmaterialien werden folgendermassen hergestellt:

### a) 5,8-Diphenyl-5,8-diaza-6-hydroxymethyl-5,6,7,8-tetrahydro-naphthalin-2,3-dicarbonsäuredimethylester

Unter Argon werden zu einer Lösung von 377 mg (1 mmol) 4,5-Dianilinophthalsäuredimethylester (Beispiel 2 b))in 10 ml DMF abs. 96 mg (2.2 mmol) Natriumhydrid (Dispersion 60% in Oel) gegeben. Bei RT wird eine Lösung von 96 µl (1.2 mmol) Epichlorhydrin in 2 ml DMF abs. zugetropft, das Reaktionsgemisch auf 100°C erwärmt und 12 h gerührt. Das Reaktionsgemisch wird abgekühlt, das Lösungsmittel abgedampft, der schwarze Rückstand in Dichlormethan gelöst und die Lösung mit Wasser gewaschen, mit Natriumsulfat getrocknet und eingedampft. Der Eindampfrückstand wird mit Ethylacetat/Hexan 1:2 an Kieselgel chromatographiert, die Produktfraktionen vereinigt und eingedampft. Auf diese Weise wird die Titelverbindung in Form eines schwach gelben Pulvers erhalten, Smp. 68°, FAB-MS: 433 [M⁺+H].

### Beispiel 17:5,8-Diphenyl-5,8-diaza-6-hydroxymethyl-5,6,7,8-tetrahydro-naphthalin-2,3-dicarboxamid

In einem Autoklaven werden 200 mg (0,4 mmol) 5,8-Diphenyl-5,8-diaza-6-hydroxymethyl-5,6,7,8-tetrahydro-naphthalin-2,3-dicarbonsäure(2-hydroxyethyl)ester in 2 ml Methanol gelöst und dann 10 ml Ammoniak einkondensiert. Der verschlossene Autoklav wird 24 h auf 120° erwärmt. Der Autoklav wird abgekühlt, überschüssiger Ammoniak bei RT mit Stickstoff abgeblasen und der Rückstand mit Ethylacetat ausgewaschen. Die gelbe Suspension wird filtriert und gut mit Ethylacetat nachgewaschen. Auf diese Weise wird die Titelverbindung in Form gelber Kristalle erhalten, Smp. 286°, FAB-MS: 403 [M⁺+H].

### Beispiel 18: Analog zu den vor- oder nachstehend genannten Verfahren und/oder Beispielen werden hergestellt:

(a) 4,5-Dianilino-phthalsäurediamid
(b) 4,5-Bis (2-iodanilino)phthalsäurediamid
(c) 4,5-Bis(3-iodanilino)phthalsäurediamid (Herstellung analog Beispiel 1 aus 4,5-Bis(3-iodanilino)phthalsäuremethylester, welches aus der Titelverbindung aus Beispiel 1b und 3-Iodanilin (Fluka, Schweiz) analog Beispiel la hergestellt wird), hellgelbe Kristalle aus Dichlormethan, Smp.: 244-245°C, FAB-MS. 599 (M⁺+H).
(d) 4,5-Bis(4-iodanilino)phthalsäurediamid
(e) 4,5-Bis(2-methoxyanilino)phthalsäurediamid
(f) 4,5-Bis(3-methoxyanilino)phthalsäurediamid
(g) 4,5-Bis(4-methoxyanilino)phthalsäurediamid
(h) 4,5-Bis(2-cyananilino)phthalsäurediamid
(i) 4,5-Bis(3-cyananilino)phthalsäurediamid
(j) 4,5-Bis(4-cyananilino)phthalsäurediamid
(k) 4,5-Bis (2-pyridinamino)phthalsäurediamid
(l) 4,5-Bis(3-pyridinamino)phthalsäurediamid
(m) 4,5-Bis(4-pyridinamino)phthalsäurediamid
(n) 4,5-Bis(2-pyrimidinamino)phthalsäurediamid
(o) 4,5-Bis(4-pyrimidinamino)phthalsäurediamid
(p) 4,5-Bis(5-pyrimidinamino)phthalsäurediamid
(q) 4,5-Bis(2-(1,3,5-triazinyl)amino)phthalsäurediamid
(r) 4,5-Bis(2-fluoranilino)phthalsäurediamid
(s) 4,5-Bis(3-fluoranilino)phthalsäurediamid
(t) 4,5-Bis(pentafluoranilino)phthalsäurediamid
(u) 4,5-Bis(4-hydroxyanilino)phthalsäurediamid
(v) 4,5-Bis(3-hydroxyanilino)phthalsäurediamid
(w) 4,5-Bis(2-hydroxyanilino)phthalsäurediamid
(x) 4,5-Bis (4-ethylanilino)phthalsäurediamid
(y) 4,5-Bis (3-ethylanilino)phthalsäurediamid
(z) 4,5-Bis(2-ethylanilino)phthalsäurediamid.

### Beispiel 19: Analog zu den vor- oder nachstehend genannten Verfahren und/oder Beispielen werden hergestellt:

(a) 4,5-Bis(4-methylanilino)phthalsäurediamid
(b) 4,5-Bis(3-methylanilino)phthalsäurediamid
(c) 4,5-Bis(2-methylanilino)phthalsäurediamid
(d) 4,5-Bis(4-carboxyanilino)phthalsäurediamid
(e) 4,5-Bis(3-carboxyanilino)phthalsäurediamid
(f) 4,5-Bis(2-carboxyanilino)phthalsäurediamid
(g) 4,5-Bis(4-methoxycarbonyl-anilino)phthalsäurediamid
(h) 4,5-Bis(3-methoxycarbonyl-anilino)phthalsäurediamid
(i) 4,5-Bis(2-methoxycarbonyl-anilino)phthalsäurediamid
(j) 4,5-Bis(4-ethoxycarbonyl-anilino)phthalsäurediamid
(k) 4,5-Bis (3-ethoxycarbonyl-anilino)phthalsäurediamid
(l) 4,5-Bis(2-ethoxycarbonyl-anilino)phthalsäurediamid
(m) 4,5-Bis(2-fluoranilino)phthalsäuremonoamid-monomethylester
(n) 4,5-Bis(2-iodanilino)phthalsäuremonoamid-monomethylester
(o) 4,5-Bis(3-iodanilino)phthalsäuremonoamid-monomethylester
(p) 4,5-Bis (4-iodanilino)phthalsäuremonoamid-monomethylester
(q) 4,5-Bis(2-methoxyaniino)phthalsäuremonoamid-monomethylester
(r) 4,5 -Bis(3-methoxyanilino)phthalsäuremonoamid-monomethylester
(s) 4,5 -Bis(4-methoxyanilino)phthalsäuremonoamid-monomethylester
(t) 4,5-Bis(2-cyananilino)phthalsäuremonoamid-monomethylester
(u) 4,5-Bis(3-cyananilino)phthalsäuremonoamid-monomethylester
(v) 4,5-Bis(4-cyananilino)phthalsäuremonoamid-monomethylester
(w) 4,5-Bis (2-pyridinamino)phthalsäuremonoamid-monomethylester
(x) 4,5-Bis(3-pyridinamino)phthalsäuremonoamid-monomethylester
(y) 4,5-Bis(4-pyridinamino)phthalsäuremonoamid-monomethylester
(z) 4,5-Bis(2-pyrimidinamino)phthalsäuremonoamid-monomethylester.

### Beispiel 20: Analog zu den vor- oder nachstehend genannten Verfahren und/oder Beispielen werden hergestellt:

(a) 4,5-Bis(4-pyrimidinamino)phthalsäuremonoamid-monomethylester
(b) 4,5-Bis(5-pyrimidinamino)phthalsäuremonoamid-monomethylester
(c) 4,5-Bis(2-(1,3,5-triazinyl)amino)phthalsäuremonoamid-monomethylester
(d) 4,5-Bis(3-fluoranilino)phthalsäuremonoamid-monomethylester
(e) 4,5-Bis(4-fluoranilino)phthalsäuremonoamid-monomethylester
(f) 4,5-Bis(pentafluoranilino)phthalsäuremonoamid-monomethylester
(g) 4,5-Bis (4-hydroxyanilino)phthals äuremonoamid-monomethylester
(h) 4,5-Bis(3-hydroxyanilino)phthalsäuremonoamid-monomethylester
(i) 4,5-Bis(2-hydroxyanilino)phthalsäuremonoamid-monomethylester
(j) 4,5-Bis(4-ethylanilino)phthalsäuremonoamid-monomethylester
(k) 4,5-Bis(3-ethylanilino)phthalsäuremonoamid-monomethylester
(l) 4,5-Bis(2-ethylanilino)phthalsäuremonoamid-monomethylester
(m) 4,5-Bis(4-methylanilino)phthalsäuremonoamid-monomethylester
(n) 4,5-Bis(3-methylanilino)phthalsäuremonoamid-monomethylester
(o) 4,5-Bis(2-methylanilino)phthalsäuremonoamid-monomethylester
(p) 4,5-Bis(4-carboxyanilino)phthalsäuremonoamid-monomethylester
(q) 4,5-Bis(3-carboxyanilino)phthalsäuremonoamid-monomethylester
(r) 4,5-Bis(2-carboxyanilino)phthalsäuremonoamid-monomethylester
(s) 4,5-Bis(methoxycarbonyl-anilino)phthalsäuremonoamid-monomethylester
(t) 4,5-Bis(3-methoxycarbonyl-anilino)phthalsäuremonoamid-monomethylester
(u) 4,5-Bis(2-methoxycarbonyl-anilino)phthalsäuremonoamid-monomethylester
(v) 4,5-Bis(4-ethoxycarbonyl-anilino)phthalsäuremonoamid-monomethylester
(w) 4,5-Bis(3-ethoxycarbonyl-anilino)phthalsäuremonoamid-monomethylester
(x) 4,5-Bis(2-ethoxycarbonyl-anilino)phthalsäuremonoamid-monomethylester
(y) 4-(3-[3-Methylureido]propyl)-dianilinophthalsäundiamid.

### Beispiel 21: Analog zu den vor- oder nachstehend genannten Verfahren und/oder Beispielen werden hergestellt:

(a) 4-(2-Aminoethyl)-4,5-dianilinophthalsäurediamid
(b) 4-(3-Aminopropyl)-4,5-dianilinophthalsäurediamid
(c) 4-(3-Carbamoylpropyl)-4,5-dianilinophthalsäurediamid
(d) 5,8-Diphenyl-5,8-diaza-6-hydroximinomethyl-5,6,7,8-tetrahydro-naphthalin-2,3-dicarboxamid
(e) 6-Aminomethyl-5,8-diphenyl-5,8-diaza-5,6,7,8-tetrahydro-naphthalin-2,3 dicarboxamid
(f) 5,8-Diphenyl-5,8-diaza-6-hydroxymethyl-5,6,7,8-tetrahydro-naphthalin-2,3-dicarbonsäuremonoamid-monomethylester
(g) 4-(3-Aminopropyl)-4,5-dianilinophthalsauremonoamid-monomethylester
(h) 4-(3-[3-Methylureido]propyl)-dianilinophthalsäuremonoamid-monomethylester
(i) 4-(2-Aminoethyl)-4,5-dianilinophthalsäuremonoamid-monomethylester
(j) 4-(3-Carbamoylpropyl)-4,5-dianilinophthalsäuremonoamid-monomethylester
(k) 5,8-Diphenyl-5,8-diaza-6-hydroximinomethyl-5,6,7,8-tetrahydro-naphthalin-2,3-dicarbonsäuremonoamid-monomethylester.
(l) 6-Aminomethyl-5,8-diphenyl-5,8-diaza-5,6,7,8-tetrahydro-naphthalin-2,3-dicarbonsäuremonoamid-monomethylester
(m) 5,8-Bis(4-fluorphenyl)-5,8-diaza-6-hydroxymethyl-5,6,7,8-tetrahydro-naphthalin-2,3-dicarboxamid
(n) 4-(3-Aminopropyl)-4,5-bis(4-fluoranilino)phthalsaurediamid
(o) 4-(3-[3-Methylureido]propyl)-4,5-bis(4-fluoranilino)phthalsäurediamid
(p) 4-(2-Aminoethyl)-4,5-bis(4-fluoranilino)phthalsäurediamid
(q) 4-(3-Carbamoylpropyl)-4,5-bis(4-fluoranilino)phthalsäurediamid
(r) 5,8-Bis(4-fluorphenyl)-5,8-diaza-6-hydroximinomethyl-5,6,7,8-tetrahydronaphthalin-2,3-dicarboxamid.
(s) 6-Aminomethyl-5,8-bis(4-fluorphenyl)-5,8-diaza-5,6,7,8-tetrahydro-naphthalin-2,3-dicarboxamid
(t) 5,8-Bis(4-fluorphenyl)-5,8-diaza-6-hydroxymethyl-5,6,7,8-tetrahydro-naphthalin-2,3-dicarbonsäuremonoamid-monomethylester
(u) 4-(3-Aminopropyl)-4,5-bis(4-fluoranilino)phthalsäuremonoamid-monomethylester
(v) 4-(3-[3-Methylureido]propyl)-4,5-bis(4-fluoranilino)phthalsäuremonoamid-monomethylester
(w) 4-(2-Aminoethyl)4,5-bis(4-fluoranilino)phthalsäuremonoamid-monomethylester
(x) 4-(3-Carbamoylpropyl)-4,5-bis(4-fluoranilino)phthalsäuremonoamid-monomethylester
(y) 5,8-Bis(4-fluorphenyl)-5,8-diaza-6-hydroximinomethyl-5,6,7,8-tetrahydronaphthalin-2,3-dicarbonsäuremonoamid-monomethylester
(z) 6-Aminomethyl-5,8-bis(4-fluorphenyl)-5,8-diaza-5,6,7,8-tetrahydro-naphthalin-2,3-dicarbonsäuremonoamid-monomethylester.

### Beispiel 22: Analog zu den vor- oder nachstehend genannten Verfahren und/oder Beispielen werden hergestellt:

(a) 5,8-Bis(4-fluorphenyl)-5,8-diaza-6-hydroximinomethyl-5,6,7,8-tetrahydro-naphthalin-2,3-dicarbonsäuremonoamid-monomethylester.
(b) 4,5-Bis(N-allyl-N-phenyl)aminophthalsäuremonoamid (Herstellung analog Beispiel 1 aus 4,5-Bis(N-allylanilino)phthalsäuredimethylester als Nebenprodukt); Smp.:
   114-116°C, FAB-MS: 428 (M⁺+H)

Herstellung des Ausgangsmaterials,
4,5-Bis(N-allylanilino)phthalsäuredimethylester:
Eine Lösung von 3.76 g (10 mmol) 4,5-Bis(anilino)phthalsäuredimethylester (Beispiel 2b) in 15 ml HMPT (Hexamethylphosphorsäuretriamid) oder DMPU wird bei RT unter Argon mit 0.6 g (15.4 mmol) Natriumamid versetzt und während 30 min. auf 60°C erwärmt. Die tiefrote Lösung wird auf RT gekühlt und für 5 min evakuiert (1 Torr). Darauf wird eine Lösung von 1.5 ml (15.2 mmol) 1-Brom-3-chlorpropan in 2 ml THF zugetropft und das Reaktionsgemisch 18 Std. bei RT gerührt. Das Reaktionsgemisch wird auf Eiswasser gegossen, mit Essigester extrahiert, die organischen Phasen vereinigt und ausgiebig mit Wasser gewaschen, über Natriumsulfat getrocknet und eingedampft. Der Eindampfrückstand wird an Kieselgel mit Hexan/Essigester 5:1 chromatographiert. Auf diese Weise werden die Titelverbindungen erhalten: 4,5-Bis(anilino)-N⁴,N⁵-propandiyl-phthalsäuredimethylester in Form farbloser Kristalle, FAB-MS: 417 [M⁺+H]; und 4,5-Bis(N-allylanilino)phthalsäuredimethylester in Form eines farblosen Oels, FAB-MS: 457 [M⁺+H].

### Beispiel 23: Analog zu den vor- oder nachstehend genannten Verfahren wird hergestellt:

3-Hydroxy-4,5-bis(4-fluoranilino-2-azaindan-1-on (Tautomeres zu
4,5-Bis(4-fluoranilino)-2-formyl-benzoesäureamid) (Herstellung analog Beispiel 12 aus 4,5-Bis(4-fluoranilino)phthalimid (Beispiel 7a))).

### Beispiel 24: Analog zu den vor- oder nachstehend genannten Verfahren wird hergestellt:

4,5-Dianilino-2-carbamoyl-benzoesäure.

### Beispiel 25: 4,5-Bis(4-methylanilino)-2-hydroxymethyl-benzoesäure-amid:

Man versetzt eine Lösung von 4,5-Bis(4-methylanilino)phthalimid (0,35 g, 0,98 mmol) in Methanol (40 ml) bei 40 °C unter Rühren solange protionsweise mit festem Natriumborhydrid (0,3 g, 8 mmol), bis die anfangs gelbgefärbte Reaktionslösung farblos geworden ist und sich im Dünnschichtchromatogramm (Kieselgel, Essigsäureethylester/Hexan 1:1) kein Ausgangsmaterial mehr nachweisen lässt. Danach versetzt man mit Kochsalzlösung und extrahiert das gebildete Produkt mit Essigester. Nach dem Trocknen über Na₂SO₄ und Eindampfen des Essigsäureethylesterextraktes bleibt ein farbloser Rückstand, der aus Aceton/Diethylether kristallisiert. Nach zweimaliger Kristallisation erhält man die Titelverbindung in farblosen Kristallen, die zwischen 170 und 172 unter Zersetzung schmelzen: C₂₂H₂₃N₃O₂: Molekulargewicht ber. 361 gef. 361 (FD-MS).

Das Ausgangsmaterial wird wie folgt hergestellt:
a) 4,5-Bis(trimethylsilyloxy)cyclohexal,4-dien-1,2-dicarbonsäuredimethylester:
   Unter Argon wird eine Lösung von 7,1 g (50 mmol) Acetylendicarbonsäuredimethylester (Fluka, Schweiz) in 30 ml Toluol zu 12,5 g (50 mmol) 2,3-Bis(trimethylsilyloxy)1,3-butadien (Aldrich, Bundesrepublik Deutschland) (95%) getropft und anschliessend 19 h unter Rückflussbedingungen gekocht. Das Reaktionsgemisch wird abgekühlt, das Lösungsmittel abgedampft und der Rückstand im Hochvakuum (0,1 mbar, 124-127 °C) destilliert. Auf diese Weise wird die Titelverbindung als gelbes, hochviskoses Öl erhalten, ¹H-NMR (CDCl₃): δ=0,18 (s, 18H); 3,09 (s, 4H); 3,78 (s, 6H).
b) 4,5-Bis(4-methylanilino)phthalsäuredimethylester:
   Eine Lösung von 50 g (0,134 mol) 4,5-Bis(trimethylsilyloxy)cyclohexa-1,4-dien-1,2-dicarbonsäuredimethylester und 57,4 g (0,54 mol) p-Toluidin in 540 ml Eisessig wird während 4 h unter Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesattigter NaHCO₃ und zweimal 20 ml Wasser gewaschen, mit Na₂SO₄ getrocknet und eingedampft. Das Rohprodukt wird aus Ethanol umkristallisiert. Auf diese Weise wird die Titelverbindung in Form gelber Kristalle erhalten, Smp. 170 °C, C₂₄H₂₄N₂O₄, Molekulargewicht ber. 404, gef 404 (FD-MS).
c) 4,5-Bis(4-methylanilino)-phthalsäure:
   Man erhitzt 4,5-Bis(4-methylanilinophthalsäuredimethylester (0,98 g) in einem Gemisch aus Methanol (150 ml) und 2 M Natronlauge (25 ml) während 24 h unter Sauerstoffausschluss und Rückfluss. Danach dampft man das Methanol im Vakuum ab und säuert die alkalische Lösung des Reaktionsproduktes mit Salzsäure an. Die ausgeschiedene Dicarbonsäure wird in Tetrahydrofuran und Essigsäureethylester aufgenommen und direkt in roher Form weiterverwendet.
d) 4,5-Bis(4-methylanilino)phthalsäureanhydrid:
   Eine Lösung der zuletzt genannten rohen 4,5-Bis(4-methylanilino)-phthalsäure in Essigsäureanhydrid wird 30 min auf 60 °C erhitzt, wobei starke Gelbfärbung auftritt. Nach dem Eindampfen bleiben Kristalle von 4,5-Bis(4-methylanilino)phthalsäureanhydrid zurück, die aus Aceton/Diethylether umkristallisiert werden und dann bei 221-223 °C schmelzen. C₂₂H₁₈N₂O₃ Molekulargewicht ber. 358, gef. 358 (FD-MS).
e) 4,5-Bis(4-methylanilino)phthalimid:
   In eine Lösung von 4,5-Bis(4-methylanilino)-phthalsäureanhydrid (0,74 g) in 2-Ethoxyethanol (40 ml) wird bei 120 °C während 4 h Ammoniak eingeleitet. Danach wird eingedampft und der dunkelgefärbte Rückstand durch Filtration an Kieselgel (Laufmittel Essigsäureethylester/Hexan 1:1) von polarem, verharztem Material befreit. Die erhaltene gereinigte Titelverbindung ergibt aus Diethylether/Pentan orangerote, sehr derbe Kristalle, die bei 233-235 °C unter Zersetzung schmelzen. C₂₂H₁₉N₃O₂, Molekulargewicht ber. 357, gef. 357 (FD-MS).

### Beispiel 26: 3-Hydroxy-2-methyl-4,5-dianilino-2-azaindan-1-on (Tautomeres von 4,5-Dianilino-2-formyl-benzoesäure-N-methylamid):

Man versetzt eine Lösung von 4,5-Dianilinophthalsäure-N-methylimid (0,07 g, 0,2 mmol) in Methanol (10 ml) bei 40 °C unter Rühren solange portionsweise mit festem Natriumborhydrid (0,07 g, 1,8 mmol), bis die anfangs gelbgefärbte Reaktionslösung farblos geworden ist und sich im Dünnschichtchromatogramm (Kieselgel, Essigsäureethylester/Hexan 1:1) kein Ausgangsmaterial mehr nachweisen lässt. Danach versetzt man das gebildete Produkt mit Kochsalzlösung und extrahiert das gebildete Produkt mit Essigester. Nach dem Trocknen (Na₂SO₄) und Eindampfen des Essigsäureethylester-Extraktes wird der Rückstand durch Chromatographie an Kieselgel mit Essigsäureethylester/Hexan 1:1 als Elutionsmittel gereinigt. Nach einer sehr schwachen, gelbgefärbten Bande wird als Hauptmenge die Titeiverbindung eluiert. Sie bildet beim Eindampfen aus Methylenchlorid einen blassgelblichen Schaum. C₂₁H₁₉N₃O₂, Molekulargewicht ber. 345, gef. 345 (FD-MS).

Das Ausgangsmaterial wird folgendermassen hergestellt:

### a) 4,5-Dianilinophthalsäure-N-methylimid:

Es werden 376 mg (1 mmol) 4,5-Bis(anilino)phthalsäuredimethylester (Beispiel 2 b)) in 33 ml Ethylenglykol auf 120° erwärmt, und unter Rühren während 18 h Methylamin durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 1:1 an Kieselgel chromatographiert, die Produktfraktionen vereinigt und eingedampft. Auf diese Weise wird die Titelverbindung in Form schwach gelber Kristalle erhalten, FAB-MS: 344 [M⁺+H], Smp. 195-6°C.

### Beispiel 27: 4,5-Bis(4-methylanilino)phthalsäuremonoamid-monomethylester:

Eine Lösung von 4,5-Bis(4-methylanilino)phthalsäuremonomethylester (0,192 g, 0,49 mmol) in Acetonitril (25 ml) und Tetrahydrofuran (25 ml) wird mit Ammoniumhydrogencarbonat (0,080 g, 1 mmol) und 2-Ethoxy-1-ethoxycarbonyl-1,2-dihydrochinolin (0,130 g, 0,52 mmol) während 48 h bei Raumtemperatur stehengelassen. Das eingedampfte Reaktionsgemisch wird durch Chromatographie an Kieselgel gereinigt, wobei als Hauptprodukt ein Material mit R_{f}=0,53 (Dünnschichtchromatogramm an Kieselgel 60, Merck, Darmstadt, Bundesrepublik Deutschland; Laufmittel Essigsäureethylester) isoliert wird, welches die Titelverbindung ist. Die Titelverbindung bildert aus Tetrahydrofuran/Diethylether derbe Prismen, die sich ab 175 °C unter Gelbfärbung (Imidbildung) zersetzen. C₂₃H₂₃N₃O₃, Molekulargewicht ber. 389, gef. 389 (FD-MS).

Das Ausgangsmaterial wird wie folgt hergestellt:

### a) 4,5-Bis(4-methylanilinophthalsäuremonomethylester:

4,5-Bis(4-methylanilino)phthalsäureanhydrid (0,10 g; Beispiel 25 d)) wird mit Methanol (0,30 ml) und Pyridin (3 ml) unter Zusatz von 4-Dimethylaminopyridin (0,05 g ) 30 min unter Rückfluss gekocht. Die nahezu farblose Lösung wird dann eingedampft, der Rückstand in Essigsäureethylester aufgenommen und nacheinander mit Zitronensäurelösung und konzentrierter Kochsalzlösung gewaschen. Nach dem Trocknen (Na₂SO₄) und Eindampfen der Essigsäureethylesterlösung kristallisiert man den gelblich gewordenen Rückstand aus Tetrahydrofuran/Diethylether aus. Man erhält die Titelverbindung als Kristalle vom Smp. 167-169 °C. C₂₃H₂₂N₂O₄, Molekulargewicht ber. 390, gef. 391 (M⁺+1, FAB-MS).

### Beispiel 28:

Es werden 5000 Kapseln hergestellt, die je 0,25 g Wirkstoff, z.B. eine der in den Beispielen 1-27 hergestellten Verbindungen, enthalten:

| Zusammensetzung | |
|---|---|
| Wirkstoff | 1250 g |
| Talk | 180 g |
| Weizenstärke | 120 g |
| Magnesiumstearat | 80 g |
| Laktose | 20 g |

### Verfahren:

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

1. Verbindungen der Formel I, 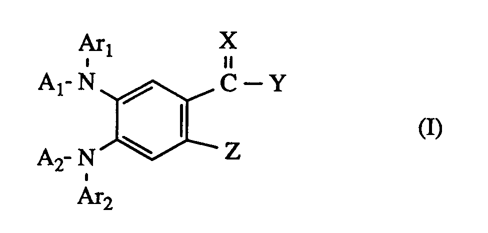 worin A₁ und A₂ unabhängig voneinander Wasserstoff, unsubstituiertes oder substituiertes Niederalkyl, unsubstituiertes oder substituiertes Niederalkenyl, unsubstituiertes oder substituiertes Niederalkinyl, Heterocyclylniederalkyl, Acyl, Niederalkylsulfonyl oder Arylsulfonyl bedeuten, oder worin A₁ und A₂ zusammen unsubstituiertes oder substituiertes Niederalkylen bedeuten; Ar₁ und Ar₂ unabhängig voneinander Aryl, Heteroaryl oder unsubstituiertes oder substituiertes Cycloalkyl bedeuten, X für O oder S, Y für Amino, substituiertes Amino, substituiertes Niederalkoxy oder unsubstituiertes oder substituiertes Niederalkylthio; oder ausserdem, falls X für O und Z für substituiertes Methyl stehen, für Hydroxy steht; und Z für substituiertes Methyl, Carboxy, verestertes Carboxy oder einen analogen Rest, in dem das Oxo des Carbonylrestes durch Thio ersetzt ist, oder amidiertes Carboxy oder oder einen analogen Rest, in dem das Oxo des Carbonylrestes durch Thio ersetzt ist, steht; oder Y und Z zusammen 1-Oxamethylen bedeuten, das über den Sauerstoff an der Stelle von Y und das Methylenkohlenstoffatom anstelle von Z gebunden ist; Salze davon, sofern salzbildende Gruppen vorliegen, und/oder Tautomere davon, sofern tautomerisierbare Reste vorliegen.

2. Verbindungen der Formel I gemäss Anspruch 1, worin A₁ und A₂ unabhängig voneinander Wasserstoff, Niederalkyl, substituiertes Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, C₃-C₈-Cycloalkylamino, Phenylniederalkylamino, Phenylamino, Niederalkanoylamino, Phenylniederalkanoylamino, Phenylcarbonylamino, Hydroxy, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert oder unsubstituiert ist, Phenylniederalkoxy, Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Phenylniederalkylthio, Niederalkanoylthio, Carboxy, Niederalkoxycarbonyl, Phenylniederalkoxycarbonyl, Cyano, Carbamoyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, Thioureido, an einem oder beiden Stickstoffatomen durch Niederalkyl, Aryl oder Arylniederalkyl substituiertes Thioureido, Hydrazino, an einem oder beiden Stickstoffatomen durch Niederalkyl, Aryl oder Arylniederalkyl substituiertes Hydrazino, Amidino, an einem oder beiden Stickstoffatomen durch Niederalkyl, Aryl oder Arylniederalkyl substituiertes Amidino, Guanidino, an einem, zwei oder allen drei Stickstoffatomen durch Niederalkyl, Aryl oder Arylniederalkyl substituiertes Guanidino, Oxo, welches nicht am Kohlenstoff gebunden ist, welcher an den A₁ oder A₂ tragenden Stickstoff gebunden ist, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono, Niederalkoxycarbonylhydrazono, Niederalkylthioimino, durch einen der für substituiertes Niederalkyl genannten Reste substituiertes oder unsubstituiertes Niederalkenyl oder Niederalkinyl, Heterocyclylniederalkyl, worin Heterocyclyl ein unsubstituierter oder durch Niederalkyl, Niederalkanoyl, Hydroxy, Niederalkoxy, Halogen, Cyano und/oder Trifluormethyl substituierter, über ein Ringstickstoffatom gebundener Rest ausgewählt aus Pyrrolyl, 2,5-Dihydropyrrolyl, Pyrrolinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl und 1,2-Dihydro- oder 1,2,3,4-Tetrahydroisochinolyl ist, der endständig an Niederalkyl gebunden ist, Niederalkylsulfonyl, Benzolsulfonyl oder Toluolsulfonyl bedeuten; oder worin A₁ und A₂ zusammen Niederalkylen bilden, welches unsubstituiert oder substituiert ist durch bis zu 3 Substituenten ausgewählt aus Niederalkyl, Amino, Aminoniederalkyl, Mono- oder Diniederalkylamino oder Mono- oder Diniederalkylamino-niederalkyl, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Cycloalkylamino, Cycloalkylaminoniederalkyl, Phenylniederalkylamino, Phenylniederalkylaminoniederalkyl, Phenylamino, Phenylaminoniederalkyl, Niederalkanoylamino, Phenylniederalkanoylamino, Phenylcarbonylamino, Niederalkanoylaminoniederalkyl, Phenylniederalkanoylaminoniederalkyl, Phenylcarbonylaminoniederalkyl, Hydroxy, Hydroxyniederalkyl, Niederalkoxy oder Niederalkoxyniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Phenylniederalkoxy, Phenylniederalkoxyniederalkyl, Niederalkanoyloxy, Niederalkanoyloxyniederalkyl, Mercapto, Mercaptoniederalkyl, Niederalkylthio oder Niederalkylthioniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Phenylniederalkylthio, Phenylniederalkylthioniederalkyl, Niederalkanoylthio, Niederalkanoylthioniederalkyl, Carboxy, Carboxyniederalkyl, Niederalkoxycarbonyl, Niederalkoxycarbonylniederalkyl, Phenylniederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylnieder-alkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, Thioureido, Thioureidoniederalkyl, an einem oder beiden Stickstoffatomen durch Niederalkyl, Aryl oder Arylniederalkyl substituiertes Thioureido oder Thioureidoniederalkyl, Hydrazino, Hydrazinoniederalkyl, an einem oder beiden Stickstoffatomen durch Niederalkyl, Aryl oder Arylniederalkyl substituiertes Hydrazino oder Hydrazinoniederalkyl, Amidino, Amidinoniederalkyl, an einem oder beiden Stickstoffatomen durch Niederalkyl, Aryl oder Arylniederalkyl substituiertes Amidino oder Amidinoniederalkyl, Guanidino, Guanidinoniederalkyl, an einem, zwei oder allen drei Stickstoffatomen durch Niederalkyl, Aryl oder Arylniederalkyl substituiertes Guanidino oder Guanidinoniederalkyl, Oxo, Oxoniederalkyl, Thioxo, Thioxoniederalkyl, Imino, Iminoniederalkyl, Niederalkylimino, Niederalkyliminoniederalkyl, Niederalkanoylimino, Niederalkanoyliminoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl; Ar₁ und Ar₂ unabhängig voneinander Aryl, Heteroaryl ausgewählt aus Imidazolyl, Triazolyl, Pyridyl, Pyrimidinyl und Triazinyl, welche unsubstituiert oder durch Niederalkyl, Hydroxy, Niederalkoxy, Halogen, Cyano und/oder Trifluormethyl substituiert sind, oder C₃-C₈-Cycloalkyl, welches unsubstituiert oder durch Niederalkoxy oder Hydroxy substituiert ist, bedeuten; X für O oder S steht; Y für Amino, Mono- oder Diniederalkylamino, worin Niederalkyl unsubstituiert oder durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, C₅-C₇-Cycloalkylamino, Phenylniederalkylamino, Phenylamino, Niederalkanoylamino, Halogenniederalkanoylamino, Phenylniederalkanoylamino, Phenylcarbonylamino, Hydrazino, an einem oder beiden Stickstoffatomen durch Niederalkyl, welches durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Aryl oder Arylniederalkyl substituiertes Hydrazino, Hydroxyamino, Niederalkoxyamino, worin der Niederalkylrest durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Niederalkylenamino oder durch -O-, -S- oder -NR'- unterbrochenes Niederalkylenamino, worin R' Niederalkyl oder Niederalkanoyl bedeutet, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, oder Niederalkylthio, welches unsubstituiert oder im Niederalkylthiorest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist, oder ausserdem, falls X Sauerstoff und Z substituiertes Methyl bedeuten, für Hydroxy steht; Z für substituiertes Methyl, worin der Substituent aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkyl- carbamoyl, N,N-Diniederalkylcarbamoyl und/oder Cyano substituiert oder unsubstituiert ist, Niederalkanoylamino, Hydroxy, Niederalkoxy, worin der Niederalkylrest ein- oder zweifach durch Hydroxy, Niederalkoxy, Phenylniederalkoxy, Niederalkanoyloxy, Amino, Niederalkylamino und/oder Diniederalkylamino substituiert oder unsubstituiert ist, Niederalkanoyloxy, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Cyano, Ureido, 1- oder 3-Mononiederalkylureido, Hydrazino, an einem oder beiden Stickstoffatomen durch Niederalkyl substituiertes Hydrazino, Guanidino, an einem, zwei oder allen drei Stickstoffatomen durch Niederalkyl substituiertes Guanidino, Oxo, Imino, Niederalkylimino, Hydroxyimino, Hydrazono, N-Mono- oder N,N-Diniederalkylhydrazono und Niederalkylthioimino ausgewählt ist; Carboxy, Niederalkoxycarbonyl oder Niederalkylthio-carbonyl, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist; Aryloxycarbonyl oder Arylniederalkoxycarbonyl, Carbamoyl, N-Mono- oder N,N-Diniederalkylcarbamoyl, worin Niederalkyl durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, C₅-C₇-Cycloalkylaminocarbonyl, N-Phenylniederalkylcarbamoyl, Phenylcarbamoyl, Hydrazinocarbonyl, an einem oder beiden Stickstoffatomen durch Niederalkyl, welches durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist,. Aryl oder Arylniederalkyl substituiertes Hydrazinocarbonyl, Hydroxyaminocarbonyl, Niederalkoxyaminocarbonyl, worin der Niederalkylrest durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Niederalkylenaminocarbonyl oder durch -O-, -S- oder -NR'-, worin R' Niederalkyl oder Niederalkanoyl bedeutet, unterbrochenes Niederalkylenaminocarbonyl, oder ein entsprechendes Thioanaloges der genannten Reste, worin die bindende Carbonylgruppe durch Thiocarbonyl ersetzt ist; oder worin Y und Z zusammen ein bivalentes Radikal der Formel -O-CH₂- bilden, wobei der Sauerstoff anstelle von Y und der Methylenkohlenstoff anstelle von Z gebunden ist;
wobei in den genannten Resten Aryl Phenyl bedeutet, das unsubstituiert oder durch einen oder mehrere Substituenten aus der Gruppe bestehend aus Niederalkyl, Niederalkenyl, Niederalkinyl, Niederalkylen (angeknüpft an zwei benachbarten C-Atomen), C₅-C₇-Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy oder Niederalkinyloxy; Niederalkylendioxy (angeknüpft an zwei benachbarten C-Atomen); Niederalkanoyloxy, Phenylniederalkanoyloxy oder Phenylcarbonyloxy; Mercapto; Niederalkylthio, Phenylniederalkylthio, Phenylthio, Niederalkylsulfinyl, Phenylniederalkylsulfinyl, Benzolsulfinyl, Niederalkylsulfonyl, Phenylniederalkylsulfonyl oder Benzolsulfonyl; Halogen, Nitro, Amino; Niederalkylamino, C₅-C₇-Cycloalkylamino, Phenylniederalkylamino oder Phenylamino; Diniederalkylamino, N-Niederalkyl-N-phenylamino, N-Niederalkyl-N-phenylniederalkylamino, Niederalkylenamino; Niederalkanoylamino, Phenylniederalkanoylamino oder Phenylcarbonylamino; Niederalkanoyl, Phenylniederalkanoyl oder Phenylcarbonyl; Carboxy; Niederalkoxycarbonyl; Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy und Phenylniederalkoxy, wie Benzyloxy, ausgewählte Reste am Phosphor substituiertes Phosphoryloxy oder durch Phenylen-1,2-dioxy am Phosphor substituiertes Phosphoryloxy, Sulfo; Niederalkoxysulfonyl; Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Salze davon, sofern salzbildende Gruppen vorliegen, und/oder Tautomere davon, sofern tautomerisierbare Reste vorliegen.

3. Verbindungen der Formel I gemäss Anspruch 1, worin A₁ und A₂ unabhängig voneinander Wasserstoff, Aminoniederalkyl, Carbamoylniederalkyl, (3-Niederalkylureido)niederalkyl oder Niederalkenyl bedeuten oder worin A₁ und A₂ zusammen 1,2-Ethylen bilden, welches unsubstituiert oder durch einen Substituenten ausgewählt aus Aminoniederalkyl, Hydroxyniederalkyl und Hydroxyimino-niederalkyl substituiert ist; Ar₁ und Ar₂ unabhängig voneinander unsubstituiertes oder durch einen Rest ausgewählt aus Niederalkyl, Hydroxy, Niederalkoxy, Halogen, Carboxy, Niederalkoxycarbonyl und Cyano o-, m- oder p-substituiertes Phenyl, 2-, 3- oder 4-Pyridyl, 2-, 4- oder 5-Pyrimidinyl oder 1,3,5-Triazin-2-yl bedeuten; X Sauerstoff bedeutet; Y Amino, Niederalkylamino, Phenylamino, Hydroxyniederalkoxy oder Hydrazino bedeutet, oder ausserdem, falls Z Hydroxymethyl, Niederalkanoyloxymethyl, Formyl oder Hydroxyiminomethyl bedeutet, Hydroxy bedeutet; Z Hydroxymethyl, Niederalkanoyloxymethyl, Carboxy, Niederalkoxycarbonyl, Hydroxyniederalkoxycarbonyl, Carbamoyl, Formyl oder Hydroxyiminomethyl bedeutet; oder worin Y und Z zusammen ein bivalentes Radikal der Formel -O-CH₂-bilden, wobei der Sauerstoff anstelle von Y und der Methylenkohlenstoff anstelle von Z gebunden ist, Salze davon, sofern salzbildende Gruppen vorliegen, und/oder Tautomere/n davon, sofern tautomerisierbare Reste vorliegen.

4. Verbindungen der Formel I gemäss Anspruch 1, worin A₁ und A₂ je ein Wasserstoffatom bedeuten; Ar₁ und Ar₂ unabhängig voneinander unsubstituiertes oder durch einen Rest ausgewählt aus Niederalkyl, Hydroxy, Niederalkoxy, Halogen, Carboxy, Niederalkoxycarbonyl und Cyano o-, m- oder p-substituiertes Phenyl oder Pentafluorphenyl bedeuten; X Sauerstoff bedeutet; Y Amino bedeutet; und Z Carbamoyl bedeutet; oder Salze davon, sofern salzbildende Gruppen vorliegen.

5. Verbindungen der Formel I gemäss Anspruch 1, worin A₁ und A₂ zusammen unsubstituiertes oder durch einen Rest ausgewählt aus Hydroxyniederalkyl, Aminoniederalkyl und Hydroxyiminoniederalkyl substituiertes 1,2-Ethylen bedeuten, Ar₁ und Ar₂ unabhängig voneinander unsubstituiertes oder durch einen Rest ausgewählt aus Niederalkyl, Hydroxy, Niederalkoxy, Halogen, Carboxy, Niederalkoxycarbonyl und Cyano o-, m- oder p-substituiertes Phenyl oder Pentafluorphenyl bedeuten; X Sauerstoff bedeutet; Y Amino oder Hydroxyniederalkoxy bedeutet; und Z Carboxy, Niederalkoxycarbonyl, Hydroxyniederalkoxycarbonyl, oder Carbamoyl bedeutet, oder Salze davon, sofern salzbildende Gruppen vorliegen.

6. Verbindungen der Formel I gemäss Anspruch 1, worin A₁ und A₂ unabhängig voneinander Wasserstoff oder Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkansulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist; C₅-C₇-Cycloalkylamino, Phenylniederalkylamino oder Phenylamino; Niederalkanoylamino, Phenylniederalkanoylamino oder Phenylcarbonylamino; Hydroxy, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Cyano, Carbamoyl, N-Niederalkylcarbamoyl, N,N-Diniederalkylcarbamoyl und/oder Cyano substituiert oder unsubstituiert ist, Phenylniederalkoxy, Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Phenylniederalkylthio, Niederalkanoylthio, Carboxy, Niederalkoxycarbonyl, Phenylniederalkoxycarbonyl, Cyano, Carbamoyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, Thioureido, an einem oder beiden Stickstoffatomen durch Niederalkyl, Aryl oder Arylniederalkyl substituiertes Thioureido, Hydrazino, an einem oder beiden Stickstoffatomen durch Niederalkyl, Aryl oder Arylniederalkyl substituiertes Hydrazino, Amidino, an einem oder beiden Stickstoffatomen durch Niederalkyl, Aryl oder Arylniederalkyl substituiertes Amidino, Guanidino, an einem, zwei oder allen drei Stickstoffatomen durch Niederalkyl, Aryl oder Arylniederalkyl substituiertes Guanidino, Oxo, welches nicht am Kohlenstoff gebunden ist, welches am A₁ oder A₂ tragenden Stickstoff vorliegt, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono und Niederalkylthioimino substituiert ist; Ar₁ und Ar₂ unabhängig voneinander für Aryl oder für Heteroaryl stehen; X für O steht; Y für Amino, für Niederalkylamino, Phenylamino, Hydrazino oder Hydroxyniederalkoxy steht, oder ausserdem, falls Z substituiertes Methyl bedeutet, für Hydroxy steht; und Z für substituiertes Methyl, für Carboxy oder für verestertes Carboxy steht; oder worin Y und Z zusammen ein bivalentes Radikal der Formel -O-CH₂- bilden, wobei der Sauerstoff anstelle von Y und der Methylenkohlenstoff anstelle von Z gebunden ist, wobei in den oben genannten Definitionen
Aryl Phenyl bedeutet, das unsubstituiert oder durch einen oder mehrere Substituenten aus der Gruppe bestehend aus Niederalkyl, Niederalkenyl, Niederalkinyl, Niederalkylen (angeknüpft an zwei benachbarten C-Atomen), C₅-C₇-Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy oder Niederalkinyloxy; Niederalkylendioxy (angeknüpft an zwei benachbarten C-Atomen); Niederalkanoyloxy, Phenylniederalkanoyloxy oder Phenylcarbonyloxy; Mercapto; Niederalkylthio, Phenylniederalkylthio, Phenylthio, Niederalkylsulfinyl, Phenylniederalkylsulfinyl, Benzolsulfinyl, Niederalkylsulfonyl, Phenylniederalkylsulfonyl oder Benzolsulfonyl; Halogen, Nitro, Amino; Niederalkylamino, C₅-C₇-Cycloalkylamino, Phenylniederalkylamino oder Phenylamino; Diniederalkylamino, N-Niederalkyl-N-phenylamino, N-Niederalkyl-N-phenylniederalkylamino, Niederalkylenamino, Niederalkanoylamino, Phenylniederalkanoylamino oder Phenylcarbonylamino; Niederalkanoyl, Phenylniederalkanoyl oder Phenylcarbonyl; Carboxy; Niederalkoxycarbonyl; Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy und Phenylniederalkoxy, wie Benzyloxy, ausgewählte Reste am Phosphor substituiertes Phosphoryloxy oder durch Phenylen-1,2-dioxy am Phosphor substituiertes Phosphoryloxy; Sulfo; Niederalkoxysulfonyl; Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind;
und Heteroaryl einen über ein Ringkohlenstoffatom verknüpften 5- oder 6-gliedrigen Ring mit bis zu 3 Ringstickstoffatomen, wobei der Ring unsubstituiert oder durch Niederalkyl, Hydroxy, Niederalkoxy, Halogen, Cyano und/oder Trifluormethyl substituiert sein kann, bedeutet; Salze davon, sofern salzbildende Gruppen vorliegen, und/oder Tautomere davon, sofern tautomerisierbare Reste vorliegen.

7. Verbindungen der Formel I gemäss Anspruch 1, worin A₁ und A₂ unabhängig voneinander Wasserstoff oder Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkansulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Phenylniederalkylamino, Phenylamino, Niederalkanoylamino, Phenylniederalkanoylamino, Phenylcarbonylamino, Hydroxy, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, Phenylniederalkoxy, Niederalkanoyloxy, Carboxy, Niederalkoxycarbonyl, Phenylniederalkoxycarbonyl, Carbamoyl, N-Niederalkylcarbamoyl, N,N-Diniederalkylcarbamoyl, Ureido, 1- oder 3-Mono- oder 1,3- oder 3,3-Di-niederalkylureido, Oxo, welches nicht am Kohlenstoff gebunden ist, welcher an den A₁ oder A₂ tragenden Stickstoff gebunden ist, Imino und Hydroxyimino substituiert ist, bedeuten; Ar₁ und Ar₂ unabhängig voneinander für Phenyl, das unsubstituiert oder durch einen oder zwei Substituenten aus der Gruppe bestehend aus Niederalkyl, Trifluormethyl, Hydroxy, Niederalkoxy, Halogen, Carboxy, Niederalkoxycarbonyl, Carbamoyl, N-Niederalkylcarbamoyl, N,N-Diniederalkylcarbamoyl und Cyano substituiert ist, oder für Heteroaryl ausgewählt aus 2-, 3- oder 4-Pyridyl, 2-, 4- oder 5-Pyrimidinyl und 1,3,5-Triazin-2-yl, welche unsubstituiert oder durch Niederalkyl, Hydroxy, Niederalkoxy, Halogen, Cyano und/oder Trifluormethyl substituiert sind, stehen; X für O steht; Y für Amino, Niederalkylamino, Phenylamino, Hydrazino oder Hydroxyniederalkoxy steht, oder ausserdem, falls Z Hydroxymethyl, Niederalkanoyloxymethyl, Formyl oder Hydroxyiminomethyl bedeutet, für Hydroxy steht; und Z für Hydroxymethyl, Niederalkanoyloxymethyl, Formyl, Carboxy, Niederalkoxycarbonyl, Hydroxyniederalkoxycarbonyl oder Hydroxyiminomethyl steht; oder worin Y und Z zusammen ein bivalentes Radikal der Formel -O-CH₂- bilden, wobei der Sauerstoff anstelle von Y und der Methylenkohlenstoff anstelle von Z gebunden ist; Salze davon, sofern salzbildende Gruppen vorliegen, und/oder Tautomere davon, sofern tautomerisierbare Reste vorliegen.

8. Eine Verbindung der Formel I gemäss Anspruch 1 ausgewählt aus den Verbindungen mit den Bezeichnungen
4,5-Bis(4-fluoranilino)phthalsäurediamid, Bis(2-hydroxyethyl)-5,8-Diphenyl-5,8-diaza-5,6,7,8-tetrahydro-naphthalin-2,3-dicarboxylat, 4,5-Dianilino-phthalsäurediamid, 4,5-Bis(2-iodanilino)phthalsäurediamid, 4,5-Bis(3-iodanilino)phthalsäurediamid, 4,5-Bis(4-iodanilino)phthalsäurediamid, 4,5-Bis(2-methoxyanilino)phthalsäurediamid, 4,5-Bis(3-methoxyanilino)phthalsäurediamid, 4,5-Bis(4-methoxyanilino)phthalsäurediamid, 4,5-Bis(2-cyananilino)phthalsäurediamid, 4,5-Bis(3-cyananilino)phthalsäurediamid, 4,5-Bis(4-cyananilino)phthalsäurediamid, 4,5-Bis(2-fluoranilino)phthalsäurediamid, 4,5-Bis(3-fluoranilino)phthalsäurediamid, 4,5-Bis(pentafluoranilino)phthalsäurediamid, 4,5-Bis(4-hydroxyanilino)phthalsäurediamid, 4,5-Bis(3-hydroxyanilino)phthalsäurediamid, 4,5-Bis(2-hydroxyanilino)phthalsäurediamid, 4,5-Bis(4-ethylanilino)phthalsäurediamid, 4,5-Bis(3-ethylanilino)phthalsäurediamid, 4,5-Bis(2-ethylanilino)phthalsäurediamid, 4,5-Bis(4-methylanilino)phthalsäurediamid, 4,5-Bis(3-methylanilino)phthalsäurediamid, 4,5-Bis(2-methylanilino)phthalsäurediamid, 5,8-Diphenyl-5,8-diaza-6-hydroxymethyl-5,6,7,8-tetrahydro-naphthalin-2,3-dicarboxamid, 6-Aminomethyl-5,8-diphenyl-5,8-diaza-5,6,7,8-tetrahydro-naphthalin-2,3-dicarboxamid, 5,8-Diphenyl-5,8-diaza-6-hydroxymethyl-5,6,7,8-tetrahydro-naphthalin-2,3-dicarbonsäuremonoamid-mono-methylester, 6-Aminomethyl-5,8-diphenyl-5,8-diaza-5,6,7,8-tetrahydro-naphthalin-2,3-dicarbonsäuremonoamid-monomethylester, 5,8-Bis(4-fluorphenyl)-5,8-diaza-6-hydroxymethyl-5,6,7,8-tetrahydro-naphthalin-2,3-dicarboxamid, 6-Aminomethyl-5,8-bis(4-fluorphenyl)-5,8-diaza-5,6,7,8-tetrahydro-naphthalin-2,3-dicarboxamid, 5,8-Bis(4-fluorphenyl)-5,8-diaza-6-hydroxymethyl-5,6,7,8-tetrahydro-naphthalin-2,3-dicarbonsäuremonoamid-monomethylester, 6-Aminomethyl-5,8-bis(4-fluorphenyl). -5,8-diaza-5,6,7,8-tetrahydro-naphthalin-2,3-dicarbonsäuremonoamid-monomethylester, 5,8-Bis(4-fluorphenyl)-5,8-diaza-6-hydroximinomethyl-5,6,7,8-tetrahydro-naphthalin-2,3-dicarbonsäuremonoamid-monomethylester, 5,8-Bis(4-fluorphenyl)-5,8-diaza-6-hydroximinomethyl-5,6,7,8-tetrahydro-naphthalin-2,3-dicarbonsäuremonoamid-monomethylester, 5,8-Bis(4-fluorphenyl)-5,8-diaza-6-hydroximinomethl-5,6,7,8-tetrahydro-naphthalin-2,3-dicarboxamid, 5,8-Diphenyl-5,8-diaza-6-hydroximinomethyl-5,6,7,8-tetrahydro-naphthalin-2,3-dicarbonsäuremonoamid-monomethylester oder 5,8-Diphenyl-5,8-diaza-6-hydroximinomethyl-5,6,7,8-tetrahydro-naphthalin-2,3-dicarboxamid, oder pharmazeutsch verwendbare Salze davon, sofern salzbildende Gruppen vorliegen.

9. 4,5-Bis(4-fluoranilino)phthalsäurediamid gemäss Anspruch 4.

10. 4,5-Dianilino-phthalsäuremonoamid-monomethylester gemäss Anspruch 6.

11. 4,5-Dianilino-phthalsäuremonamid gemäss Anspruch 6, oder ein pharmazeutisch verwendbares Salz davon.

12. 4,5-Dianilino-phthalsäure-di-2-hydroxyethylester gemäss Anspruch 6.

13. 4,5-Bis(anilino)phthalsäuremonomethyl-mono-2-hydroxyethylester gemäss Anspruch 6.

14. Bis(2-hydroxyethyl)-5,8-Diphenyl-5,8-diaza-5,6,7,8-tetrahydro-naphthalin-2,3-dicarboxylat gemäss Anspruch 5, oder ein pharmazeutisch verwendbares Salz davon.

15. 4,5-Dianilino-2-hydroxymethyl-benzoesäureamid gemäss Anspruch 6.

16. 4,5-Bis(4-fluoranilino)-2-hydroxymethyl-benzoesäureamid gemäss Anspruch 6.

17. 4,5-Dianilino-2-hydroxymethyl-benzoesäure gemäss Anspruch 6, oder ein pharmazeutisch verwendbares Salz davon.

18. 5,6-Dianilino-2-oxa-indan-1-on gemäss Anspruch 6.

19. 4,5-Dianilino-2-hydroxymethyl-benzoesäuremethylamid gemäss Anspruch 6.

20. 4,5-Dianilino-2-hydroxymethyl-benzoesäurehydrazid gemäss Anspruch 6.

21. 3-Hydroxy-4,5-dianilino-2-azaindan-1-on gemäss Anspruch 6.

22. 4,5-Dianilino-2-hydroxyiminomethyl-benzoesäureamid gemäss Anspruch 6.

23. 2-Acetoxymethyl-4,5-dianilino-benzoesäureamid gemäss Anspruch 6.

24. 4,5-Dianilinophthalsäuremonoanilid gemäss Anspruch 6.

25. Eine Verbindung der Formel I gemäss Anspruch 6, ausgewählt aus 4,5-Dianilino-3-carbamoyl-2-carbonsäure und
3-Hydroxy-4,5-bis(4-fluoranilino-2-azaindan-1-on, und Salzen davon, sofern salzbildende Gruppen vorliegen.

26. 4,5-Dianilinophthalsäuremonoanilid gemäss Anspruch 6.

27. 5,8-Diphenyl-5,8-diaza-6-hydroxymethyl-5,6,7,8-tetrahydro-naphthalin-2,3-dicarbonsäuredi(2-hydroxyethyl)ester gemäss Anspruch 4.

28. 5,8-Diphenyl-5,8-diaza-5,6,7,8-tetrahydro-naphthalin-2,3-dicarbonsäuremono(2-hydroxyethyl)ester-monomethylester gemäss Anspruch 4.

29. 5,8-Diphenyl-5,8-diaza-6-hydroxymethyl-5,6,7,8-tetrahydro-naphthalin-2,3-dicarboxamid gemäss Anspruch 4.

30. 4,5-Bis(3-iodanilino)phthalsäurediamid gemäss Anspruch 4 oder 6.

31. 4,5-Bis(N-allyl-N-phenyl)aminophthalsäuremonoamid gemäss Anspruch 1.

32. 4,5-Bis(4-methylanilino)-2-hydroxymethyl-benzoesäure-amid gemäss Anspruch 6.

33. 3-Hydroxy-2-methyl-4,5-dianilino-2-azaindan-1-on gemäss Anspruch 6.

34. 4,5-Bis(4-methylanilino)phthalsäuremonoamid-monomethylester gemäss Anspruch 6.

35. Pharmazeutische Präparate umfassend Verbindungen der Formel I oder ein pharmazeutisch verwendbares Salz einer solchen Verbindung mit mindestens einer salzbildenden Gruppe gemäss einem der Ansprüche 1 bis 34 zusammen mit pharmazeutisch verwendbarem Trägermaterial.

36. Eine Verbindung der Formel I oder ein pharmazeutisch verwendbares Salz einer solchen Verbindung mit mindestens einer salzbildenden Gruppe gemäss einem der Ansprüche 1 bis 34 zur Anwendung in einem Verfahren zur therapeutischen Behandlung des tierischen oder menschlichen Körpers.

37. Verwendung einer Verbindung der Formel I oder eines pharmazeutisch verwendbaren Salzes einer solchen Verbindung mit mindestens einer salzbildenden Gruppe gemäss einem der Ansprüche 1 bis 34 zur Herstellung pharmazeutischer Präparate gegen solche Erkrankungen, die auf eine Hemmung von Protein-Tyrosinkinasen ansprechen.

38. Verfahren zur Herstellung einer Verbindung der Formel I gemäss Anspruch 1, dadurch gekennzeichnet, dass man
a) zur Herstellung der Verbindungen der Formel I, worin X für O steht, Y für Amino, substituiertes Amino, substituiertes Niederälkoxy oder unsubstituiertes oder substituiertes Niederalkylthio steht und Z für Carboxy, verestertes Carboxy oder amidiertes Carboxy steht, und worin die übrigen Reste die für Verbindungen der Formel I genannten Bedeutungen haben, oder eines Salzes davon, ein reaktionsfähiges Säurederivat einer Dicarbonsäure der Formel II, 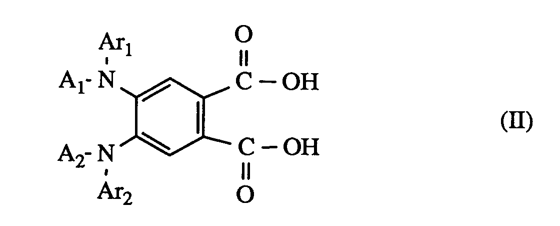 worin A₁, A₂, Ar₁ und Ar₂ die genannten Bedeutungen haben, zur Einführung des Restes Y mit einer Verbindung der Formel III,
W₁-H (III)
worin W₁ unsubstituiertes oder substituiertes Amino, substituiertes Niederalkoxy oder unsubstituiertes oder substituiertes Niederalkylthio bedeutet, und/oder zur Herstellung des Restes Z mit Wasser oder einer Verbindung der Formel IV
W₂-H (IV),
worin W₂ einen zur Herstellung von verestertem Carboxy oder amidiertem Carboxy Z geeigneten komplementären Rest bedeutet, umsetzt, wobei in den Ausgangsmaterialien der Formeln II, III und IV vorhandene funktionelle Gruppen, die nicht an der Reaktion teilnehmen sollen, erforderlichenfalls in geschützter Form vorliegen, und vor und/oder nach einer der unten genannten zusätzlichen Verfahrensmassnahmen vorhandene Schutzgruppen abspaltet, oder
zur Herstellung von Verbindungen der Formel I, worin Z für Hydroxymethyl oder Formyl steht und worin die übrigen Reste die für Verbindungen der Formel I genannten Bedeutungen haben, oder eines Salzes und/oder Tautomeren davon, eine Carbonsäure der Formel V, 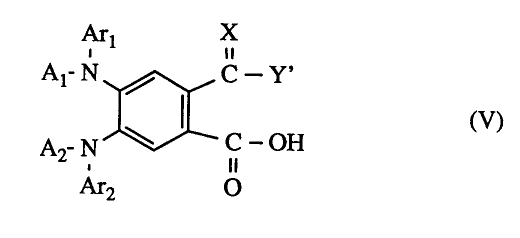
worin Y' Hydroxy oder einen der Reste Y bedeutet, wie für Verbindungen der Formel I definiert, mit der Massgabe, dass X Sauerstoff bedeutet, falls Y' Hydroxy bedeutet, und worin die übrigen Reste die für Verbindungen der Formel I genannten Bedeutungen haben, oder ein reaktionsfähiges Derivat davon reduziert, wobei im Ausgangsmaterial der Formel V vorhandene funktionelle Gruppen, die nicht an der Reaktion teilnehmen sollen, erforderlichenfalls in geschützter Form vorliegen, und vor und/oder nach einer der unten genannten zusätzlichen Verfahrensmassnahmen vorhandene Schutzgruppen abspaltet,
und gewünschtenfalls als zusätzliche Verfahrensmassnahme eine erhältliche Verbindung der Formel I in eine andere Verbindung der Formel I umwandelt, und/oder ein erhältliches Salz in die freie Verbindung oder in ein anderes Salz umwandelt, und/oder eine erhältliche freie Verbindung der Formel I in ein Salz umwandelt und/oder ein erhältliches Gemisch von isomeren Verbindungen der Formel I in die einzelnen Isomeren auftrennt.
